(19) **Europäisches Patentamt / European Patent Office / Office européen des brevets**

(11) **EP 4 596 718 A1**

(12) **EUROPEAN PATENT APPLICATION**
published in accordance with Art. 153(4) EPC

(43) Date of publication:
06.08.2025 Bulletin 2025/32

(21) Application number: 23873265.5

(22) Date of filing: 27.09.2023

(51) International Patent Classification (IPC):
*C12Q 1/6886* $^{(2018.01)}$    *G01N 33/574* $^{(2006.01)}$

(52) Cooperative Patent Classification (CPC):
C12Q 1/6886; G01N 33/574

(86) International application number:
PCT/KR2023/015129

(87) International publication number:
WO 2024/072158 (04.04.2024 Gazette 2024/14)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 28.09.2022 KR 20220123808

(71) Applicants:
• Huvet Bio, Inc.
 Seoul 05836 (KR)
• Seoul National University Hospital
 Seoul 03080 (KR)

(72) Inventors:
• JEONG, Hyoung Hwa
 Seoul 05649 (KR)

• KIM, Hong Sik
 Seoul 08018 (KR)
• SIM, Eun Jin
 Seoul 05534 (KR)
• HWANG, Jin-Hyeok
 Seoul 06630 (KR)
• KIM, Jihie
 Seoul 04722 (KR)

(74) Representative: Reddie & Grose LLP
The White Chapel Building
10 Whitechapel High Street
London E1 8QS (GB)

Remarks:
The complete document including Reference Table(s) and the Sequence Listing(s) can be downloaded from the EPO website

(54) **COMPOSITION FOR DIAGNOSING PANCREATIC CANCER**

(57) Provided are a biomarker for diagnosing pancreatic cancer, a composition containing an agent capable of detecting the biomarker for diagnosing pancreatic cancer, and a method for diagnosing pancreatic cancer using same.

**EP 4 596 718 A1**

EP 4 596 718 A1

[FIG. 30]

| Test set | Total test size | Positive group size | Negative group size | Marker set name | P-value | Youden index (J) | AUC (95% CI) | Sensitivity | Specificity |
|---|---|---|---|---|---|---|---|---|---|
| Pancreatic Cancer (Stage: Early stage only) vs Normal | 212 | 35 | 177 | 9 Huvet markers | <0.0001 | 0.8634 | 0.979 (0.949 to 0.993) | 91.43 | 94.92 |
| | 212 | 35 | 177 | Marker Set 1 (12 markers) | <0.0001 | 0.8804 | 0.979 (0.949 to 0.994) | 91.43 | 96.61 |
| | 212 | 35 | 177 | Marker Set 2 (15 markers) | <0.0001 | 0.8892 | 0.980 (0.951 to 0.994) | 91.18 | 97.74 |
| | 212 | 35 | 177 | Marker Set 3 (8 markers) | <0.0001 | 0.8418 | 0.972 (0.939 to 0.989) | 100.00 | 84.18 |
| | 211 | 34 | 177 | All huvet markers | <0.0001 | 0.9084 | 0.984 (0.957 to 0.996) | 97.06 | 93.79 |
| Pancreatic Cancer (Stage: Late stage only) vs Normal | 244 | 67 | 177 | 9 Huvet markers | <0.0001 | 0.7938 | 0.951 (0.916 to 0.975) | 89.55 | 89.83 |
| | 244 | 67 | 177 | Marker Set 1 (12 markers) | <0.0001 | 0.7216 | 0.943 (0.907 to 0.969) | 97.01 | 75.14 |
| | 244 | 67 | 177 | Marker Set 2 (15 markers) | <0.0001 | 0.7841 | 0.955 (0.920 to 0.977) | 92.54 | 85.88 |
| | 244 | 67 | 177 | Marker Set 3 (8 markers) | <0.0001 | 0.6829 | 0.905 (0.861 to 0.938) | 88.06 | 80.23 |
| | 244 | 67 | 177 | All huvet markers | <0.0001 | 0.7991 | 0.956 (0.922 to 0.978) | 94.03 | 85.88 |
| Pancreatic Cancer (Stage: All) vs Normal | 279 | 102 | 177 | 9 Huvet markers | <0.0001 | 0.7589 | 0.942 (0.908 to 0.967) | 95.10 | 80.79 |
| | 279 | 102 | 177 | Marker Set 1 (12 markers) | <0.0001 | 0.7547 | 0.947 (0.914 to 0.970) | 94.12 | 81.36 |
| | 278 | 101 | 177 | Marker Set 2 (15 markers) | <0.0001 | 0.7837 | 0.952 (0.920 to 0.974) | 89.11 | 89.27 |
| | 279 | 102 | 177 | Marker Set 3 (8 markers) | <0.0001 | 0.7340 | 0.922 (0.884 to 0.951) | 89.22 | 84.18 |
| | 278 | 101 | 177 | All huvet markers | <0.0001 | 0.7739 | 0.951 (0.919 to 0.974) | 92.08 | 85.31 |

**Description**

**Technical Field**

CROSS-REFERENCE TO RELATED APPLICATION(S)

The present application claims priority to and the benefit of priority based on Korean Patent Application No. 10-2022-0123808, filed on September 28, 2022, the disclosure of which is incorporated herein by reference in its entirety.
The present disclosure relates to a biomarker for diagnosing pancreatic cancer, a composition for diagnosing pancreatic cancer, comprising an agent capable of detecting the biomarker, and a method of diagnosing pancreatic cancer using same.

**Background Art**

A biomarker refers to an indicator that can identify changes induced within a living organism due to external influences and is actively studied for diagnosing various diseases such as cancer and neurological disorders or predicting the efficacy of specific therapeutic agents.
The pancreas is an organ approximately 20 cm in length located behind the stomach that secretes digestive enzymes and hormones. Generally, pancreatic cancer may refer to pancreatic ductal adenocarcinoma. The incidence of pancreatic cancer has been increasing with the widespread adoption of Western-style diets and is known to occur more frequently in men, with a high mortality rate.
Pancreatic cancer is associated with various risk factors, including smoking, coffee consumption, alcohol consumption, a diet rich in meat, diabetes, chronic pancreatitis, and a history of nonpolyposis colorectal cancer syndrome. Additionally, exposure to substances such as beta-naphthylamine and benzidine has been identified as a potential cause.
In the early stages, pancreatic cancer typically presents no noticeable symptoms. Symptoms such as pain and weight loss generally appear only after the cancer has metastasized throughout the body, contributing to its high mortality rate.
To enable more effective treatment of pancreatic cancer, there is a need for the development of technologies for early diagnosis, determination of disease progression, and differentiation from other similar diseases.

**Disclosure**

**Technical Problem**

An embodiment provides a biomarker for diagnosing pancreatic cancer, which comprises at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, PTGES, and coding genes therefor. The biomarker may be for detection or analysis in buffy coat of blood.
Another embodiment provides a composition for diagnosing pancreatic cancer or a kit for diagnosing pancreatic cancer, which comprises an agent capable of detecting at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, PTGES, and coding genes therefor. The composition or kit for diagnosing pancreatic cancer may be for use in buffy coat of blood.
A further embodiment provides a method of diagnosing pancreatic cancer or a method of providing information for diagnosing pancreatic cancer, the method comprising a step of detecting at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, PTGES, and coding genes therefor in a blood sample isolated from a subject. The blood sample may include the buffy coat of blood. The detection step may comprise determining the presence or absence of and/or measuring a level of at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, PTGES, and coding genes therefor in the blood sample.
Still another embodiment provides a use of an agent capable of detecting at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGES2, SLC27A2, PTGES, and coding genes therefor, in diagnosing pancreatic cancer and/or in manufacturing a composition for diagnosis of pancreatic cancer or a kit for diagnosis of pancreatic cancer. The pancreatic cancer diagnosis may be performed using buffy coat of blood, or the composition or kit for diagnosing pancreatic cancer may be for use in buffy coat of blood.
The pancreatic cancer may include early-stage pancreatic cancer, late-stage pancreatic cancer, or both of them.

**Technical Solution**

Below, a detailed description will be given of the present disclosure:

Diagnosis of Pancreatic Cancer

As used herein, the term "pancreatic cancer" collectively refers to tumors occurring in the pancreas, which may include benign tumors such as cystic tumors, for example, such as serous cystic neoplasms, mucinous cystic neoplasms, intraductal papillary mucinous neoplasms, solid pseudopapillary tumors, lymphoepithelial cysts, and cystic teratomas, and malignant tumors, such as pancreatic ductal adenocarcinoma, acinar cell carcinoma, and neuroendocrine tumors. The pancreatic cancer that can be diagnosed using the biomarker provided in the present disclosure may be selected from the aforementioned pancreatic cancers, and, for example, may be selected from malignant tumors, but is not limited thereto.

Additionally, the term "pancreatic cancer," as used herein, may be classified based on its stage of progression and may refer to early-stage pancreatic cancer (e.g., resectable pancreatic cancer) and/or late-stage pancreatic cancer (e.g., borderline resectable pancreatic cancer, locally late-stage pancreatic cancer, or metastatic pancreatic cancer), etc., or may encompass all of them.

As used herein, the term "diagnosis" may include, but is not limited to, determining the susceptibility of a subject to a particular disease or condition, determining whether a subject currently has a specific disease or condition, assessing the risk of developing a specific disease or condition, prognosis determination for a subject with a specific disease or condition (e.g., identifying pre-metastatic or metastatic cancer status, determining cancer stage, evaluating cancer response to treatment, etc.), and/or therametrics, such as monitoring the state of an object to provide information on therapeutic efficacy.

Herein, the term "diagnosis of pancreatic cancer" may refer to identifying the presence, onset likelihood (risk), and/or progression of pancreatic cancer (e.g., early-stage pancreatic cancer, late-stage pancreatic cancer, or both) in a subject, and/or distinguishing pancreatic cancer from normal conditions or other similar diseases (e.g., benign pancreatic diseases (pancreatitis (chronic and/or acute pancreatitis), benign pancreatic tumors, intraductal papillary mucinous neoplasm (IPMN), autoimmune pancreatitis (AIP), other pancreatic disorders (excluding pancreatic malignant tumors), etc., and biliary tract cancer).

Biomarkers for Diagnosing Pancreatic Cancer

Provided as a biomarker for diagnosing pancreatic cancer in the present disclosure is at least one, for example, one or more (e.g., a combination of two, three, four, five, six, seven, eight, nine, ten, eleven, twelve, thirteen, fourteen, fifteen, sixteen, seventeen, eighteen, nineteen, or twenty). The biomarkers described in the present disclosure may refer to at least one of the above proteins and/or a coding gene therefor.

In a specific embodiment, the biomarker for diagnosing pancreatic cancer may be IFNL1, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer), compared to a reference sample (e.g., a normal sample). The biomarker may be used for the diagnosis of pancreatic cancer, including early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be IFNG, a coding gene therefor, or a combination thereof. The biomarker may be characterized by a low (decreased) expression level and/or concentration in pancreatic cancer samples, for example, examples of early-stage pancreatic cancer, late-stage pancreatic cancer, or all pancreatic cancers irrespective of cancer stages (early-stage pancreatic cancer and late-stage pancreatic cancer), compared to a reference sample (e.g., a normal sample). The biomarker may be used for the diagnosis of pancreatic cancer, including early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be CXCL11, a coding gene therefor, or a combination thereof. The biomarker may be characterized by a high (increased) expression level and/or concentration in early-stage pancreatic cancer samples and/or a low (decreased) expression level and/or concentration in late-stage pancreatic cancer samples compared to a reference sample (e.g., a normal sample). The biomarker may be used for the diagnosis of pancreatic cancer, including, for example, early-stage pancreatic cancer and/or late-stage pancreatic cancer.

In another specific embodiment, the biomarker for diagnosing pancreatic cancer may be TNF, a coding gene therefore, or a combination thereof. The biomarker may be characterized by a high (increased) expression level and/or concentration in pancreatic cancer samples, for example, examples of early-stage pancreatic cancer, late-stage pancreatic cancer, or all pancreatic cancers irrespective of cancer stages, compared to a reference sample (e.g., a normal sample). The biomarker may be used for the diagnosis of pancreatic cancer, including early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be CLEC7A, a coding gene therefor, or a combination thereof. The biomarker may be characterized by a high (increased) expression level and/or concentration in late-stage pancreatic cancer samples and/or a low (decreased) expression level and/or concentration in early-stage pancreatic cancer samples compared to a reference sample (e.g., a normal sample). The biomarker may be used for the diagnosis of pancreatic cancer, for example, late-stage pancreatic cancer and/or early-stage pancreatic cancer.

In another specific embodiment, the biomarker for diagnosing pancreatic cancer may be CXCL8, a coding gene therefor, or a combination thereof. The biomarker may be characterized by a high (increased) expression level and/or concentration in pancreatic cancer samples, for example, early-stage pancreatic cancer, late-stage pancreatic cancer, or all pancreatic cancers irrespective of cancer stages, compared to a reference sample (e.g., a normal sample). The biomarker may be used for the diagnosis of pancreatic cancer, including early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be FOXP3, a coding gene therefor, or a combination thereof. The biomarker may be characterized by a low (decreased) expression level and/or concentration in late-stage pancreatic cancer samples and/or a high (increased) expression level and/or concentration in early-stage pancreatic cancer samples compared to a reference sample (e.g., a normal sample). The biomarker may be used for the diagnosis of pancreatic cancer, for example, early-stage pancreatic cancer and/or late-stage pancreatic cancer.

In another specific embodiment, the biomarker for diagnosing pancreatic cancer may be VEGFA, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer), compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be CCL2, a coding gene therefor, or a combination thereof. The biomarker may be characterized by a high (increased) expression level and/or concentration in pancreatic cancer samples, for example, examples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer.

In another specific embodiment, the biomarker for diagnosing pancreatic cancer may be CCL5, a coding gene therefor, or a combination thereof. The biomarker may be characterized by a low (decreased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be CCR5, a coding gene therefor, or a combination thereof. The biomarker may be characterized by a low (decreased) expression level and/or concentration in late-stage pancreatic cancer samples and/or a high (increased) expression level and/or concentration in early-stage pancreatic cancer samples compared to a reference sample (e.g., a normal sample). The biomarker may be used for the diagnosis of pancreatic cancer, for example, early-stage pancreatic cancer and/or late-stage pancreatic cancer.

In another specific embodiment, the biomarker for diagnosing pancreatic cancer may be CXCR4, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be ARG1, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be CXCR2, a coding gene therefor, or a combination thereof. The biomarker may be characterized by a high (increased) expression level and/or concentration in late-stage pancreatic cancer samples and/or a low (decreased) expression level and/or concentration in early-stage pancreatic cancer samples compared to a reference sample (e.g., a normal sample). The biomarker may be used for the diagnosis of pancreatic cancer, for example, early-stage pancreatic cancer and/or late-stage pancreatic cancer.

In another specific embodiment, the biomarker for diagnosing pancreatic cancer may be PTGS2, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be PTGES2, a coding gene therefor, or a combination thereof. The biomarker may be characterized by a low (decreased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be SLC27A2, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be PTGES, a coding gene therefor, or a combination thereof. The biomarker may be characterized by a high (increased) expression level and/or concentration in pancreatic cancer samples, for example, samples of early-stage pancreatic cancer, late-stage pancreatic cancer, or all pancreatic cancers irrespective of cancer stages (early-stage pancreatic cancer and late-stage pancreatic cancer) compared to a reference sample (e.g., a normal sample). The biomarker may be used for the diagnosis of pancreatic cancer, for example, early-stage pancreatic cancer, late-stage pancreatic cancer, or all pancreatic cancers irrespective of cancer stages (early-stage pancreatic cancer and late-stage pancreatic cancer).

In another specific embodiment, the biomarker for diagnosing pancreatic cancer may be at least one (one or two) selected from the group consisting of IFNB1 and IFNA1, a coding gene therefor, or a combination thereof. The biomarker may be characterized by a low (decreased) expression level and/or concentration in pancreatic cancer samples compared to samples from similar diseases (e.g., benign pancreatic diseases (pancreatitis (chronic and/or acute pancreatitis), benign pancreatic tumors, intraductal papillary mucinous neoplasm (IPMN), autoimmune pancreatitis (AIP), other pancreatic disorders (excluding pancreatic malignant tumors), etc.). The biomarker may be used for the diagnosis of pancreatic cancer, particularly early-stage pancreatic cancer, and/or for distinguishing pancreatic cancer from benign pancreatic diseases (e.g., pancreatitis (chronic pancreatitis and/or acute pancreatitis), benign pancreatic tumors, intraductal papillary mucinous neoplasm (IPMN), autoimmune pancreatitis (AIP), and other pancreatic diseases (excluding pancreatic cancer (pancreatic malignant tumors) and/or cholangiocarcinoma.

Twenty types of the biomarkers may be derived from mammals such as primates including humans, monkeys, etc., and rodents such as mice, rats, etc., but with no limitations thereto.

Details of the biomarkers are given in Table 1, below.

TABLE 1

| Marker name | Representative example (gene) | Representative example (protein) | Full Name / Synonym |
|---|---|---|---|
| IFNL1(INFλ1) | NM_172140 | NP_742152 | Interferon lambda 1 / Interleukin-29 (IL-29) |
| IFNG | NM_000619 | NP_000610 | Interferon gamma |
| CXCL11 | NM_005409 | NP_005400 | C-X-C motif chemokine ligand 11 / I-TAC, IP-9 |
| TNF | NM_000594 | NP_000585 | Tumor necrosis factor / TNF-alpha |
| IFNB1 | NM_002176 | NP_002167 | Interferon beta 1 |
| IFNA1 | NM_024013 | NP_076911 | Interferon alpha 1 |
| CLEC7A | NM_022570 | NP_072092 | C-type lectin domain containing 7A / DECTIN1 |
| CXCL8 | NM_000584 | NP_000575 | C-X-C motif chemokine ligand 8 / IL8, NAF, GCP1 |
| FOXP3 | NM_014009 | NP_054728 | Forkhead box P3 |
| VEGFA | NM_003376 | NP_003367 | Vascular endothelial growth factor A / VEGF, MVCD1, VPF |
| CCL2 | NM_002982 | NP_002973 | C-C motif chemokine ligand 2 / MCP1, SCYA2 |
| CCL5 | NM_002985 | NP_002976 | C-C motif chemokine ligand 5 / RANTES, SCYA5 |
| CCR5 | NM_000579 | NP_000570 | C-C motif chemokine receptor 5 / CKR5, CD195, CCCKR5 |
| CXCR4 | NM_003467 | NP_003458 | C-X-C motif chemokine receptor 4 / LESTR, CD184, Fusin |
| ARG1 | NM_001244438 | NP_001231367 | Arginase 1 |
| CXCR2 | NM_001557 | NP_001548 | C-X-C motif chemokine receptor 2 / CD182, IL8R2, IL8RA |
| PTGS2 | NM_000963 | NP_000954 | Prostaglandin-endoperoxide synthase 2 / COX2, PHS-2 |
| PTGES2 | NM_198938 | NP_945176 | Prostaglandin E synthase 2 / GBF1, mPGES-2 |
| SLC27A2 | NM_003645 | NP_003636 | Solute carrier family 27 member 2 / FATP2, VLCS |
| PTGES | NM_004878 | NP_004869 | Prostaglandin E synthase / PGES, MPGES, mPGES-1 |

Agent Capable of Detecting Biomarker

As used herein, the term "detection of a biomarker" may refer to determining the presence or absence of the biomarker and/or measuring level (concentration) of the biomarker in a sample.

Herein, an agent capable for detecting at least one biomarker selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES, and coding genes therefor (e.g., one or a combination of two or more (e.g., 2, 3, 4, 5, 6, 7, 8, 9, 10, 11, 12, 13, 14, 15, 16, 17, 18, 19, or 20) selected from the group) may be selected from small molecule compounds, proteins, and nucleic acid molecules capable of binding to the biomarker.

In an embodiment, when the biomarker is at least one protein selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES, the agent capable for detecting the biomarker may be selected from the group consisting of: proteins (e.g., antibodies, antigen-binding fragments of antibodies, antibody mimetics containing antigen-binding fragments, receptors, etc.), peptides, nucleic acid molecules (e.g., polynucleotides, oligonucleotides, etc.), and small molecule compounds (chemicals, small molecules), which all bind to the biomarker, but with no limitations thereto.

In another embodiment, when the biomarker is at least one gene selected from the group consisting of coding genes for IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20) genes (full-length DNA, cDNA, or mRNA) selected from the group), the agent capable of detecting the biomarker may be selected from the group consisting of: nucleic acid molecules (e.g., oligonucleotides, polynucleotides, primers, probes, aptamers, antisense oligonucleotides, etc.), small molecule compounds, proteins, and peptides, which are all capable of binding (or hybridizing) to the biomarker gene, but with no limitations thereto.

The detectable agents for the biomarkers may be labeled or unlabeled with conventional labeling substances such as fluorescent substances, chromogenic substances, luminescent substances, radioactive isotopes, or heavy metals.

In an embodiment, when the biomarker is at least one protein selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20) proteins selected from the group), detection of the biomarker may be performed using conventional protein detection (or measurement or analysis) methods such as enzymatic reactions, fluorescence, luminescence, and/or radiation detection. Specifically, the detection may be carried out using a method selected from the group consisting of: immunochromatography, immunohistochemical staining, enzyme-linked immunosorbent assay (ELISA), radioimmunoassay (RIA), enzyme immunoassay (EIA), fluorescence immunoassay (FIA), luminescence immunoassay (LIA), western blotting, microarray method, and flow cytometry, but is not limited thereto.

In another embodiment, when the biomarker is at least one gene (full-length DNA, cDNA, or mRNA) selected from the group consisting of coding genes for IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20) genes selected from the group), detection of the biomarker may be performed using conventional gene detection (or measurement or analysis) methods. For example, conventional gene analysis methods using primers, probes, aptamers, or antisense oligonucleotides that can hybridize to the gene may be employed. Specifically, detection may be performed using polymerase chain reaction (PCR) (e.g., qPCR, real-time PCR, real-time qPCR), fluorescent in situ hybridization (FISH), southern blotting, and microarray methods, but with no limitations thereto. In a specific embodiment, the primer may detect a consecutive 5 to 1000 bp sequence within the gene (full-length DNA, cDNA, or mRNA), such as 10 to 500 bp, 20 to 200 bp, or 50 to 200 bp. The primer pair may include sequences hybridizable (e.g., complementary) to the consecutive 5' and 3' terminal sequences of 5 to 100 bp within the gene fragment, such as 5 to 50 bp, 5 to 30 bp, or 10 to 25 bp.

The probe, aptamer, or antisense oligonucleotide may have a total length of 5 to 1000 bp, 5 to 500 bp, 5 to 200 bp, 5 to 100 bp, 5 to 50 bp, 5 to 30 bp, or 5 to 25 bp. These molecules may have complementary sequences that can bind or hybridize to a consecutive 5 to 1000 bp, 5 to 500 bp, 5 to 200 bp, 5 to 100 bp, 5 to 50 bp, 5 to 30 bp, or 5 to 25 bp sequence within the marker gene (full-length DNA, cDNA, or mRNA). The term "capable of binding" may refer to the ability to bind to all or part of the gene through chemical and/or physical interactions such as covalent bonding. The term "capable of hybridizing" may refer to the ability to form complementary binding with at least 80% sequence complementarity, for example, 90% or more, 95% or more, 98% or more, 99% or more, or 100%.

Detection of Biomarker

Herein, as described in the foregoing, the term "detection of a biomarker" may refer to determining the presence or absence and/or measuring level (concentration) of the biomarker.

When the biomarker is a protein, the detection thereof may be performed by a conventional protein detection method, for example, a method selected from the group consisting of immunochromatography, immunohistochemical staining, enzyme-linked immunosorbent assay (ELISA), radioimmunoassay (RIA), enzyme immunoassay (EIA), fluorescence immunoassay (FIA), luminescence immunoassay (LIA), western blotting, microarray techniques, and flow cytometry, but with no limitations thereto.

When the biomarker is a gene, the detection thereof may be performed by a conventional gene detection method, for example, a method selected from the group consisting of polymerase chain reaction (PCR) (e.g., qPCR, real-time PCR, real-time qPCR, etc.), fluorescent in situ hybridization (FISH), southern blotting, microarray techniques, but with no limitations thereto.

In the present disclosure, "measurement of a level of a biomarker" may be performed by qualitative and/or quantitative analysis of the results obtained from the above-mentioned biomarker detection methods (for proteins and/or genes).

In an embodiment, when a labeled detection agent is used, the measurement of a level of the biomarker may be performed by qualitatively analyzing the signal intensity (e.g., comparing the intensity or area of fluorescence and/or luminescence, or the thickness and/or darkness of a gel electrophoresis band relative to a control sample) by conventional methods, or by quantitatively analyzing (e.g., by numerically evaluating the intensity or area of fluorescence and/or luminescence, or the thickness and/or darkness of a gel electrophoresis band) by conventional methods, but with no limitations thereto.

In another embodiment, when conventional polymerase chain reaction (PCR; e.g., qPCR, real-time PCR, real-time qPCR, etc.) is employed, the measurement of a level of the biomarker may be performed using conventional quantitative PCR methods, such as by measuring the Ct (cycle threshold) value, $\Delta$Ct value ($\Delta$Ct = Ct (Gene) - Ct (Control gene)), and/or $\Delta\Delta$Ct value ($\Delta\Delta$Ct = $\Delta$Ct (Target gene) - $\Delta$Ct (Reference gene)), but with no limitations thereto.

In yet another embodiment, the measurement of a level of the biomarker may further include an additional step of performing conventional statistical analysis on the quantified biomarker levels (e.g., Ct, $\Delta$Ct, and/or $\Delta\Delta$Ct values in the case of PCR). Here, the statistical analysis is intended to encompass all conventional statistical analysis tools, including various machine learning models and/or algorithms. Examples of such statistical analysis include, but are not limited to, regression analysis (e.g., logistic regression, stepwise logistic regression, etc.), Ensemble, Decision Tree, Random Forest, Gradient Boosting, XGBoost, Light GBM (Light Gradient Boosting Machine), Gaussian Naive Bayes, SVM (Support Vector Machine), Bagging (Bootstrap Aggregating), Boosting, and the like. For example, the statistical analysis may be performed using regression analysis (e.g., logistic regression, stepwise logistic regression, etc.) with additional appropriate statistical analyses (e.g., one or more of the aforementioned methods) as necessary.

Composition and Kit for Diagnosing Pancreatic Cancer

The composition for diagnosing pancreatic cancer provided herein may include an agent capable of detecting the biomarker, described above.

The kit for diagnosing pancreatic cancer (also referred to as a biosensor) provided herein may include an agent capable of detecting the biomarker or a composition for diagnosing pancreatic cancer that contains such an agent. In an embodiment, the kit for diagnosing pancreatic cancer may include the agent capable of detecting agent for the biomarker or the composition for diagnosing pancreatic cancer in a conventional format such as a microarray or panel.

The kit for diagnosing pancreatic cancer may further include a detection means. The detection means may be a tool capable of qualitatively and/or quantitatively analyzing the presence and/or level of the biomarker detected by the agent capable of detecting the biomarker. In an embodiment, the detection means may be selected from the protein and/or gene detection methods described in the foregoing.

In an embodiment, the kit may be a RT-PCR kit, a DNA chip kit, an ELISA kit, a protein chip kit, a rapid diagnostic kit, or a multiple reaction monitoring (MRM) kit, but is not limited thereto.

In a specific embodiment, the composition and/or kit for diagnosing pancreatic cancer may comprise an agent capable of detecting at least one biomarker selected from the group consisting of: IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20 selected from the group)); a coding gene therefor, or a combination thereof. The pancreatic cancer that can be diagnosed using the composition or kit may be early-stage pancreatic cancer, late-stage pancreatic cancer, or both.

In another embodiment, the composition and/or kit for diagnosing pancreatic cancer may comprise an agent capable of detecting: at least one selected from the group consisting of IFNL1, IFNG, CXCL11, TNF, CLEC7A, CXCL8, FOXP3, VEGFA, CCL2, CCL5, CCR5, CXCR4, ARG1, CXCR2, PTGS2, PTGES2, SLC27A2, PTGES, IFNB1, and IFNA1 (for example, one or more (e.g., 2, 3, 4, 5, 6, 7, 8, 9, 10, 11, 12, 13, 14, 15, 16, 17, 18, 19, or 20) selected from the group) or at least one selected from the group consisting of IFNL1, IFNG, CXCL11, TNF, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, or 18) selected from the group); a coding gene therefor; or combinations thereof. In this regard, the pancreatic cancer that can be diagnosed using the composition or kit may be early-stage pancreatic cancer, late-stage pancreatic cancer, or both.

In an embodiment, the composition and/or kit for diagnosing pancreatic cancer may comprise at least one agent capable of detecting at least one biomarker selected from the following groups:

at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

at least one selected from the group consisting of SLC27A2, IFNG, CCL2, TNF, PTGES, CLEC7A, ARG1, PTGES2, FOXP3, CXCR4, VEGFA, CXCR2, and PTGS2 (for example, one or more (e.g., 2, 3, 4, 5, 6, 7, 8, 9, 10, 11, 12, or 13) selected from the group), a coding gene therefor, or a combination thereof;

at least one selected from the group consisting of IFNG, SLC27A2, CCL5, FOXP3, PTGES2, TNF, IFNA1, IFNL1, CCL2, CXCR2, PTGES, CXCR4, and IFNB1 (for example, one or more (e.g., 2, 3, 4, 5, 6, 7, 8, 9, 10, 11, 12, or 13) selected from the group), a coding gene therefor, or a combination thereof;

at least one selected from the group consisting of SLC27A2, IFNG, CCL2, TNF, PTGES, FOXP3, PTGES2, CXCR4, and CXCR2 (for example, one or more (e.g., 2, 3, 4, 5, 6, 7, 8, or 9) selected from the group), a coding gene therefor, or a combination thereof;

at least one selected from the group consisting of IFNL1, IFNG, CXCL11, CCL2, PTGES2, SLC27A2, and PTGES (for example, one or more (e.g., 2, 3, 4, 5, 6, or 7) selected from the group), a coding gene therefor, or a combination thereof;

at least one selected from the group consisting of ARG1, CCL2, CLEC7A, SLC27A2, IFNG, PTGES, PTGES2, TNF, and FOXP3 (for example, one or more (e.g., 2, 3, 4, 5, 6, 7, 8, or 9) selected from the group), a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof;

at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof;

at least one selected from the group consisting of TNF, IFNG, IFNL1, CXCL11, CLEC7A, VEGFA, CCL2, CXCR4, CXCR2, ARG1, SLC27A2, PTGES2, and PTGES (for example, one or more (e.g., 2, 3, 4, 5, 6, 7, 8, 9, 10, 11, 12, or 13) selected from the group), a coding gene therefor, or a combination thereof;

at least one selected from the group consisting of TNF, IFNG, CCL2, CXCR2, ARG1, SLC27A2, PTGES2, and PTGES (for example, one or more (e.g., 2, 3, 4, 5, 6, 7, or 8) selected from the group), a coding gene therefor, or a combination thereof;

at least one selected from the group consisting of VEGFA, CXCR4, SLC27A2, TNF, PTGES2, CCL5, CXCL11, CXCR2, FOXP3, PTGS2, and CXCL8 (for example, one or more (e.g., 2, 3, 4, 5, 6, 7, 8, 9, 10, or 11) selected from the group), a coding gene therefor, or a combination thereof;

at least one selected from the group consisting of PTGES2, IFNG, CCL5, VEGFA, PTGS2, CLEC7A, CXCR4, TNF, FOXP3, CCL2, CXCR2, CXCL8, PTGES, IFNL1, and SLC27A2 (for example, one or more (e.g., 2, 3, 4, 5, 6, 7, 8, 9, 10, 11, 12, 13, 14, or 15) selected from the group), a coding gene therefor, or a combination thereof;

at least one selected from the group consisting of VEGFA, CXCR4, SLC27A2, TNF, PTGES2, CCL5, CXCR2, FOXP3, PTGS2, and CXCL8 (for example, one or more (e.g., 2, 3, 4, 5, 6, 7, 8, 9, or 10) selected from the group), a coding gene therefor, or a combination thereof;

at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2 (for example, one or more (e.g., 2, 3, 4, 5, 6, 7, 8, 9, 10, 11, 12, 13, or 14) selected from the group), a coding gene therefor, or a combination thereof;

at least one selected from the group consisting of CCL5, PTGS2, CXCR2, CXCR4, SLC27A2, CXCL11, PTGES2, TNF, and VEGFA (for example, one or more (e.g., 2, 3, 4, 5, 6, 7, 8, or 9) selected from the group), a coding gene therefor, or a combination thereof;

at least one selected from the group consisting of SLC27A2, PTGES2, VEGFA, TNF, CXCR4, IFNG, CCL5, CXCR2, CCL2, CXCL11, CLEC7A, and ARG1 (for example, one or more (e.g., 2, 3, 4, 5, 6, 7, 8, 9, 10, 11, or 12,) selected from the group), a coding gene therefor, or a combination thereof;

at least one selected from the group consisting of IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2 (for example, one or more (e.g., 2, 3, 4, 5, 6, 7, 8, 9, 10, 11, 12, 13, 14, 15, 16, or 17) selected from the group), a coding gene therefor, or a combination thereof;

at least one selected from the group consisting of SLC27A2, PTGES2, VEGFA, TNF, CXCR4, IFNG, CCL5, CXCR2, CCL2, and CLEC7A (for example, one or more (e.g., 2, 3, 4, 5, 6, 7, 8, 9, or 10) selected from the group), a coding gene therefor, or a combination thereof;

at least one selected from the group consisting of IFNG, CCL2, and PTGES2 (for example, one, two, or three selected from the group), a coding gene therefor, or a combination thereof;

at least one selected from the group consisting of ARG1, CCL2, SLC27A2, IFNG, PTGES2, TNF, VEGFA, CXCR4, and CCL5 (for example, one or more (e.g., 2, 3, 4, 5, 6, 7, 8, or 9) selected from the group), a coding gene therefor, or a combination thereof;

at least one selected from the group consisting of TNF, IFNG, CXCL11, VEGFA, CCL2, CCL5, CXCR4, CXCR2, PTGS2, SLC27A2, and PTGES2 (for example, one or more (e.g., 2, 3, 4, 5, 6, 7, 8, 9, 10, or 11) selected from the group), a coding gene therefor, or a combination thereof; and

at least one selected from the group consisting of IFNG, VEGFA, SLC27A2, PTGES2, TNF, CXCR4, CXCR2, CCL5, CCL2, and CXCL11 (for example, one or more (e.g., 2, 3, 4, 5, 6, 7, 8, 9, or 10) selected from the group), a coding gene therefor, or a combination thereof.

For instance, the composition and/or kit for diagnosing pancreatic cancer may include an agent capable of detecting a biomarker set selected from the following groups, a coding gene therefor; or a combination thereof:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;
SLC27A2, IFNG, CCL2, TNF, PTGES, CLEC7A, ARG1, PTGES2, FOXP3, CXCR4, VEGFA, CXCR2, and PTGS2;
IFNG, SLC27A2, CCL5, FOXP3, PTGES2, TNF, IFNA1, IFNL1, CCL2, CXCR2, PTGES, CXCR4, and IFNB1;
SLC27A2, IFNG, CCL2, TNF, PTGES, FOXP3, PTGES2, CXCR4, and CXCR2;
IFNL1, IFNG, CXCL11, CCL2, PTGES2, SLC27A2, and PTGES;
ARG1, CCL2, CLEC7A, SLC27A2, IFNG, PTGES, PTGES2, TNF, and FOXP3;
CCL5, CXCR4, SLC27A2, PTGES2, TNF, VEGFA, ARG1, CCL2, CLEC7A, IFNG, PTGES, and FOXP3;
CCL5, CXCR4, SLC27A2, PTGES2, TNF, VEGFA, ARG1, CCL2, CLEC7A, IFNG, PTGES, FOXP3, PTGS2, CXCR2, and CXCL11;
CCL2, CCL5, CXCR2, SLC27A2, IFNL1, IFNG, PTGES2, and TNF;
TNF, IFNG, IFNL1, CXCL11, CLEC7A, VEGFA, CCL2, CXCR4, CXCR2, ARG1, SLC27A2, PTGES2, and PTGES;
TNF, IFNG, CCL2, CXCR2, ARG1, SLC27A2, PTGES2, and PTGES;
VEGFA, CXCR4, SLC27A2, TNF, PTGES2, CCL5, CXCL11, CXCR2, FOXP3, PTGS2, and CXCL8;
PTGES2, IFNG, CCL5, VEGFA, PTGS2, CLEC7A, CXCR4, TNF, FOXP3, CCL2, CXCR2, CXCL8, PTGES, IFNL1, and SLC27A2;
VEGFA, CXCR4, SLC27A2, TNF, PTGES2, CCL5, CXCR2, FOXP3, PTGS2, and CXCL8;
IFNG, TNF, CLEC7A, CXCL8, FOXP3, VEGFA, CCL2, CCL5, CCR5, CXCR4, ARG1, CXCR2, PTGS2, and PTGES2;
CCL5, PTGS2, CXCR2, CXCR4, SLC27A2, CXCL11, PTGES2, TNF, and VEGFA;
SLC27A2, PTGES2, VEGFA, TNF, CXCR4, IFNG, CCL5, CXCR2, CCL2, CXCL11, CLEC7A, and ARG1;
IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2;
SLC27A2, PTGES2, VEGFA, TNF, CXCR4, IFNG, CCL5, CXCR2, CCL2, and CLEC7A;
IFNG, CCL2, and PTGES2;
ARG1, CCL2, SLC27A2, IFNG, PTGES2, TNF, VEGFA, CXCR4, and CCL5;
TNF, IFNG, CXCL11, VEGFA, CCL2, CCL5, CXCR4, CXCR2, PTGS2, SLC27A2, and PTGES2; and
IFNG, VEGFA, SLC27A2, PTGES2, TNF, CXCR4, CXCR2, CCL5, CCL2, and CXCL11.

More specifically, the composition and/or kit for diagnosing pancreatic cancer may include an agent capable of detecting at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20) selected from the group); a coding gene thereof; or a combination thereof. In this regard, the pancreatic cancer that can be detected with the composition or kit may be early-stage pancreatic cancer.

In an embodiment, the composition or kit for diagnosing pancreatic cancer is for use in diagnosing early-stage pancreatic cancer and may include at least one selected from the group consisting of:

(1-1) an agent capable of detecting at least one selected from the group consisting of SLC27A2, IFNG, CCL2, TNF, PTGES, CLEC7A, ARG1, PTGES2, FOXP3, CXCR4, VEGFA, CXCR2, and PTGS2 (for example, one or more (e.g., 2, 3, 4, 5, 6, 7, 8, 9, 10, 11, 12, or 13) selected from the group), a coding gene therefor, or a combination thereof;
(1-2) an agent capable of detecting at least one selected from the group consisting of IFNG, SLC27A2, CCL5, FOXP3, PTGES2, TNF, IFNA1, IFNL1, CCL2, CXCR2, PTGES, CXCR4, and IFNB1 (for example, one or more (e.g., 2, 3, 4, 5, 6, 7, 8, 9, 10, 11, 12, or 13) selected from the group), a coding gene therefor, or a combination thereof;
(1-3) an agent capable of detecting at least one selected from the group consisting of SLC27A2, IFNG, CCL2, TNF, PTGES, FOXP3, PTGES2, CXCR4, and CXCR2 (for example, one or more (e.g., 2, 3, 4, 5, 6, 7, 8, or 9) selected from the group), a coding gene therefor, or a combination thereof;

(1-4) an agent capable of detecting at least one selected from the group consisting of IFNL1, IFNG, CXCL11, CCL2, PTGES2, SLC27A2, and PTGES (for example, one or more (e.g., 2, 3, 4, 5, 6, or 7) selected from the group), a coding gene therefor, or a combination thereof;

(1-5) an agent capable of detecting at least one selected from the group consisting of ARG1, CCL2, CLEC7A, SLC27A2, IFNG, PTGES, PTGES2, TNF, and FOXP3 (for example, one or more (e.g., 2, 3, 4, 5, 6, 7, 8, or 9) selected from the group), a coding gene therefor, or a combination thereof;

(1-6) an agent capable of detecting at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

(1-7) an agent capable of detecting at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group); a coding gene therefor, or a combination thereof;

(1-8) an agent capable of detecting at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof;

(1-9) an agent capable of detecting at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof;

(1-10) an agent capable of detecting at least one selected from the group consisting of TNF, IFNG, IFNL1, CXCL11, CLEC7A, VEGFA, CCL2, CXCR4, CXCR2, ARG1, SLC27A2, PTGES2, and PTGES (for example, one or more (e.g., 2, 3, 4, 5, 6, 7, 8, 9, 10, 11, 12, or 13) selected from the group), a coding gene therefor, or a combination thereof; and

(1-11) an agent capable of detecting at least one selected from the group consisting of TNF, IFNG, CCL2, CXCR2, ARG1, SLC27A2, PTGES2, and PTGES (for example, one or more (e.g., 2, 3, 4, 5, 6, 7, or 8) selected from the group), a coding gene therefor, or a combination thereof.

For instance, the composition and/or kit for diagnosing pancreatic cancer may include an agent capable of detecting a biomarker set selected from the following sets; a coding gene therefor; or a combination thereof. In this regard, the pancreatic cancer that can be diagnosed with the composition and/or kit may be early-stage pancreatic cancer:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;
SLC27A2, IFNG, CCL2, TNF, PTGES, CLEC7A, ARG1, PTGES2, FOXP3, CXCR4, VEGFA, CXCR2, and PTGS2;
IFNG, SLC27A2, CCL5, FOXP3, PTGES2, TNF, IFNA1, IFNL1, CCL2, CXCR2, PTGES, CXCR4, and IFNB1;
SLC27A2, IFNG, CCL2, TNF, PTGES, FOXP3, PTGES2, CXCR4, and CXCR2;
IFNL1, IFNG, CXCL11, CCL2, PTGES2, SLC27A2, and PTGES;
ARG1, CCL2, CLEC7A, SLC27A2, IFNG, PTGES, PTGES2, TNF, and FOXP3;
CCL5, CXCR4, SLC27A2, PTGES2, TNF, VEGFA, ARG1, CCL2, CLEC7A, IFNG, PTGES, and FOXP3;
CCL5, CXCR4, SLC27A2, PTGES2, TNF, VEGFA, ARG1, CCL2, CLEC7A, IFNG, PTGES, FOXP3, PTGS2, CXCR2, and CXCL11;
CCL2, CCL5, CXCR2, SLC27A2, IFNL1, IFNG, PTGES2, and TNF;
TNF, IFNG, IFNL1, CXCL11, CLEC7A, VEGFA, CCL2, CXCR4, CXCR2, ARG1, SLC27A2, PTGES2, and PTGES; and
TNF, IFNG, CCL2, CXCR2, ARG1, SLC27A2, PTGES2, and PTGES.

In another embodiment, the composition and/or kit for diagnosing pancreatic cancer may include an agent capable of detecting: at least one selected from the group consisting of IFNL1, IFNG, CXCL11, TNF, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (2, 3, 4, 5, 6, 7, 8, 9, 10, 11, 12, 13, 14, 15, 16, 17, or 18) selected from the group); a coding gene therefor; or a combination thereof. In this regard, the pancreatic cancer that can be diagnosed using the composition and/or kt may be late-stage pancreatic cancer.

In an embodiment, the composition and/or kit for diagnosing pancreatic cancer is for use in diagnosis of late-stage pancreatic cancer and may include at least one agent capable of detecting:

(2-1) at least one selected from the group consisting of VEGFA, CXCR4, SLC27A2, TNF, PTGES2, CCL5, CXCL11, CXCR2, FOXP3, PTGS2, and CXCL8 (for example, one or more (e.g., 2, 3, 4, 5, 6, 7, 8, 9, 10, or 11) selected from the group), a coding gene therefor, or a combination thereof;

(2-2) at least one selected from the group consisting of PTGES2, IFNG, CCL5, VEGFA, PTGS2, CLEC7A, CXCR4, TNF, FOXP3, CCL2, CXCR2, CXCL8, PTGES, IFNL1, and SLC27A2 (for example, one or more (e.g., 2, 3, 4, 5, 6, 7, 8, 9, 10, 11, 12, 13, 14, or 15) selected from the group); a coding gene therefor, or a combination thereof;

(2-3) at least one selected from the group consisting of VEGFA, CXCR4, SLC27A2, TNF, PTGES2, CCL5, CXCR2, FOXP3, PTGS2, and CXCL8 (for example, one or more (e.g., 2, 3, 4, 5, 6, 7, 8, 9, or 10) selected from the group); a coding gene therefor, or a combination thereof;

(2-4) at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2 (for example, one or more (e.g., 2, 3, 4, 5, 6, 7, 8, 9, 10, 11, 12, 13, or 14) selected from the group); a coding gene therefor, or a combination thereof;

(2-5) at least one selected from the group consisting of CCL5, PTGS2, CXCR2, CXCR4, SLC27A2, CXCL11, PTGES2, TNF, and VEGFA (for example, one or more (e.g., 2, 3, 4, 5, 6, 7, 8, or 9) selected from the group), a coding gene therefor, or a combination thereof;

(2-6) at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

(2-7) at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

(2-8) at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof; and

(2-9) at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof.

For instance, the composition and/or kit for diagnosing pancreatic cancer may include an agent capable of detecting a biomarker set selected from the following sets; a coding gene therefor; or a combination thereof. In this regard, the pancreatic cancer that can be diagnosed with the composition and/or kit may be late-stage pancreatic cancer:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;
VEGFA, CXCR4, SLC27A2, TNF, PTGES2, CCL5, CXCL11, CXCR2, FOXP3, PTGS2, and CXCL8;
PTGES2, IFNG, CCL5, VEGFA, PTGS2, CLEC7A, CXCR4, TNF, FOXP3, CCL2, CXCR2, CXCL8, PTGES, IFNL1, and SLC27A2;
VEGFA, CXCR4, SLC27A2, TNF, PTGES2, CCL5, CXCR2, FOXP3, PTGS2, and CXCL8;
IFNG, TNF, CLEC7A, CXCL8, FOXP3, VEGFA, CCL2, CCL5, CCR5, CXCR4, ARG1, CXCR2, PTGS2, and PTGES2;
CCL5, PTGS2, CXCR2, CXCR4, SLC27A2, CXCL11, PTGES2, TNF, and VEGFA;
CCL5, CXCR4, SLC27A2, PTGES2, TNF, VEGFA, ARG1, CCL2, CLEC7A, IFNG, PTGES, and FOXP3;
CCL5, CXCR4, SLC27A2, PTGES2, TNF, VEGFA, ARG1, CCL2, CLEC7A, IFNG, PTGES, FOXP3, PTGS2, CXCR2, and CXCL11; and
CCL2, CCL5, CXCR2, SLC27A2, IFNL1, IFNG, PTGES2, and TNF.

In another embodiment, the composition and/or kit for diagnosing pancreatic cancer may include an agent capable of detecting: at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (2, 3, 4, 5, 6, 7, 8, 9, 10, 11, 12, 13, 14, 15, 16, 17, 18, 19, or 20) selected from the group); coding genes thereof; or a combination thereof. In this regard, the pancreatic cancer that can be detected using the composition or kit may be a pancreatic cancer at any stage regardless of progression, for example, early-stage pancreatic cancer, late-stage pancreatic cancer, or both of them.

In an embodiment, the composition and/or kit for diagnosing pancreatic cancer is for use in diagnosis of all-stage pancreatic cancers and may include at least one detectable agent selected from the group consisting of:

(3-1) an agent capable of detecting at least one selected from the group consisting of SLC27A2, PTGES2, VEGFA, TNF, CXCR4, IFNG, CCL5, CXCR2, CCL2, CXCL11, CLEC7A, and ARG1 (for example, one or more (e.g., 2, 3, 4, 5, 6, 7, 8, 9, 10, 11, or 12) selected from the group), a coding gene therefor, or a combination thereof;

(3-2) an agent capable of detecting at least one selected from the group consisting of IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2 (for example, one or more (e.g., 2, 3, 4, 5, 6, 7, 8, 9, 10, 11, 12, 13, 14, 15, 16, or 17) selected from the group), a coding gene therefor, or a combination thereof;

(3-3) an agent capable of detecting at least one selected from the group consisting of SLC27A2, PTGES2, VEGFA, TNF, CXCR4, IFNG, CCL5, CXCR2, CCL2, and CLEC7A (for example, one or more (e.g., 2, 3, 4, 5, 6, 7, 8, 9, or 10) selected from the group), a coding gene therefor, or a combination thereof;

(3-4) an agent capable of detecting at least one selected from the group consisting of IFNG, CCL2, and PTGES2 (for example, one, two, or three selected from the group), a coding gene therefor, or a combination thereof;

(3-5) an agent capable of detecting at least one selected from the group consisting of ARG1, CCL2, SLC27A2, IFNG, PTGES2, TNF, VEGFA, CXCR4, and CCL5 (for example, one or more (e.g., 2, 3, 4, 5, 6, 7, 8, or 9) selected from the group), a coding gene therefor, or a combination thereof;

(3-6) an agent capable of detecting at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), coding genes thereof, or a combination thereof;

(3-7) an agent capable of detecting at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

(3-8) an agent capable of detecting at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof;

(3-9) an agent capable of detecting at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof;

(3-10) an agent capable of detecting at least one selected from the group consisting of TNF, IFNG, CXCL11, VEGFA, CCL2, CCL5, CXCR4, CXCR2, PTGS2, SLC27A2, and PTGES2 (for example, one or more (e.g., 2, 3, 4, 5, 6, 7, 8, 9, 10, or 11) selected from the group), a coding gene therefor, or a combination thereof; or

(3-11) an agent capable of detecting at least one selected from the group consisting of IFNG, VEGFA, SLC27A2, PTGES2, TNF, CXCR4, CXCR2, CCL5, CCL2, and CXCL11 (for example, one or more (e.g., 2, 3, 4, 5, 6, 7, 8, 9, or 10) selected from the group), a coding gene therefor, or a combination thereof. In this regard, the pancreatic cancer that can be detected using the composition or kit may be pancreatic cancer at any stage regardless of progression, for example, early-stage pancreatic cancer, late-stage pancreatic cancer, or both.

For instance, the composition and/or kit for diagnosing pancreatic cancer may include an agent capable of detecting a biomarker set selected from the following sets; a coding gene therefor; or a combination thereof. In this regard, the pancreatic cancer that can be diagnosed using the composition and/or kit may be early-stage pancreatic cancer, late-early pancreatic cancer, or both of them:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;
SLC27A2, PTGES2, VEGFA, TNF, CXCR4, IFNG, CCL5, CXCR2, CCL2, CXCL11, CLEC7A, and ARG1;
IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2;
SLC27A2, PTGES2, VEGFA, TNF, CXCR4, IFNG, CCL5, CXCR2, CCL2, and CLEC7A;
IFNG, CCL2, and PTGES2;
ARG1, CCL2, SLC27A2, IFNG, PTGES2, TNF, VEGFA, CXCR4, and CCL5;
CCL5, CXCR4, SLC27A2, PTGES2, TNF, VEGFA, ARG1, CCL2, CLEC7A, IFNG, PTGES, and FOXP3;
CCL5, CXCR4, SLC27A2, PTGES2, TNF, VEGFA, ARG1, CCL2, CLEC7A, IFNG, PTGES, FOXP3, PTGS2, CXCR2, and CXCL11;
CCL2, CCL5, CXCR2, SLC27A2, IFNL1, IFNG, PTGES2, and TNF;
TNF, IFNG, CXCL11, VEGFA, CCL2, CCL5, CXCR4, CXCR2, PTGS2, SLC27A2, and PTGES2; and
IFNG, VEGFA, SLC27A2, PTGES2, TNF, CXCR4, CXCR2, CCL5, CCL2, and CXCL11.

In another embodiment, the composition and/or kit for diagnosing pancreatic cancer may include an agent capable of detecting at least one biomarker selected from a group consisting of IFNB1 and IFNA1 (one or both), coding genes thereof, or a combination thereof. In this regard, the diagnostic composition and/or kit may be designed to differentiate pancreatic cancer (e.g., early-stage pancreatic cancer and/or late-stage pancreatic cancer) from similar diseases (e.g., benign pancreatic diseases (pancreatitis (chronic pancreatitis and/or acute pancreatitis), benign pancreatic tumors, intraductal papillary mucinous neoplasm (IPMN), autoimmune pancreatitis (AIP), and other pancreatic diseases (excluding pancreatic cancer (malignant pancreatic tumors), etc.) and cholangiocarcinoma).

The composition and/or kit for diagnosing pancreatic cancer may include at least one of the biomarkers IFNB1 and IFNA1, their coding genes, or a combination thereof, to effectively distinguish pancreatic cancer from benign pancreatic diseases and other similar conditions.

Method for Diagnosing Pancreatic Cancer

The present disclosure provides a composition and method for diagnosing pancreatic cancer (or a method for providing information for diagnosis). The method may include a step of detecting the biomarker described above, that is, at least one biomarker selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20) selected from the group), and a coding gene therefor in a blood sample isolated from a subject.

In an embodiment, the biomarker may include at least one selected from the following:

at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

at least one selected from the group consisting of SLC27A2, IFNG, CCL2, TNF, PTGES, CLEC7A, ARG1, PTGES2, FOXP3, CXCR4, VEGFA, CXCR2, and PTGS2 ((for example, one or more (e.g., 2, 3, 4, 5, 6, 7, 8, 9, 10, 11, 12, or 13) selected from the group), a coding gene therefor, or a combination thereof;

at least one selected from the group consisting of IFNG, SLC27A2, CCL5, FOXP3, PTGES2, TNF, IFNA1, IFNL1, CCL2, CXCR2, PTGES, CXCR4, and IFNB1 (for example, one or more (e.g., 2, 3, 4, 5, 6, 7, 8, 9, 10, 11, 12, or 13) selected from the group), a coding gene therefor, or a combination thereof;

at least one selected from the group consisting of SLC27A2, IFNG, CCL2, TNF, PTGES, FOXP3, PTGES2, CXCR4, and CXCR2 (for example, one or more (e.g., 2, 3, 4, 5, 6, 7, 8, or 9) selected from the group), a coding gene therefor, or a combination thereof;

at least one selected from the group consisting of IFNL1, IFNG, CXCL11, CCL2, PTGES2, SLC27A2, and PTGES (for example, one or more (e.g., 2, 3, 4, 5, 6, or 7) selected from the group), a coding gene therefor, or a combination thereof;

at least one selected from the group consisting of ARG1, CCL2, CLEC7A, SLC27A2, IFNG, PTGES, PTGES2, TNF, and FOXP3 (for example, one or more (e.g., 2, 3, 4, 5, 6, 7, 8, or 9) selected from the group), a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof;

at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof;

at least one selected from the group consisting of TNF, IFNG, IFNL1, CXCL11, CLEC7A, VEGFA, CCL2, CXCR4, CXCR2, ARG1, SLC27A2, PTGES2, and PTGES (for example, one or more (e.g., 2, 3, 4, 5, 6, 7, 8, 9, 10, 11, 12, or 13) selected from the group), a coding gene therefor, or a combination thereof;

at least one selected from the group consisting of TNF, IFNG, CCL2, CXCR2, ARG1, SLC27A2, PTGES2, and PTGES (for example, one or more (e.g., 2, 3, 4, 5, 6, 7, or 8) selected from the group), a coding gene therefor, or a combination thereof;

at least one selected from the group consisting of VEGFA, CXCR4, SLC27A2, TNF, PTGES2, CCL5, CXCL11, CXCR2, FOXP3, PTGS2, and CXCL8 (for example, one or more (e.g., 2, 3, 4, 5, 6, 7, 8, 9, 10, or 11) selected from the group), a coding gene therefor, or a combination thereof;

at least one selected from the group consisting of PTGES2, IFNG, CCL5, VEGFA, PTGS2, CLEC7A, CXCR4, TNF, FOXP3, CCL2, CXCR2, CXCL8, PTGES, IFNL1, and SLC27A2 (for example, one or more (e.g., 2, 3, 4, 5, 6, 7, 8, 9, 10, 11, 12, 13, 14, or 15) selected from the group), a coding gene therefor, or a combination thereof;

at least one selected from the group consisting of VEGFA, CXCR4, SLC27A2, TNF, PTGES2, CCL5, CXCR2, FOXP3, PTGS2, and CXCL8 (for example, one or more (e.g., 2, 3, 4, 5, 6, 7, 8, 9, or 10) selected from the group), a coding gene therefor, or a combination thereof;

at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2 (for example, one or more (e.g., 2, 3, 4, 5, 6, 7, 8, 9, 10, 11, 12, 13, or 14) selected from the group), a coding gene therefor, or a combination thereof;

at least one selected from the group consisting of CCL5, PTGS2, CXCR2, CXCR4, SLC27A2, CXCL11, PTGES2, TNF, and VEGFA (for example, one or more (e.g., 2, 3, 4, 5, 6, 7, 8, or 9) selected from the group), a coding gene therefor, or a combination thereof;

at least one selected from the group consisting of SLC27A2, PTGES2, VEGFA, TNF, CXCR4, IFNG, CCL5, CXCR2, CCL2, CXCL11, CLEC7A, and ARG1 (for example, one or more (e.g., 2, 3, 4, 5, 6, 7, 8, 9, 10, 11, or 12,) selected from the group), a coding gene therefor, or a combination thereof;

at least one selected from the group consisting of IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2 (for example, one or more (e.g., 2, 3, 4, 5, 6, 7, 8, 9, 10, 11, 12, 13, 14, 15, 16, or 17) selected from the group), a coding gene therefor, or a combination thereof;

at least one selected from the group consisting of SLC27A2, PTGES2, VEGFA, TNF, CXCR4, IFNG, CCL5, CXCR2, CCL2, and CLEC7A (for example, one or more (e.g., 2, 3, 4, 5, 6, 7, 8, 9, or 10) selected from the group), a coding gene therefor, or a combination thereof;

at least one selected from the group consisting of IFNG, CCL2, and PTGES2 (for example, one, two, or three selected from the group), a coding gene therefor, or a combination thereof;

at least one selected from the group consisting of ARG1, CCL2, SLC27A2, IFNG, PTGES2, TNF, VEGFA, CXCR4, and CCL5 (for example, one or more (e.g., 2, 3, 4, 5, 6, 7, 8, or 9) selected from the group); a coding gene therefor, or a combination thereof;

at least one selected from the group consisting of TNF, IFNG, CXCL11, VEGFA, CCL2, CCL5, CXCR4, CXCR2, PTGS2, SLC27A2, and PTGES2 (for example, one or more (e.g., 2, 3, 4, 5, 6, 7, 8, 9, 10, or 11) selected from the group); a coding gene therefor, or a combination thereof; and

at least one selected from the group consisting of IFNG, VEGFA, SLC27A2, PTGES2, TNF, CXCR4, CXCR2, CCL5, CCL2, and CXCL11 (for example, one or more (e.g., 2, 3, 4, 5, 6, 7, 8, 9, or 10) selected from the group); a coding gene therefor, or a combination thereof.

For instance, the biomarker may be a biomarker set selected from the following sets; a coding gene therefor, or a combination thereof:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;

SLC27A2, IFNG, CCL2, TNF, PTGES, CLEC7A, ARG1, PTGES2, FOXP3, CXCR4, VEGFA, CXCR2, and PTGS2;

IFNG, SLC27A2, CCL5, FOXP3, PTGES2, TNF, IFNA1, IFNL1, CCL2, CXCR2, PTGES, CXCR4, and IFNB1;

SLC27A2, IFNG, CCL2, TNF, PTGES, FOXP3, PTGES2, CXCR4, and CXCR2;

IFNL1, IFNG, CXCL11, CCL2, PTGES2, SLC27A2, and PTGES;

ARG1, CCL2, CLEC7A, SLC27A2, IFNG, PTGES, PTGES2, TNF, and FOXP3;

CCL5, CXCR4, SLC27A2, PTGES2, TNF, VEGFA, ARG1, CCL2, CLEC7A, IFNG, PTGES, and FOXP3;

CCL5, CXCR4, SLC27A2, PTGES2, TNF, VEGFA, ARG1, CCL2, CLEC7A, IFNG, PTGES, FOXP3, PTGS2, CXCR2, and CXCL11;

CCL2, CCL5, CXCR2, SLC27A2, IFNL1, IFNG, PTGES2, and TNF;

TNF, IFNG, IFNL1, CXCL11, CLEC7A, VEGFA, CCL2, CXCR4, CXCR2, ARG1, SLC27A2, PTGES2, and PTGES;

TNF, IFNG, CCL2, CXCR2, ARG1, SLC27A2, PTGES2, and PTGES;

VEGFA, CXCR4, SLC27A2, TNF, PTGES2, CCL5, CXCL11, CXCR2, FOXP3, PTGS2, and CXCL8;

PTGES2, IFNG, CCL5, VEGFA, PTGS2, CLEC7A, CXCR4, TNF, FOXP3, CCL2, CXCR2, CXCL8, PTGES, IFNL1, and SLC27A2;

VEGFA, CXCR4, SLC27A2, TNF, PTGES2, CCL5, CXCR2, FOXP3, PTGS2, and CXCL8;

IFNG, TNF, CLEC7A, CXCL8, FOXP3, VEGFA, CCL2, CCL5, CCR5, CXCR4, ARG1, CXCR2, PTGS2, and PTGES2;

CCL5, PTGS2, CXCR2, CXCR4, SLC27A2, CXCL11, PTGES2, TNF, and VEGFA;

SLC27A2, PTGES2, VEGFA, TNF, CXCR4, IFNG, CCL5, CXCR2, CCL2, CXCL11, CLEC7A, and ARG1;

IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2;

SLC27A2, PTGES2, VEGFA, TNF, CXCR4, IFNG, CCL5, CXCR2, CCL2, and CLEC7A;

IFNG, CCL2, and PTGES2;

ARG1, CCL2, SLC27A2, IFNG, PTGES2, TNF, VEGFA, CXCR4, and CCL5;

TNF, IFNG, CXCL11, VEGFA, CCL2, CCL5, CXCR4, CXCR2, PTGS2, SLC27A2, and PTGES2; and

IFNG, VEGFA, SLC27A2, PTGES2, TNF, CXCR4, CXCR2, CCL5, CCL2, and CXCL11.

More specifically, the biomarker may include at least one selected from the following sets, wherein the pancreatic cancer diagnosable therewith is early-stage pancreatic cancer:

at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

at least one selected from the group consisting of SLC27A2, IFNG, CCL2, TNF, PTGES, CLEC7A, ARG1, PTGES2, FOXP3, CXCR4, VEGFA, CXCR2, and PTGS2 (for example, one or more (e.g., 2, 3, 4, 5, 6, 7, 8, 9, 10, 11, 12, or 13) selected from the group), a coding gene therefor, or a combination thereof;

at least one selected from the group consisting of IFNG, SLC27A2, CCL5, FOXP3, PTGES2, TNF, IFNA1, IFNL1, CCL2, CXCR2, PTGES, CXCR4, and IFNB1 (for example, one or more (e.g., 2, 3, 4, 5, 6, 7, 8, 9, 10, 11, 12, or 13) selected from the group); a coding gene therefor, or a combination thereof;

at least one selected from the group consisting of SLC27A2, IFNG, CCL2, TNF, PTGES, FOXP3, PTGES2, CXCR4, and CXCR2 (for example, one or more (e.g., 2, 3, 4, 5, 6, 7, 8, or 9) selected from the group); a coding gene therefor, or a combination thereof;

at least one selected from the group consisting of IFNL1, IFNG, CXCL11, CCL2, PTGES2, SLC27A2, and PTGES (for example, one or more (e.g., 2, 3, 4, 5, 6, or 7) selected from the group); a coding gene therefor, or a combination thereof;

at least one selected from the group consisting of ARG1, CCL2, CLEC7A, SLC27A2, IFNG, PTGES, PTGES2, TNF, and FOXP3 (for example, one or more (e.g., 2, 3, 4, 5, 6, 7, 8, or 9) selected from the group); a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group); a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group); a coding gene therefor, or a combination thereof;

at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group); a coding gene therefor, or a combination thereof;

at least one selected from the group consisting of TNF, IFNG, IFNL1, CXCL11, CLEC7A, VEGFA, CCL2, CXCR4, CXCR2, ARG1, SLC27A2, PTGES2, and PTGES (for example, one or more (e.g., 2, 3, 4, 5, 6, 7, 8, 9, 10, 11, 12, or 13) selected from the group); a coding gene therefor, or a combination thereof; and

at least one selected from the group consisting of TNF, IFNG, CCL2, CXCR2, ARG1, SLC27A2, PTGES2, and PTGES (for example, one or more (e.g., 2, 3, 4, 5, 6, 7, or 8) selected from the group); a coding gene therefor, or a combination thereof.

More specifically, for instance, the biomarker may be a biomarker set selected from the following sets; coding genes thereof; or a combination thereof, wherein the pancreatic cancer diagnosable therewith is early-stage pancreatic cancer:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;
SLC27A2, IFNG, CCL2, TNF, PTGES, CLEC7A, ARG1, PTGES2, FOXP3, CXCR4, VEGFA, CXCR2, and PTGS2;
IFNG, SLC27A2, CCL5, FOXP3, PTGES2, TNF, IFNA1, IFNL1, CCL2, CXCR2, PTGES, CXCR4, and IFNB1;
SLC27A2, IFNG, CCL2, TNF, PTGES, FOXP3, PTGES2, CXCR4, and CXCR2;
IFNL1, IFNG, CXCL11, CCL2, PTGES2, SLC27A2, and PTGES;
ARG1, CCL2, CLEC7A, SLC27A2, IFNG, PTGES, PTGES2, TNF, and FOXP3;
CCL5, CXCR4, SLC27A2, PTGES2, TNF, VEGFA, ARG1, CCL2, CLEC7A, IFNG, PTGES, and FOXP3;
CCL5, CXCR4, SLC27A2, PTGES2, TNF, VEGFA, ARG1, CCL2, CLEC7A, IFNG, PTGES, FOXP3, PTGS2, CXCR2, and CXCL11;
CCL2, CCL5, CXCR2, SLC27A2, IFNL1, IFNG, PTGES2, and TNF;
TNF, IFNG, IFNL1, CXCL11, CLEC7A, VEGFA, CCL2, CXCR4, CXCR2, ARG1, SLC27A2, PTGES2, and PTGES; and
TNF, IFNG, CCL2, CXCR2, ARG1, SLC27A2, PTGES2, and PTGES.

In another embodiment, the biomarker may include at least one selected from the following sets, the pancreatic cancer diagnosable therewith is late-stage pancreatic cancer:

at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;
at least one selected from the group consisting of VEGFA, CXCR4, SLC27A2, TNF, PTGES2, CCL5, CXCL11, CXCR2, FOXP3, PTGS2, and CXCL8 (for example, one or more (e.g., 2, 3, 4, 5, 6, 7, 8, 9, 10, or 11) selected from the group); a coding gene therefor, or a combination thereof;
at least one selected from the group consisting of PTGES2, IFNG, CCL5, VEGFA, PTGS2, CLEC7A, CXCR4, TNF, FOXP3, CCL2, CXCR2, CXCL8, PTGES, IFNL1, and SLC27A2 (for example, one or more (e.g., 2, 3, 4, 5, 6, 7, 8, 9, 10, 11, 12, 13, 14, or 15) selected from the group); a coding gene therefor, or a combination thereof;
at least one selected from the group consisting of VEGFA, CXCR4, SLC27A2, TNF, PTGES2, CCL5, CXCR2, FOXP3, PTGS2, and CXCL8 (for example, one or more (e.g., 2, 3, 4, 5, 6, 7, 8, 9, or 10) selected from the group); a coding gene therefor, or a combination thereof;
at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2 (for example, one or more (e.g., 2, 3, 4, 5, 6, 7, 8, 9, 10, 11, 12, 13, or 14) selected from the group); a coding gene therefor, or a combination thereof;
at least one selected from the group consisting of CCL5, PTGS2, CXCR2, CXCR4, SLC27A2, CXCL11, PTGES2, TNF, and VEGFA (for example, one or more (e.g., 2, 3, 4, 5, 6, 7, 8, or 9) selected from the group); a coding gene therefor, or a combination thereof;
at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group); a coding gene therefor, or a combination thereof;
at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group); a coding gene therefor, or a combination thereof; and
at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group); a coding gene therefor, or a combination thereof.

For instance, the biomarker may be a biomarker set selected from the following sets; a coding gene; or a combination thereof, the pancreatic cancer diagnosable therewith is late-stage pancreatic cancer:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;
VEGFA, CXCR4, SLC27A2, TNF, PTGES2, CCL5, CXCL11, CXCR2, FOXP3, PTGS2, and CXCL8;
PTGES2, IFNG, CCL5, VEGFA, PTGS2, CLEC7A, CXCR4, TNF, FOXP3, CCL2, CXCR2, CXCL8, PTGES, IFNL1, and SLC27A2;
VEGFA, CXCR4, SLC27A2, TNF, PTGES2, CCL5, CXCR2, FOXP3, PTGS2, and CXCL8;
IFNG, TNF, CLEC7A, CXCL8, FOXP3, VEGFA, CCL2, CCL5, CCR5, CXCR4, ARG1, CXCR2, PTGS2, and PTGES2;
CCL5, PTGS2, CXCR2, CXCR4, SLC27A2, CXCL11, PTGES2, TNF, and VEGFA;
CCL5, CXCR4, SLC27A2, PTGES2, TNF, VEGFA, ARG1, CCL2, CLEC7A, IFNG, PTGES, and FOXP3;
CCL5, CXCR4, SLC27A2, PTGES2, TNF, VEGFA, ARG1, CCL2, CLEC7A, IFNG, PTGES, FOXP3, PTGS2, CXCR2, and CXCL11; and
CCL2, CCL5, CXCR2, SLC27A2, IFNL1, IFNG, PTGES2, and TNF.

In another embodiment, the biomarker may include at least one selected from the following, the pancreatic cancer diagnosable therewith is a pancreatic cancer at any stage, regardless of progression, such as early-stage pancreatic cancer, late-stage pancreatic cancer, or both:

at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;
at least one selected from the group consisting of SLC27A2, PTGES2, VEGFA, TNF, CXCR4, IFNG, CCL5, CXCR2, CCL2, CXCL11, CLEC7A, and ARG1 (for example, one or more (e.g., 2, 3, 4, 5, 6, 7, 8, 9, 10, 11, or 12,) selected from the group); a coding gene therefor, or a combination thereof;

at least one selected from the group consisting of IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2 (for example, one or more (e.g., 2, 3, 4, 5, 6, 7, 8, 9, 10, 11, 12, 13, 14, 15, 16, or 17) selected from the group); a coding gene therefor, or a combination thereof;

at least one selected from the group consisting of SLC27A2, PTGES2, VEGFA, TNF, CXCR4, IFNG, CCL5, CXCR2, CCL2, and CLEC7A (for example, one or more (e.g., 2, 3, 4, 5, 6, 7, 8, 9, or 10) selected from the group); a coding gene therefor, or a combination thereof;

at least one selected from the group consisting of IFNG, CCL2, and PTGES2 (for example, one, two, or three selected from the group), a coding gene therefor, or a combination thereof;

at least one selected from the group consisting of ARG1, CCL2, SLC27A2, IFNG, PTGES2, TNF, VEGFA, CXCR4, and CCL5 (for example, one or more (e.g., 2, 3, 4, 5, 6, 7, 8, or 9) selected from the group), a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof;

at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group); a coding gene therefor, or a combination thereof;

at least one selected from the group consisting of TNF, IFNG, CXCL11, VEGFA, CCL2, CCL5, CXCR4, CXCR2, PTGS2, SLC27A2, and PTGES2 (for example, one or more (e.g., 2, 3, 4, 5, 6, 7, 8, 9, 10, or 11) selected from the group); a coding gene therefor, or a combination thereof; and

at least one selected from the group consisting of IFNG, VEGFA, SLC27A2, PTGES2, TNF, CXCR4, CXCR2, CCL5, CCL2, and CXCL11 (for example, one or more (e.g., 2, 3, 4, 5, 6, 7, 8, 9, or 10) selected from the group); a coding gene therefor, or a combination thereof.

For instance, the biomarker may be a biomarker set selected from the following sets; a coding gene; or a combination thereof, the pancreatic cancer diagnosable therewith is a pancreatic cancer at any stage, regardless of progression, such as early-stage pancreatic cancer, late-stage pancreatic cancer, or both:

ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA;

SLC27A2, PTGES2, VEGFA, TNF, CXCR4, IFNG, CCL5, CXCR2, CCL2, CXCL11, CLEC7A, and ARG1;

IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2;

SLC27A2, PTGES2, VEGFA, TNF, CXCR4, IFNG, CCL5, CXCR2, CCL2, and CLEC7A;

IFNG, CCL2, and PTGES2;

ARG1, CCL2, SLC27A2, IFNG, PTGES2, TNF, VEGFA, CXCR4, and CCL5;

CCL5, CXCR4, SLC27A2, PTGES2, TNF, VEGFA, ARG1, CCL2, CLEC7A, IFNG, PTGES, and FOXP3;

CCL5, CXCR4, SLC27A2, PTGES2, TNF, VEGFA, ARG1, CCL2, CLEC7A, IFNG, PTGES, FOXP3, PTGS2, CXCR2, and CXCL11;

CCL2, CCL5, CXCR2, SLC27A2, IFNL1, IFNG, PTGES2, and TNF;

TNF, IFNG, CXCL11, VEGFA, CCL2, CCL5, CXCR4, CXCR2, PTGS2, SLC27A2, and PTGES2; and

IFNG, VEGFA, SLC27A2, PTGES2, TNF, CXCR4, CXCR2, CCL5, CCL2, and CXCL11.

In the method, the step of detecting the biomarker may involve determining the presence or absence of the biomarker in the sample, measuring a level (concentration) of the biomarker, or performing both. In the method, when the biomarker in the sample is present at a higher and/or lower level compared to a reference sample (e.g., a normal sample or a sample from a subject with a similar disease), the sample or the subject from which the sample was derived may be diagnosed (or identified or determined) as a pancreatic cancer patient. The diagnosis of the pancreatic cancer patient may involve confirming or determining that the subject has pancreatic cancer and/or distinguishing the pancreatic cancer patient from a subject with a similar disease. The pancreatic cancer may refer to early-stage pancreatic cancer, late-stage pancreatic cancer, or both. The normal subject may refer to a subject who does not have pancreatic cancer (including early-stage pancreatic cancer, late-stage pancreatic cancer, or both). The similar disease may refer to benign pancreatic diseases (including pancreatitis (chronic pancreatitis and/or acute pancreatitis), benign pancreatic tumors, intraductal papillary mucinous neoplasm (IPMN), autoimmune pancreatitis (AIP), or other pancreatic diseases (excluding pancreatic cancer (malignant pancreatic tumors)) and/or cholangiocarcinoma.

In an embodiment, the method for diagnosing pancreatic cancer or the method for providing information for pancreatic cancer diagnosis provided in the present disclosure may include:

(a) detecting at least one of the aforementioned biomarkers in a blood sample isolated from a subject; and
(b) diagnosing (or identifying or determining) the sample or the subject from which the sample was derived as a pancreatic cancer patient (including early-stage pancreatic cancer, late-stage pancreatic cancer, or both) when the biomarker in the blood sample is present at a higher and/or lower level compared to a reference sample (e.g., a normal sample or a sample from a subject with a similar disease).

In the method, step (b) may include one or more (e.g., 1, 2, 3, 4, 5, 6, 7, 8, 9, 10, 11, 12, 13, 14, 15, 16, 17, 18, 19, or 20) of the following steps of:

- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when IFNL1, a coding gene therefor, or a combination thereof in the blood sample is present at a higher (increased) level compared to a reference sample (e.g., a normal sample);
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when IFNG, a coding gene therefor, or a combination thereof in the blood sample is present at a lower (decreased) level compared to a reference sample (e.g., a normal sample);
- Diagnosing (or identifying or determining) the sample or the subject from which the sample was derived as an early-stage pancreatic cancer patient when CXCL11, a coding gene therefor, or a combination thereof in the blood sample is present at a higher (increased) level compared to a reference sample (e.g., a normal sample), and/or diagnosing as a late-stage pancreatic cancer patient when CXCL11, a coding gene therefor, or a combination thereof in the blood sample is present at a lower (decreased) level;
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when TNF, a coding gene therefor, or a combination thereof in the blood sample is present at a higher (increased) level compared to a reference sample (e.g., a normal sample);
- Diagnosing (or identifying or determining) the sample or the subject from which the sample was derived as an early-stage pancreatic cancer patient when CLEC7A, a coding gene therefor, or a combination thereof in the blood sample is present at a lower (decreased) level compared to a reference sample (e.g., a normal sample), and/or diagnosing as a late-stage pancreatic cancer patient when CLEC7A, a coding gene therefor, or a combination thereof in the blood sample is present at a higher (increased) level;
- Diagnosing (or identifying or determining) the sample or the subject from which the sample was derived as a pancreatic cancer patient, for example a patient with early-stage pancreatic cancer, late-stage pancreatic cancer, or pancreatic cancer at any stage regardless of progression (including early-stage pancreatic cancer and late-stage pancreatic cancer), when CXCL8, a coding gene therefor, or a combination thereof in the blood sample is present at a higher (increased) level compared to a reference sample (e.g., a normal sample);
- Diagnosing (or identifying or determining) the sample or the subject from which the sample was derived as an early-stage pancreatic cancer patient when FOXP3, a coding gene therefor, or a combination thereof in the blood sample is present at a higher (increased) level compared to a reference sample (e.g., a normal sample), and/or diagnosing as a late-stage pancreatic cancer patient when FOXP3, a coding gene therefor, or a combination thereof in the blood sample is present at a lower (decreased) level;
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when VEGFA, a coding gene therefor, or a combination thereof in the blood sample is present at a higher (increased) level compared to a reference sample (e.g., a normal sample);
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when CCL2, a coding gene therefor, or a combination thereof in the blood sample is present at a higher (increased) level compared to a reference sample (e.g., a normal sample);

- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when CCL5, a coding gene therefor, or a combination thereof in the blood sample is present at a lower (decreased) level compared to a reference sample (e.g., a normal sample);
- Diagnosing (or identifying or determining) the sample or the subject from which the sample was derived as an early-stage pancreatic cancer patient when CCR5, a coding gene therefor, or a combination thereof in the blood sample is present at a higher (increased) level compared to a reference sample (e.g., a normal sample), and/or diagnosing as a late-stage pancreatic cancer patient when CCR5, a coding gene therefor, or a combination thereof in the blood sample is present at a lower (decreased) level;
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when CXCR4, a coding gene therefor, or a combination thereof in the blood sample is present at a higher (increased) level compared to a reference sample (e.g., a normal sample);
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when ARG1, a coding gene therefor, or a combination thereof in the blood sample is present at a higher (increased) level compared to a reference sample (e.g., a normal sample).
- Diagnosing (or identifying or determining) the sample or the subject from which the sample was derived as an early-stage pancreatic cancer patient when CXCR2, a coding gene therefor, or a combination thereof in the blood sample is present at a lower (decreased) level compared to a reference sample (e.g., a normal sample), and/or diagnosing as a late-stage pancreatic cancer patient when CXCR2, a coding gene therefor, or a combination thereof in the blood sample is present at a higher (increased) level;
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when **PTGS2,** a coding gene therefor, or a combination thereof in the blood sample is present at a higher (increased) level compared to a reference sample (e.g., a normal sample);
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when PTGES2, a coding gene therefor, or a combination thereof in the blood sample is present at a lower (decreased) level compared to a reference sample (e.g., a normal sample);
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when SLC27A2, a coding gene therefor, or a combination thereof in the blood sample is present at a higher (increased) level compared to a reference sample (e.g., a normal sample);
- Diagnosing (or identifying or determining) the sample or the subject from which the sample was derived as a pancreatic cancer patient, for example, a patient with early-stage pancreatic cancer, late-stage pancreatic cancer, or pancreatic cancer at any stage regardless of progression (including early-stage pancreatic cancer and/or late-stage pancreatic cancer), when PTGES, a coding gene therefor, or a combination thereof in the blood sample is present at a higher (increased) level compared to a reference sample (e.g., a normal sample).
- Diagnosing (or identifying or determining) the sample or the subject from which the sample was derived as a pancreatic cancer patient when IFNB1, a coding gene therefor, or a combination thereof in the blood sample is present at a lower (decreased) level compared to a reference sample (e.g., a similar disease sample); and
- Diagnosing (or identifying or determining) the sample or the subject from which the sample was derived as a pancreatic cancer patient when IFNA1, a coding gene therefor, or a combination thereof in the blood sample is present at a lower (decreased) level compared to a reference sample (e.g., a similar disease sample).

More specifically, the method may be a method for diagnosing an early pancreatic cancer or a method for providing information for early pancreatic cancer diagnosis, including the following step of

(a) detecting: at least one selected from a group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, 18, 19, or 20) selected from the group);

a coding gene therefor; or a combination thereof in a blood sample isolated from a subject.

More specifically, the method for diagnosing early pancreatic cancer or the method for providing information for early pancreatic cancer diagnosis may include a step of detecting one or more of the following markers:

(1-1) at least one selected from a group consisting of SLC27A2, IFNG, CCL2, TNF, PTGES, CLEC7A, ARG1, PTGES2, FOXP3, CXCR4, VEGFA, CXCR2, and PTGS2 (for example, one or more (e.g., 2, 3, 4, 5, 6, 7, 8, 9, 10, 11, 12, or 13) selected from the group) (for example, at least one (1, 2, 3, 4, 5, 6, 7, 8, or 9) selected from the group consisting of ARG1, CCL2, CLEC7A, SLC27A2, IFNG, PTGES, PTGES2, TNF, and FOXP3); a coding gene therefor; or a combination thereof;

(1-2) at least one selected from a group consisting of IFNG, SLC27A2, CCL5, FOXP3, PTGES2, TNF, IFNA1, IFNL1, CCL2, CXCR2, PTGES, CXCR4, and IFNB1 (for example, one or more (e.g., 2, 3, 4, 5, 6, 7, 8, 9, 10, 11, 12, or 13) selected from the group), a coding gene therefor, or a combination thereof;

(1-3) at least one selected from a group consisting of SLC27A2, IFNG, CCL2, TNF, PTGES, FOXP3, PTGES2, CXCR4, and CXCR2 (for example, one or more (e.g., 2, 3, 4, 5, 6, 7, 8, or 9) selected from the group), a coding gene therefor, or a combination thereof;

(1-4) at least one selected from a group consisting of IFNL1, IFNG, CXCL11, CCL2, PTGES2, SLC27A2, and PTGES (for example, one or more (e.g., 2, 3, 4, 5, 6, or 7) selected from the group), a coding gene therefor, or a combination thereof;

(1-5) at least one selected from the group consisting of ARG1, CCL2, CLEC7A, SLC27A2, IFNG, PTGES, PTGES2, TNF, and FOXP3 (for example, one or more (e.g., 2, 3, 4, 5, 6, 7, 8, or 9) selected from the group), a coding gene therefor, or a combination thereof;

(1-6) at least one selected from a group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

(1-7) at least one selected from a group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

(1-8) at least one selected from a group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, or 8) selected from the group), a coding gene therefor, or a combination thereof; and

(1-9) at least one selected from a group consisting of CCL2, CCL5, CXCR2, SLC27A2, IFNL1, IFNG, PTGES2, and TNF (for example, one or more (e.g., 2, 3, 4, 5, 6, 7, or 8) selected from the group); a coding gene therefor, or a combination thereof.

The method for diagnosing early pancreatic cancer or the method for providing information for early pancreatic cancer diagnosis may further include the following step of:

(b) in the blood sample,

- diagnosing (or confirming or determining) the blood sample or the subject from which the blood sample is derived as a pancreatic cancer patient, more specifically, an early pancreatic cancer patient when at least one selected from a group consisting of IFNL1, CXCL11, TNF, CXCL8, FOXP3, VEGFA, CCL2, CCR5, CXCR4, ARG1, PTGS2, SLC27A2, and PTGES (for example, one or more (e.g., 2, 3, 4, 5, 6, 7, 8, 9, 10, or 11) selected from the group), a coding gene therefor, or a combination thereof, is present at a higher (increased) level compared to a reference sample (e.g., a normal sample); and/or
- diagnosing (or confirming or determining) the blood sample or the subject from which the blood sample is derived as a pancreatic cancer patient, more specifically, an early pancreatic cancer patient when at least one (e.g., one, two, three, four, or five) selected from a group consisting of IFNG, CLEC7A, CCL5, CXCR2, and PTGES2, a coding gene therefor, or a combination thereof is present at a lower (decreased) level compared to a reference sample (e.g., a normal sample).

In another embodiment, the method may be a method for diagnosing late-stage pancreatic cancer or providing information for the diagnosis of late-stage pancreatic cancer, comprising the following step of:

(a) detecting at least one from the group consisting of IFNL1, IFNG, CXCL11, TNF, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (e.g., 2, 3, 4, 5, 6, 7, 8, 9, 10, 11, 12, 13, 14, 15, 16, 17, or 18) selected from the group), a coding gene therefor, or a combination thereof in a blood sample isolated from a subject.

More specifically, the method for diagnosing late-stage pancreatic cancer or providing information for the diagnosis of late-stage pancreatic cancer may include a step of detecting a marker selected from the following group of:

(2-1) at least one selected from a group consisting of VEGFA, CXCR4, SLC27A2, TNF, PTGES2, CCL5, CXCL11, CXCR2, FOXP3, PTGS2, and CXCL8 (for example, one or more (2, 3, 4, 5, 6, 7, 8, 9, 10, or 11) selected from the group) (e.g., one or more (1, 2, 3, 4, 5, 6, 7, 8, or 9) selected from the group consisting of CCL5, PTGS2, CXCR2, CXCR4, SLC27A2, CXCL11, PTGES2, TNF, and VEGFA), a coding gene therefor, or a combination thereof;

(2-2) at least one selected from the group consisting of PTGES2, IFNG, CCL5, VEGFA, PTGS2, CLEC7A, CXCR4, TNF, FOXP3, CCL2, CXCR2, CXCL8, PTGES, IFNL1, and SLC27A2 (for example, one or more (e.g., 2, 3, 4, 5, 6, 7, 8, 9, 10, 11, 12, 13, 14, or 15) selected from the group), a coding gene therefor, or a combination thereof;

(2-3) at least one selected from the group consisting of VEGFA, CXCR4, SLC27A2, TNF, PTGES2, CCL5, CXCR2, FOXP3, PTGS2, and CXCL8 (for example, one or more (e.g., 2, 3, 4, 5, 6, 7, 8, 9, or 10) selected from the group), a coding gene therefor, or a combination thereof;

(2-4) at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2 (for example, one or more (e.g., 2, 3, 4, 5, 6, 7, 8, 9, 10, 11, 12, 13, or 14) selected from the group), a coding gene therefor, or a combination thereof;

(2-5) at least one selected from the group consisting of CCL5, PTGS2, CXCR2, CXCR4, SLC27A2, CXCL11, PTGES2, TNF, and VEGFA (for example, one or more (e.g., 2, 3, 4, 5, 6, 7, 8, or 9) selected from the group), a coding gene therefor, or a combination thereof;

(2-6) at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

(2-7) at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

(2-8) at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof; or

(2-9) at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof.

The method for diagnosing late-stage pancreatic cancer or the method for providing information for the diagnosis of late-stage pancreatic cancer may further include the following step of:
(b) in the blood sample,

- diagnosing (or identifying or determining) the blood sample or the subject from which the blood sample was derived as a pancreatic cancer patient, more specifically, a late-stage pancreatic cancer patient when at least one selected from the group consisting of IFNL1, TNF, CLEC7A, CXCL8, VEGFA, CCL2, CXCR4, ARG1, CXCR2, PTGS2, SLC27A2, and PTGES (for example, one or more (e.g., 2, 3, 4, 5, 6, 7, 8, 9, 10, 11, or 12) selected from the group); a coding gene therefor, or a combination thereof is present at a higher (increased) level compared to a reference sample (e.g., a normal sample); and/or

- diagnosing (or identifying or determining) the blood sample or the subject from which the blood sample was derived as a pancreatic cancer patient, more specifically, a late-stage pancreatic cancer patient when at least one selected from the group consisting of IFNG, CXCL11, FOXP3, CCL5, CCR5, and PTGES2 (for example, one or more (e.g., 2, 3, 4, 5, or 6) selected from the group), a coding gene therefor, or a combination thereof is present at a lower (decreased) level compared to a reference sample (e.g., a normal sample).

In another embodiment, the method may be a method for diagnosing all pancreatic cancers (early pancreatic cancer and/or late-stage pancreatic cancer) or a method for providing information for diagnosing all pancreatic cancers (early pancreatic cancer and/or late-stage pancreatic cancer), including the following step of:

(a) detecting: at least one selected from the group consisting of IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, SLC27A2, and PTGES (for example, one or more (2, 3, 4, 5, 6, 7, 8, 9, 10, 11, 12, 13, 14, 15, 16, 17, 18, 19, or 20) selected from the group); a coding gene therefor; or a combination thereof in a blood sample separated from a subject.
More specifically, the method for diagnosing all pancreatic cancers (early pancreatic cancer and/or late-stage

EP 4 596 718 A1

pancreatic cancer) or the method for providing information for diagnosis of all pancreatic cancers (early pancreatic cancer and/or late-stage pancreatic cancer) may include detecting a marker from the following groups of:

(3-1) at least one selected from the group consisting of SLC27A2, PTGES2, VEGFA, TNF, CXCR4, IFNG, CCL5, CXCR2, CCL2, CXCL11, CLEC7A, and ARG1 (for example, one or more (2, 3, 4, 5, 6, 7, 8, 9, 10, 11, or 12) selected from the group), a coding gene therefor, or a combination thereof;

(3-2) at least one selected from the group consisting of IFNG, PTGES2, CCL5, SLC27A2, VEGFA, CCL2, TNF, FOXP3, CXCR4, IFNA1, IFNL1, PTGES, CCR5, CLEC7A, IFNB1, CXCR2, and PTGS2 (for example, one or more (2, 3, 4, 5, 6, 7, 8, 9, 10, 11, 12, 13, 14, 15, 16, or 17) selected from the group), a coding gene therefor, or a combination thereof;

(3-3) at least one selected from the group consisting of SLC27A2, PTGES2, VEGFA, TNF, CXCR4, IFNG, CCL5, CXCR2, CCL2, and CLEC7A (for example, one or more (2, 3, 4, 5, 6, 7, 8, 9, or 10) selected from the group);

(3-4) at least one selected from the group consisting of IFNG, CCL2, and PTGES2 (for example, one or more (one, two, or three) selected from the group), a coding gene therefor, or a combination thereof;

(3-5) at least one selected from the group consisting of ARG1, CCL2, SLC27A2, IFNG, PTGES2, TNF, VEGFA, CXCR4, and CCL5 (for example, one or more (2, 3, 4, 5, 6, 7, 8, or 9) selected from the group), a coding gene therefor, or a combination thereof;

(3-6) at least one selected from the group consisting of ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA (for example, one or more (e.g., 2, 3, 4, 5, 6, 7, 8, 9, 10, 11, 12, 13, 14, 15, 16, 17, 18, 19, or 20) selected from the group), a coding gene therefor, or a combination thereof;

(3-7) at least one selected from the group consisting of CCL5, CXCR4, SLC27A2, PTGES2, TNF, VEGFA, ARG1, CCL2, CLEC7A, IFNG, PTGES, and FOXP3 (for example, one or more (e.g., 2, 3, 4, 5, 6, 7, 8, 9, 10, 11, or 12) selected from the group), a coding gene therefor, or a combination thereof;

(3-8) at least one selected from the group consisting of CCL5, CXCR4, SLC27A2, PTGES2, TNF, VEGFA, ARG1, CCL2, CLEC7A, IFNG, PTGES, FOXP3, PTGS2, CXCR2, and CXCL11 (for example, one or more (e.g., 2, 3, 4, 5, 6, 7, 8, 9, 10, 11, 12, 13, 14, or 15) selected from the group), a coding gene therefor, or a combination thereof; or

(3-9) at least one selected from the group consisting of CCL2, CCL5, CXCR2, SLC27A2, IFNL1, IFNG, PTGES2, and TNF (for example, one or more (e.g., 2, 3, 4, 5, 6, 7, or 8) selected from the group), a coding gene therefor, or a combination thereof.

The method for diagnosing all pancreatic cancers (early pancreatic cancer and/or late-stage pancreatic cancer) or the method for providing information for diagnosis of all pancreatic cancers (early pancreatic cancer and/or late-stage pancreatic cancer) may further include the following step of:

(b) diagnosing (or confirming or determining) a blood sample or a subject from which the blood sample was derived as a pancreatic cancer patient (patient with early pancreatic cancer, late-stage pancreatic cancer, or both) when at least one (for example, one, two, or three) selected from the group consisting of IFNG, CCL5, and PTGES2, a coding gene therefor, or a combination thereof is present at a lower (decreased) level in the blood sample compared to a reference sample (e.g., normal sample) or when at least one (e.g., 1, 2, 3, 4, 5, 6, 7, 8, 9, or 10) selected from the group consisting of IFNL1, TNF, CXCL8, VEGFA, CCL2, CXCR4, ARG1, PTGS2, SLC27A2, and PTGES is present at a higher (increased) level in the blood sample, a coding gene therefor, or a combination thereof is present at a higher (increased) level compared to a reference sample (e.g., normal sample).

In another embodiment, the method may be a method for diagnosing pancreatic cancer (early pancreatic cancer and/or late-stage pancreatic cancer) or a method for providing information for diagnosis of all pancreatic cancers (early pancreatic cancer and/or late-stage pancreatic cancer), including the following steps of:

(a) detecting at least one (one or two) selected from the group consisting of IFNB1 and IFNA1, a coding gene therefor, or a combination thereof in a blood sample isolated from a subject; and

(b) diagnosing (or confirming or determining) the blood sample or the subject from which the blood sample was derived as a pancreatic cancer patient (early pancreatic cancer, late-stage pancreatic cancer, or both) when at least one (1 or 2) selected from the group consisting of IFNB1 and IFNA1, a coding gene therefor, or a combination thereof is present at a lower (decreased) level in the blood sample compared to a reference sample (e.g., a sample from a subject with a similar disease). This method allows differentiation between pancreatic cancer patients (early pancreatic cancer, late-stage pancreatic cancer, or both) and patients with similar diseases.

The method using IFNB1 and/or IFNA1 as markers may serve to distinguish pancreatic cancer (early pancreatic cancer, late-stage pancreatic cancer, or both) from similar diseases, such as benign pancreatic diseases (pancreatitis (chronic pancreatitis and/or acute pancreatitis), benign pancreatic tumors, intraductal papillary mucinous neoplasm (IPMN), autoimmune pancreatitis (AIP), and other pancreatic diseases (excluding pancreatic cancer (malignant pancreatic tumors)) and cholangiocarcinoma.

In addition, the method may further include the steps of:

(a) measuring a level of at least one biomarker described above in a blood sample isolated from the subject; and

(b)

(i) comparing the biomarker level measured in the blood sample with the biomarker level in a reference sample (e.g., a normal sample or a sample from a subject with a similar disease);

(ii) diagnosing (or confirming or determining) the sample or the subject from which the sample was derived as a pancreatic cancer patient (early pancreatic cancer, late-stage pancreatic cancer, or both) when the biomarker level measured in the sample is higher or lower than that in the reference sample; or

(iii) Both steps (i) and (ii). Optionally, the method may further include, prior to step (b) (step (i) and/or (ii)), an additional step of measuring the biomarker level in the reference sample.

The step of measuring a level of the biomarker may be performed by measuring a concentration of the biomarker (protein, gene (full-length DNA, cDNA, mRNA, etc.), or both) and/or a number of cells expressing the biomarker. No particular limitations are imparted to the method for measuring the concentration of the biomarker and/or the number of biomarker-expressing cells, and the method may be any commonly used method for quantitative analysis of proteins, genes, and/or cells and appropriately be selected from the group consisting of immunochromatography, immunohistochemistry, enzyme-linked immunosorbent assay (ELISA), radioimmunoassay (RIA), enzyme immunoassay (EIA), fluorescence immunoassay (FIA), luminescence immunoassay (LIA), Western blotting, microarray analysis, flow cytometry, polymerase chain reaction (PCR, e.g., qPCR, real-time PCR, real-time qPCR), and fluorescence in situ hybridization (FISH), but with no limitations thereto. In an embodiment, the step of measuring a level of the biomarker may further include applying a statistical analysis method suitable for biomarker analysis, such as regression analysis (e.g., stepwise logistic regression analysis), but is not limited thereto. With respect to further details of "biomarker level measurement", reference may be made to the previously described section on "biomarker detection."

As used herein, the wording "increased biomarker level" refers to a situation where the biomarker level in a sample (for example, the concentration (level) of the biomarker (protein and/or gene) (e.g., for a gene, Ct value, $\Delta$Ct value, and/or $\Delta\Delta$Ct value measured by PCR), or the number of cells expressing the biomarker) is approximately 1% or more, 2% or more, 3% or more, 4% or more, 5% or more, 7% or more, 8% or more, 9% or more, 10% or more, 12% or more, 15% or more, 18% or more, 20% or more, 22% or more, 25% or more, 28% or more, or 30% or more higher than that in the reference sample.

As used herein, the wording "decreased biomarker level" refers to a situation where the biomarker level in a sample (for example, the concentration (level) of the biomarker (protein and/or gene) (e.g., for a gene, Ct value, $\Delta$Ct value, and/or $\Delta\Delta$Ct value measured by PCR), or the number of cells expressing the biomarker) is approximately 1% or more, 2% or more, 3% or more, 4% or more, 5% or more, 7% or more, 8% or more, 9% or more, 10% or more, 12% or more, 15% or more, 18% or more, 20% or more, 22% or more, 25% or more, 28% or more, or 30% or more lower than that in the reference sample.

In another embodiment, the method may be a method for diagnosing early pancreatic cancer or a method for providing information for early pancreatic cancer diagnosis, comprising the following step of:

(a-1) measuring a level of at least one biomarker selected from the group consisting of TNF, IFNG, IFNL1, CXCL11, CLEC7A, VEGFA, CCL2, CXCR4, CXCR2, ARG1, SLC27A2, PTGES2, and PTGES (one or more ( 2, 3, 4, 5, 6, 7, 8, 9, 10, 11, 12, or 13) selected from the group), a coding gene therefor, or a combination thereof in a blood sample isolated from a subject.

In an embodiment, the step of measuring a level of the biomarker is as described above. For instance, the biomarker level may be measured by quantification (e.g., in the case of PCR, measuring Ct values, $\Delta$Ct values, and/or $\Delta\Delta$Ct values) and, optionally, performing regression analysis (e.g., stepwise logistic regression analysis). Additional statistical analysis may also be conducted as needed.

The method for diagnosing early pancreatic cancer or the method for providing information for diagnosis of early pancreatic cancer may further include, after step (a-1), the following step of:

(b-1) computing using the following equation:

$$\text{logit}(p) = B0 + B1 \times TNF + B2 \times IFNG + B3 \times IFNL1 + B4 \times CXCL11 + B5 \times CLEC7A + B6 \times VEGFA + B7 \times CCL2 + B8 \times CXCR4 + B9 \times CXCR2 + B10 \times ARG1 + B11 \times SLC27A2 + B12 \times PTGES2 + B13 \times PTGES \quad \text{[Equation 1]}$$

(wherein,

B0: a rational number (up to four decimal places) in the range of 30 to 50, 30 to 47.5, 30 to 45, 32.5 to 50, 32.5 to 47.5, 32.5 to 45, 35 to 50, 35 to 47.5, or 35 to 45, for example, 35.8476 or 44.4033;

TNF: expression level of the TNF biomarker;

B1: coefficient for TNF expression level, a rational number in the range of -3 to - 2 or -2.9 to -2.5 (up to four decimal places, e.g., -2.5794 or -2.8042), or 0;

IFNG: expression level of the IFNG biomarker;

B2: coefficient for IFNG expression level, a rational number in the range of 3 to 4 or 3.3 to 3.8 (up to four decimal places, e.g., 3.3956 or 3.7082), or 0;

IFNL1: expression level of the IFNL1 biomarker;

B3: coefficient for IFNL1 expression level, a rational number in the range of 0.1 to 0.5 or 0.2 to 0.3 (up to four decimal places, e.g., 0.2358), or 0;

CXCL11: expression level of the CXCL11 biomarker;

B4: coefficient for CXCL11 expression level, a rational number in the range of 0.1 to 0.2(up to four decimal places, e.g., 0.1568), or 0;

CLEC7A: expression level of the CLEC7A biomarker;

B5: coefficient for CLEC7A expression level, a rational number in the range of 0.8 to 1.3 or 1 to 1.1, (up to four decimal places, e.g., 1.0951), or 0;

VEGFA: expression level of the VEGFA biomarker;

B6: coefficient for VEGFA expression level, a rational number in the range of - 1.5 to -0.2 or -1 to -0.7 (up to four decimal places, e.g., -0.8407), or 0;

CCL2: expression level of the CCL2 biomarker;

B7: coefficient for CCL2 expression level, a rational number in the range of -1 to -0.1 or -0.7 to -0.5 (up to four decimal places, e.g., -0.5380 or -0.6622), or 0;

CXCR4: expression level of the CXCR4 biomarker;

B8: coefficient for CXCR4 expression level, a rational number in the range of - 1.5 to -0.5 or -1.1 to -0.7 (up to four decimal places, e.g., -0.9422), or 0;

CXCR2: expression level of the CXCR2 biomarker;

B9: coefficient for CXCR2 expression level, a rational number in the range of 0.7 to 1.7 or 0.9 to 1.5 (up to four decimal places, e.g., 1.4027 or 1.0804), or 0;

ARG1: expression level of the ARG1 biomarker;

B10: coefficient for ARG1 expression level, a rational number in the range of - 1.7 to -0.7 or -1.4 to -1 (up to four decimal places, e.g., -1.143 or -1.2971), or 0;

SLC27A2: expression level of the SLC27A2 biomarker;

B11: coefficient for SLC27A2 expression level, a rational number in the range of -5 to -4 or -4.8 to -4.3 (up to four decimal places, e.g., -4.3771 or -4.7971), or 0;

PTGES2: Expression level of the PTGES2 biomarker;

B12: coefficient for PTGES2 expression level, a rational number in the range of 2.7 to 3.5 or 3 to 3.3 (up to four decimal places, e.g., 3.1076 or 3.1934), or 0;

PTGES: expression level of the PTGES biomarker;

B13: coefficient for PTGES expression level, a rational number in the range of - 1 to -0.2 or -0.8 to -0.6 (up to four decimal places, e.g., -0.6396 or -0.7064), or 0;

with a proviso that at least one of B1 to B13 is not 0).

The expression levels of each biomarker applied in [Equation 1] may be values measured and quantified using ΔCt values obtained by real-time PCR (e.g., real-time qPCR; see Example 2).

The method for diagnosing early pancreatic cancer or the method for providing information for diagnosis of early pancreatic cancer may further include, after step (b-1), the following step of:

(c-1) comparing the value obtained in step (b-1) with a cut-off value.

In this regard, the cut-off value for diagnosis of early pancreatic cancer may be a rational number in the range of -2.5 to -1.5 or -2 to -1.8 (valid up to five or six decimal places), for example, -1.855994 or -1.90875.

If the numerical value obtained in step (b-1) exceeds the cut-off value, the subject may be predicted (determined or identified) as an early pancreatic cancer patient or at high risk of developing early pancreatic cancer. The determination of whether the value obtained in step (b-1) exceeds the cut-off value may be based on the comparison result in step (c-1). In another embodiment, if the numerical value obtained in step (b-1) is equal to or below the cut-off value, the subject may be predicted (determined or identified) as normal and/or at a low risk of developing pancreatic cancer (early pancreatic cancer).

Accordingly, the method for diagnosing early pancreatic cancer or the method for providing information for diagnosis of early pancreatic cancer may further comprise, after or simultaneously with step (c-1), the following step of:

(d-1) predicting (determining or identifying) the subject as an early pancreatic cancer patient or a high-risk individual for early pancreatic cancer when the value obtained in step (b-1) exceeds the cut-off value.

In another embodiment, the method for diagnosing early pancreatic cancer or the method for providing information for diagnosis of early pancreatic cancer may include the following step of:

(a-2) measuring an expression level of at least one selected from the group consisting of CCL2, CCL5, CXCR2, SLC27A2, IFNL1, IFNG, PTGES2, and TNF (for example, one or more (2, 3, 4, 5, 6, 7, or 8) selected from the group), a coding gene therefor, or combinations thereof in a blood sample isolated from a subject.

In an embodiment, the step of measuring a level of the biomarker is as described in the foregoing. For example, quantification of the biomarker levels may be performed (e.g., measuring Ct values, $\Delta$Ct values, and/or $\Delta\Delta$Ct values by PCR), and optionally by regression analysis (e.g., stepwise logistic regression analysis). Additional statistical analysis may be conducted as needed.

The method for diagnosing early pancreatic cancer or the method for providing information for diagnosis of early pancreatic cancer may further include, after step (a-2), the following step of:

(b-2) calculating using the following equation

$$\text{logit}(p) = B_0 + B_1 \times \text{IFNG} + B_2 \times \text{SLC27A2} + B_3 \times \text{CCL5} + B_4 \times \text{PTGES2} + B_5 \times \text{TNF} + B_6 \times \text{IFNL1} + B_7 \times \text{CCL2} + B_8 \times \text{CXCR2} \qquad \text{[Equation 2]}$$

(wherein:

B0: a rational number in the range of -5 to -0.1, -5 to -0.5, -5 to -1, -5 to -1.5, -2.5 to -0.1, -2.5 to -0.5, -2.5 to -1, or -2.5 to -1.5 (valid up to six decimal places), e.g., - 1.957576;

IFNG: expression level of IFNG biomarker;

B1: coefficient for IFNG biomarker expression, a rational number in the range of 1 to 3 or 1.7 to 2.3 (valid up to six decimal places, e.g., 1.942999), or 0;

SLC27A2: expression level of SLC27A2 biomarker;

B2: Coefficient for SLC27A2 biomarker expression, a rational number in the range of -3 to -1.5 or -2.5 to -2 (valid up to six decimal places, e.g., -2.370157), or 0;

CCL5: expression level of CCL5 biomarker;

B3: coefficient for CCL5 biomarker expression, a rational number in the range of 0.1 to 0.5 or 0.2 to 0.3 (valid up to six decimal places, e.g., 0.236554), or 0;

PTGES2: expression level of PTGES2 biomarker;

B4: coefficient for PTGES2 biomarker expression, a rational number in the range of 1 to 2.5 or 1.5 to 2 (valid up to six decimal places, e.g., 1.781672), or 0;

TNF: expression level of TNF biomarker;

B5: coefficient for TNF biomarker expression, a rational number in the range of - 2 to -0.5 or -1.5 to -1 (valid up to six decimal places, e.g., -1.328085), or 0;

IFNL1: expression level of IFNL1 biomarker;

B6: coefficient for IFNL1 biomarker expression, a rational number in the range of -0.5 to -0.1 or -0.3 to -0.1 (valid up to six decimal places, e.g., -0.170127), or 0;

CCL2: expression level of CCL2 biomarker;

B7: coefficient for CCL2 biomarker expression, a rational number in the range of -1 to -0.1 or -0.7 to -0.3 (valid up to six decimal places, e.g., -0.495828), or 0;

CXCR2: expression level of CXCR2 biomarker;

B8: coefficient for CXCR2 biomarker expression, a rational number in the range of 0.1 to 1 or 0.5 to 0.8 (valid up to six decimal places, e.g., 0.611586), or 0,

with a proviso that at least one of B1 to B8 is not 0.)

The expression level of each biomarker applied in [Equation 2] may be a value measured and quantified using Real-Time PCR (e.g., Real-time qPCR; see Example 2) based on the derived $\Delta$Ct. In an embodiment, to enhance predictive accuracy, the $\Delta$Ct value may undergo statistical processing using a predetermined statistical analysis method before being applied to [Equation 2]. Examples of applicable statistical analysis methods include Robust Scaler, among others. For instance, when applying Robust Scaler, the $\Delta$Ct value may be processed using [Equation 5] to obtain a transformed value (x'), which is then applied to [Equation 2]:

$$x' = [x - \text{median}(x)] / \text{IQR} \quad \text{[Equation 5]}$$

(x: the ΔCt value for each biomarker in the diagnostic subject; median (x): the median of the ΔCt values for the corresponding biomarker in a given population that includes the diagnostic subject; and IQR (interquartile range): in the range between the third quartile (Q3) and the first quartile (Q1), calculated as Q3 - Q1).

The method for diagnosing early-stage pancreatic cancer or the method for providing information for the diagnosis of early-stage pancreatic cancer may further include, after step (b-2), the following step of:

(c-2) comparing the value obtained in step (b-2) with a cut-off value.

In this regard, the cut-off value for early-stage pancreatic cancer diagnosis may be a rational number within the range of 0.1 to 0.5 or 0.2 to 0.3 (valid up to three decimal places), for example, 0.282.

When the value obtained in step (b-2) exceeds the cut-off value, the test subject may be predicted (determined, concluded) to be an early-stage pancreatic cancer patient or at high risk of developing early-stage pancreatic cancer. In this case, determining whether the value obtained in step (b-2) exceeds the cut-off value may be based on the comparison result in step (c-2). In another embodiment, when the value obtained in step (b-2) is equal to or below the cut-off value, the test subject may be predicted (determined, concluded) to be normal and/or at low risk of developing pancreatic cancer (early-stage pancreatic cancer).

Accordingly, the method for diagnosing early-stage pancreatic cancer or the method for providing information for the diagnosis of early-stage pancreatic cancer may further include, after or simultaneously with step (c-2), the following step of:

(d-2) predicting (determining, concluding) the test subject as an early-stage pancreatic cancer patient or as a patient at high risk of developing early-stage pancreatic cancer when the value obtained in step (b-2) exceeds the cut-off value.

In another embodiment, the method may be a method for diagnosing all pancreatic cancers (early-stage pancreatic cancer and/or late-stage pancreatic cancer) or a method for providing information for the diagnosis of all pancreatic cancers, including the following step of:

(a-3) measuring the level of at least one selected from the group consisting of TNF, IFNG, CXCL11, VEGFA, CCL2, CCL5, CXCR4, CXCR2, PTGS2, SLC27A2, and PTGES2 (for example, any one or more (2, 3, 4, 5, 6, 7, 8, 9, 10, or 11) selected from the group), a coding gene thereof, or a combination thereof in a blood sample isolated from the subject.

In an embodiment, the step of measuring a level of the biomarker is as described in the foregoing. For instance, the step of measuring a level of the biomarker may involve quantification of the biomarker (for example, measuring Ct values, ΔCt values, and/or ΔΔCt values by PCR) and, optionally, regression analysis (for example, stepwise logistic regression analysis). Additionally, appropriate statistical analyses may be further performed as needed.

In an embodiment, the method for diagnosing all pancreatic cancers or the method for providing information for the diagnosis of all pancreatic cancers may further include, after step (a-3), the following step of:

(b-3) calculating using the following equation:

$$\text{logit}(p) = B0 + B1 \times TNF + B2 \times IFNG + B3 \times CXCL11 + B4 \times VEGFA + B5 \times CCL2 + B6 \times CCL5 + B7 \times CXCR4 + B8 \times CXCR2 + B9 \times PTGS2 + B10 \times SLC27A2 \text{ --> } + B11 \times PTGES2 \qquad \text{[Equation 3]}$$

(wherein,

B0: a rational number selected from the ranges 30 to 40, 30 to 38, 30 to 37, 32.5 to 40, 32.5 to 38, 32.5 to 37, 36 to 40, 36 to 38, or 36 to 37 (valid up to four decimal places), e.g., 36.9898 or 36.6273;
TNF: expression level of the TNF biomarker;
B1: coefficient for the TNF biomarker, a rational number selected from -2.5 to -2 or -2.3 to -2.2 (valid up to four decimal places, e.g., -2.2200 or -2.2286), or 0;
IFNG: expression level of the IFNG biomarker;
B2: Coefficient for the IFNG biomarker, a rational number selected from 0.5 to 1.5 or 0.9 to 1.1 (valid up to four decimal places, e.g., 0.9955 or 1.0041), or 0;
CXCL11: Expression level of the CXCL11 biomarker;
B3: Coefficient for the CXCL11 biomarker, a rational number selected from 0.1 to 0.5 or 0.3 to 0.4 (valid up to four decimal places, e.g., 0.3413 or 0.3704), or 0;
VEGFA: Expression level of the VEGFA biomarker;
B4: Coefficient for the VEGFA biomarker, a rational number selected from -3.2 to -2.5 or -2.9 to -2.7 (valid up to four decimal places, e.g., -2.7990 or -2.7473), or 0;
CCL2: Expression level of the CCL2 biomarker;
B5: Coefficient for the CCL2 biomarker, a rational number selected from -0.3 to -0.01 or -0.25 to -0.15 (valid up to four decimal places, e.g., -0.1913 or -0.2240), or 0;
CCL5: Expression level of the CCL5 biomarker;
B6: Coefficient for the CCL5 biomarker, a rational number selected from 1 to 1.5 or 1.3 to 1.4 (valid up to four decimal places, e.g., 1.3628 or 1.3874), or 0;

CXCR4: Expression level of the CXCR4 biomarker;

B7: Coefficient for the CXCR4 biomarker, a rational number selected from -2.5 to -1.5 or -2.2 to -2 (valid up to four decimal places, e.g., -2.0521 or -2.0986), or 0;

CXCR2: Expression level of the CXCR2 biomarker;

B8: Coefficient for the CXCR2 biomarker, a rational number selected from 0.2 to 1.2 or 0.6 to 0.8 (valid up to four decimal places, e.g., 0.7037 or 0.6388), or 0;

PTGS2: Expression level of the PTGS2 biomarker;

B9: Coefficient for the PTGS2 biomarker, a rational number selected from -0.01 to -0.1 (valid up to four decimal places, e.g., -0.0640), or 0;

SLC27A2: Expression level of the SLC27A2 biomarker;

B10: Coefficient for the SLC27A2 biomarker, a rational number selected from - 3.5 to -3 or -3.3 to -3.1 (valid up to four decimal places, e.g., -3.1754 or -3.1990), or 0;

PTGES2: Expression level of the PTGES2 biomarker;

B11: Coefficient for the PTGES2 biomarker, a rational number selected from 4 to 5 or 4.4 to 4.6 (valid up to four decimal places, e.g., 4.4765), or 0;

with a proviso that at least one of B1 to B11 is not 0.)

The expression level of each biomarker applied in Equation 3 may be a value measured or quantified based on the $\Delta$Ct values obtained through Real-Time PCR (e.g., Real-time qPCR; see Example 2).

The method for diagnosing all pancreatic cancers or providing information for the diagnosis of any pancreatic cancer, regardless of disease progression stage, may further include, after step (b-3), the following step of:

(c-3) comparing the value obtained in step (b-3) with a cut-off value.

In this regard, the cut-off value for diagnosing all pancreatic cancers may be a rational number in the range of -3.5 to -2 or -3 to -2.5 (valid up to five decimal places), for example, -0.25109 or -0.29033.

when the value obtained in step (b-3) exceeds the cut-off value, the test subject may be predicted (determined, decided) to be a pancreatic cancer patient or to have a high risk of developing pancreatic cancer. In this case, determining whether the value obtained in step (b-3) exceeds the cut-off value may be based on the comparison result in step (c-3). In another embodiment, when the value obtained in step (b-3) is equal to or below the cut-off value, the test subject may be predicted (determined, decided) to be normal and/or to have a low risk of developing pancreatic cancer.

Accordingly, the method for diagnosing all pancreatic cancers or providing information for the diagnosis of all pancreatic cancers may further include, after or simultaneously with step (c-3), the following step of:

(d-3) predicting (determining, deciding) the test subject as a pancreatic cancer patient or as a patient with a high risk of developing pancreatic cancer when the value obtained in step (c-3) exceeds the cut-off value.

In another embodiment, the method may be a method for diagnosing all pancreatic cancers (early pancreatic cancer and/or late-stage pancreatic cancer) or a method for providing information for the diagnosis of all pancreatic cancers, including the following step of:

(a-4) measuring a level of at least one selected from the group consisting of CCL2, CCL5, CXCR2, SLC27A2, IFNL1, IFNG, PTGES2, and TNF (for example, any one or two or more (e.g., 2, 3, 4, 5, 6, 7, or 8) selected from the group), a coding gene therefor, or a combination thereof in a blood sample isolated from a subject.

In an embodiment, the step of measuring a level of the biomarker is as described above. For example, the step of measuring a level of the biomarker may be performed by quantifying the biomarker (e.g., in the case of PCR, measuring Ct values, $\Delta$Ct values, and/or $\Delta\Delta$Ct values) and, optionally, performing regression analysis (e.g., stepwise logistic regression analysis). Additionally, appropriate statistical analyses may be performed as needed.

The method for diagnosing all pancreatic cancers or providing information for the diagnosis of all pancreatic cancers may further include, after step (a-4), the following step of:

(b-4) calculating using the following equation:

$$\text{logit}(p) = B0 + B1 \times \text{IFNG} + B2 \times \text{SLC27A2} + B3 \times \text{CCL5} + B4 \times \text{PTGES2} + B5 \times \text{TNF} + B6 \times \text{IFNL1} + B7 \times \text{CCL2} + B8 \times \text{CXCR2} \qquad \text{[Equation 4]}$$

(wherein,

B0: a rational number within the range of 0.1 to 1, 0.1 to 0.8, 0.5 to 1, or 0.5 to 0.8 (valid up to six decimal places), for example, 0.654363;

IFNG: expression level of the biomarker IFNG;

B1: coefficient for the expression level of the biomarker IFNG, a rational number within the range of 0.5 to 2 or 1.2 to 1.6 (valid up to six decimal places, e.g., 1.397767) or 0;

SLC27A2: expression level of the biomarker SLC27A2;

B2: coefficient for the expression level of the biomarker SLC27A2, a rational number within the range of -2.5 to -1 or -2 to -1.6 (valid up to six decimal places, e.g., - 1.874652) or 0;

CCL5: expression level of the biomarker CCL5;

B3: coefficient for the expression level of the biomarker CCL5, a rational number within the range of 0.1 to 1.2 or 0.6 to 1 (valid up to six decimal places, e.g., 0.855608) or 0;

PTGES2: expression level of the biomarker PTGES2;

B4: coefficient for the expression level of the biomarker PTGES2, a rational number within the range of 1 to 2.5 or 1.5 to 2 (valid up to six decimal places, e.g., 1.782533) or 0;

TNF: expression level of the biomarker TNF;

B5: coefficient for the expression level of the biomarker TNF, a rational number within the range of -3 to -1.5 or -2.5 to -2.1 (valid up to six decimal places, e.g., - 2.378006) or 0;

IFNL1: expression level of the biomarker IFNL1;

B6: coefficient for the expression level of the biomarker IFNL1, a rational number within the range of -0.0002 to -0.0001 (valid up to six decimal places, e.g., - 0.000140) or 0;

CCL2: expression level of the biomarker CCL2;

B7: coefficient for the expression level of the biomarker CCL2, a rational number within the range of -1 to -0.1 or -0.7 to -0.3 (valid up to six decimal places, e.g., -0.535484) or 0;

CXCR2: expression level of the biomarker CXCR2;

B8: coefficient for the expression level of the biomarker CXCR2, a rational number within the range of 0.1 to 0.7 or 0.2 to 0.4 (valid up to six decimal places, e.g., 0.300359) or 0,

with a proviso that at least one of B1 to B8 is not 0.)

The expression level of each biomarker applied in [Equation 4] may be a value measured and quantified as ΔCt obtained through Real-Time PCR (e.g., Real-time qPCR; see Example 2). In an specific embodiment, to improve predictive accuracy, the ΔCt values may be statistically processed using a predetermined statistical analysis method before being applied to Equation 4. An example of a usable statistical analysis method is Robust Scaler. For example, when applying Robust Scaler, the ΔCt values may be processed using the following equation [Equation 5], and the resulting value (x') may be applied to Equation 4:

$$x'=[x - median(x)]/IQR \text{ [Equation 5]}$$

(x: ΔCt value for each marker of the diagnostic subject; median(x): the median of the ΔCt values for each marker in an arbitrary population that includes the diagnostic subject; IQR (Interquartile Range): the range between the third quartile (Q3) and the first quartile (Q1) (Q3 - Q1)).

The method for diagnosing all pancreatic cancers or providing information for the diagnosis of all pancreatic cancers may further include, after step (b-4), the following step of:

(c-4) comparing the value obtained in step (b-4) with a cut-off value.

In this regard, the cut-off value for diagnosing all pancreatic cancers may be a rational number within the range of 0.3 to 1.2 or 0.5 to 0.9 (valid up to three decimal places), for example, 0.732.

When the value obtained in step (b-4) exceeds the cut-off value, the test subject may be predicted (determined, decided) to be a pancreatic cancer (all pancreatic cancers) patient or to have a high risk of developing pancreatic cancer (all pancreatic cancers). In this case, determining whether the value obtained in step (b-4) exceeds the cut-off value may be based on the comparison result in step (c-4). In another embodiment, when the value obtained in step (b-4) is equal to or less than the cut-off value, the test subject may be predicted (determined, decided) to be normal and/or to have a low risk of developing pancreatic cancer (all pancreatic cancers).

Accordingly, the method for diagnosing all pancreatic cancers or providing information for diagnosing all pancreatic cancers may further include, after or simultaneously with step (c-4), the following step of:

(d-4) predicting (determining, deciding) the subject as a patient with all pancreatic cancers or as a patient with a high risk of developing all pancreatic cancers when the value obtained in step (b-4) exceeds the cut-off value.

The method for diagnosing pancreatic cancer or providing information for the diagnosis of pancreatic cancer provided in the present disclosure may further include, after the detection step, comparison step, or diagnosis (prediction, determination, or decision) step,

a step of performing pancreatic cancer treatment on a subject diagnosed as a patient with pancreatic cancer (early pancreatic cancer, late-stage pancreatic cancer, or both).

The pancreatic cancer treatment may refer to chemotherapy, such as the administration of a pancreatic cancer therapeutic agent (e.g., anticancer agents: 5-fluorouracil (5-FU), gemcitabine, Tarceva (erlotinib), antibodies, etc.), radiotherapy, surgical procedures, or a combination of two or more thereof.

The pancreatic cancer treatment may be applied differently depending on the stage and progression of pancreatic cancer. Early pancreatic cancer (e.g., resectable pancreatic cancer) is a stage of pancreatic cancer that can be treated through surgical procedures. The treatment of early pancreatic cancer may involve surgical procedures alone or performing adjuvant chemotherapy following surgical resection. Adjuvant chemotherapy refers to anticancer therapy other than surgical procedures and may include chemotherapy such as the administration of pancreatic cancer therapeutic agents (e.g., one or more anticancer agents selected from the group consisting of 5-fluorouracil (5-FU), gemcitabine, Tarceva (erlotinib), leucovorin, irinotecan, oxaliplatin, Folfirinox, Abraxane, Onivyde, TS-1, capecitabine, antibodies, etc.), chemoradiotherapy, or a combination thereof.

The treatment of late-stage pancreatic cancer (e.g., borderline resectable pancreatic cancer, locally late-stage pancreatic cancer, metastatic pancreatic cancer) may be applied differently depending on the stage of progression of each type of pancreatic cancer.

For example, for borderline resectable pancreatic cancer among late-stage pancreatic cancers, neoadjuvant therapy (e.g., administration of one or more anticancer agents selected from the group consisting of 5-fluorouracil (5-FU), leucovorin, irinotecan, oxaliplatin, etc. (e.g., FOLFIRINOX, which involves the combined administration of the four anticancer agents)) may be performed. Thereafter, depending on the treatment outcome, (i) if surgical resection is possible, adjuvant chemotherapy may be performed after surgery, and (ii) if surgical resection is not possible, chemotherapy may be performed.

For locally late-stage pancreatic cancer among later-stage pancreatic cancers, neoadjuvant therapy (e.g., administration of one or more anticancer agents selected from the group consisting of 5-fluorouracil (5-FU), leucovorin, irinotecan, oxaliplatin, etc. (e.g., FOLFIRINOX, which involves the combined administration of the four anticancer agents)) may be performed. Thereafter, depending on the progression outcome, continued neoadjuvant therapy or secondary treatment (e.g., combination therapy of nanoparticle albumin-bound paclitaxel (nab-paclitaxel) and gemcitabine), chemoradiotherapy, or a combination of two or more thereof may be performed.

For metastatic pancreatic cancer among late-stage pancreatic cancers, neoadjuvant therapy (e.g., administration of one or more anticancer agents selected from the group consisting of 5-fluorouracil (5-FU), leucovorin, irinotecan, oxaliplatin, etc. (e.g., FOLFIRINOX, which involves the combined administration of the four anticancer agents)), chemotherapy (e.g., combination therapy of nanoparticle albumin-bound paclitaxel (nab-paclitaxel) and gemcitabine, or administration of gemcitabine alone), or a combination thereof may be performed. Thereafter, depending on the patient's condition, secondary treatment may be performed. The secondary treatment may refer to chemotherapy, including, for example, the combination therapy of nanoparticle albumin-bound paclitaxel (nab-paclitaxel) and gemcitabine, combination therapy of 5-fluorouracil (5-FU) and liposomal irinotecan, or combination therapy of 5-fluorouracil (5-FU) and liposomal oxaliplatin.

Blood Sample

The diagnostic target sample or blood sample applicable to the composition, kit, and method for diagnosing pancreatic cancer provided in the present disclosure may include a liquid biopsy obtained (or isolated or derived) from a diagnostic subject, such as blood, serum, plasma, and/or cells isolated therefrom. In an embodiment, the sample may include a buffy coat isolated from the diagnostic subject.

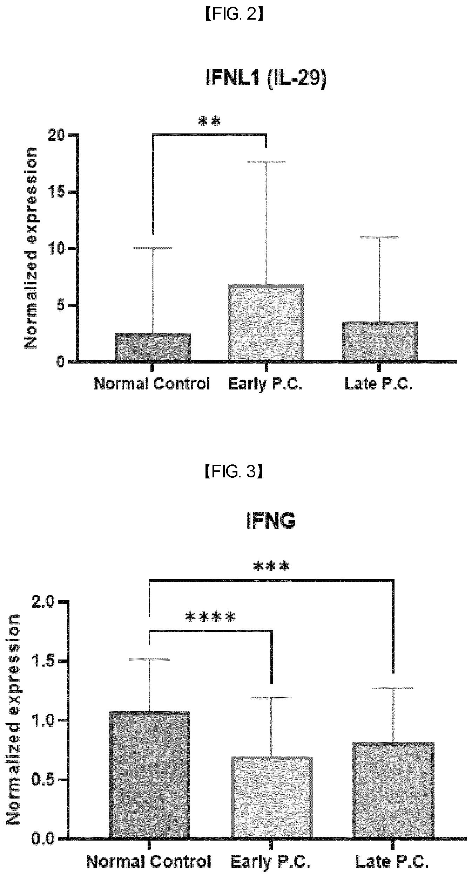

As used herein, the term "buffy coat" refers to the entire leukocyte layer formed between the upper plasma layer and the lower red blood cell layer during the centrifugation of blood. It is a mixture of components in blood excluding plasma and red blood cells (e.g., monocytes, granulocytes, lymphocytes, etc.) and is clearly distinguished from peripheral blood mononuclear cells (PBMCs) (see FIG. 3). The blood sample used in the present disclosure may be a blood-derived buffy coat and may not be a sample consisting solely of peripheral blood mononuclear cells. In an embodiment, the buffy coat may be obtained from the intermediate buffy coat layer among the sequentially separated plasma layer (top), buffy coat layer (middle), and red blood cell layer (bottom) after centrifuging the blood under one or more (1, 2, or 3) selected conditions from the following conditions (1) to (3):

(1) Temperature: 2 to 30°C, 2 to 28°C, 2 to 25°C, 2 to 23°C, 2 to 20°C, 2 to 18°C, 2 to 15°C, 2 to 13°C, 2 to 10°C, 2 to 8°C, 2 to 6°C, 2 to 5°C, 2 to 4°C, 3 to 30°C, 3 to 28°C, 3 to 25°C, 3 to 23°C, 3 to 20°C, 3 to 18°C, 3 to 15°C, 3 to 13°C, 3 to 10°C, 3 to 8°C, 3 to 6°C, 3 to 5°C, 3 to 4°C, 4 to 30°C, 4 to 28°C, 4 to 25°C, 4 to 23°C, 4 to 20°C, 4 to 18°C, 4 to 15°C, 4 to 13°C, 4 to 10°C, 4 to 8°C, 4 to 6°C, 4 to 5°C, 5 to 30°C, 5 to 28°C, 5 to 25°C, 5 to 23°C, 5 to 20°C, 5 to 18°C, 5 to 15°C, 5 to 13°C, 5 to 10°C, 5 to 8°C, 5 to 6°C, 10 to 30°C, 10 to 28°C, 10 to 25°C, 10 to 23°C, 10 to 20°C, 10 to 18°C, 10 to 15°C, 10 to 13°C, 15 to 30°C, 15 to 28°C, 15 to 25°C, 15 to 23°C, 15 to 20°C, 15 to 18°C, 20 to 30°C, 20 to 28°C, 20 to 25°C, or 20 to 23°C;

(2) Speed: 300g to 2000g, 300g to 1800g, 300g to 1500g, 300g to 1300g, 300g to 1000g, 500g to 2000g, 500g to 1800g, 500g to 1500g, 500g to 1300g, 500g to 1000g, 600g to 1000g, 700g to 1000g, 800g to 1000g, 500g to 900g, 600g to 900g, 700g to 900g, 800g to 900g, 500g to 800g, 600g to 800g, or 700g to 800g; and

(3) Time: Duration for 5 to 20 minutes, 7 to 20 minutes, 9 to 20 minutes, 5 to 15 minutes, 7 to 15 minutes, 9 to 15 minutes, 5 to 12 minutes, 7 to 12 minutes, or 9 to 12 minutes.

In the present disclosure, the diagnostic subject (individual) may be selected from mammals, including primates such as humans and monkeys, rodents such as mice and rats, and other species requiring pancreatic cancer diagnosis.

In the present disclosure, the comparison sample may be a sample obtained (or isolated or derived) from a normal individual (e.g., an individual who does not have pancreatic cancer (early pancreatic cancer, late-stage pancreatic cancer, or both)) or an individual with a similar disease (benign pancreatic diseases, such as pancreatitis (chronic pancreatitis and/or acute pancreatitis), benign pancreatic tumors, intraductal papillary mucinous neoplasm (IPMN), autoimmune pancreatitis (AIP), and other pancreatic diseases (excluding pancreatic cancer (malignant pancreatic tumor)), and/or cholangiocarcinoma). For example, the normal individual may be selected from mammals, including primates such as humans and monkeys, rodents such as mice and rats, and may be an individual of the same species as the diagnostic subject. The comparison sample may include blood, serum, plasma, and/or cells isolated therefrom, obtained (or isolated or derived) from the comparison subject. Specifically, the comparison sample may include a buffy coat.

Screening of Pancreatic Cancer Therapeutics

Another embodiment provides a method for screening candidate compounds for pancreatic cancer therapeutics by measuring a level of the biomarker upon treatment with a candidate compound.

More specifically, the screening method may include the steps of:

contacting a biological sample with a candidate compound;
measuring a level of at least one biomarker selected from the previously described biomarkers and a coding gene thereof in the biological sample; and
comparing a level of the biomarker in the biological sample treated with the candidate compound with a level of the biomarker or a coding gene therefor in a biological sample that has not been treated with the candidate compound.

The comparison step may be performed by measuring and comparing the biomarker levels in the same biological sample before and after treatment with the candidate compound, or by bringing only a portion of the biological sample into contact with the candidate compound, measuring the biomarker levels in the treated portion and non-contacted portion, and comparing the levels.

When the biomarker level in the biological sample treated with the candidate compound is lower than that in the untreated biological sample, that is, when the candidate compound reduces the level of the biomarker or inhibits the expression of the biomarker, it may be determined that the candidate compound is a potential agent for the prevention and/or treatment of pancreatic cancer.

The biological sample may encompass blood, serum, plasma, and/or cells derived (isolated or derived) from a living organism, for example, a pancreatic cancer patient. More specifically, the biological sample may include buffy coat.

The candidate compound may be selected from various types of compounds, including small molecules, proteins, polypeptides, oligopeptides, polynucleotides, oligonucleotides, or extracts derived from plants or animals.

The measurement of biomarker levels in the biological sample may be performed using conventional gene or protein quantification methods and/or by evaluating the measurement results. Specific measurement methods may be as described previously.

All numerical values provided herein should be interpreted as including the conventional margin of error, as long as the desired function and/or effect of the present application can be achieved. For example, the values may be interpreted to include a range of -10% (or -9%, -8%, -7%, -6%, -5%, -4%, -3%, -2%, or -1%) to +10% (or +9%, +8%, +7%, +6%, +5%, +4%, +3%, +2%, or +1%), but are not limited thereto.

**Advantageous Effects**

Through the present application, pancreatic cancer can be diagnosed in a non-invasive and simple manner with high accuracy using only patient-derived blood. Furthermore, the progression stage of pancreatic cancer can be more accurately diagnosed, and differentiation from other similar diseases can be achieved, thereby enabling the development of more effective treatment strategies for pancreatic cancer.

**Description of Drawings**

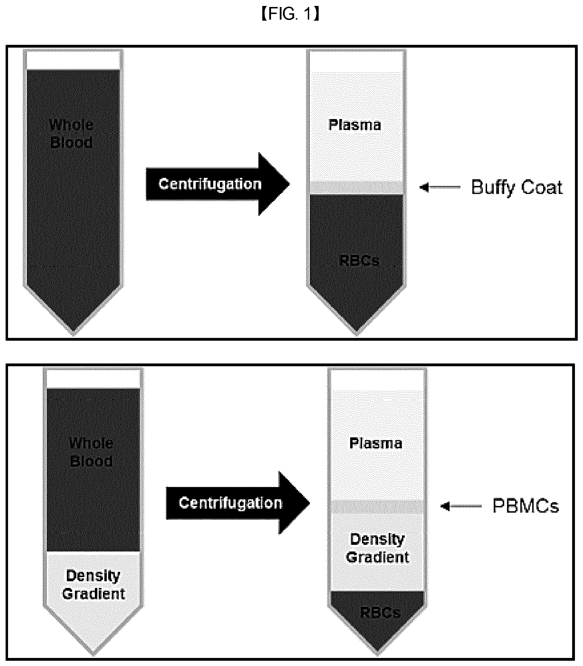

FIG. 1 is a schematic diagram exemplarily illustrating the buffy coat sample preparation process in comparison with the PBMC sample.

FIG. 2 is a graph showing the Real-time qPCR results (ΔΔCt values) for IFNL1 in the normal control group, early pancreatic cancer patient group (Early P.C.), and late pancreatic cancer patient group (Late P.C.).

FIG. 3 is a graph showing the Real-time qPCR results (ΔΔCt values) for IFNG in the normal control group, early pancreatic cancer patient group, and late pancreatic cancer patient group.

FIG. 4 is a graph showing the Real-time qPCR results (ΔΔCt values) for CXCL11 in the normal control group, early pancreatic cancer patient group, and late pancreatic cancer patient group.

FIG. 5 is a graph showing the Real-time qPCR results (ΔΔCt values) for TNF in the normal control group, early pancreatic cancer patient group, and late pancreatic cancer patient group.

FIG. 6 is a graph showing the Real-time qPCR results (ΔΔCt values) for CLEC7A in the normal control group, early pancreatic cancer patient group, and late pancreatic cancer patient group.

FIG. 7 is a graph showing the Real-time qPCR results (ΔΔCt values) for CXCL8 in the normal control group, early pancreatic cancer patient group, and late pancreatic cancer patient group.

FIG. 8 is a graph showing the Real-time qPCR results (ΔΔCt values) for FOXP3 in the normal control group, early pancreatic cancer patient group, and late pancreatic cancer patient group.

FIG. 9 is a graph showing the Real-time qPCR results (ΔΔCt values) for VEGFA in the normal control group, early pancreatic cancer patient group, and late pancreatic cancer patient group.

FIG. 10 is a graph showing the Real-time qPCR results (ΔΔCt values) for CCL2 in the normal control group, early pancreatic cancer patient group, and late pancreatic cancer patient group.

FIG. 11 is a graph showing the Real-time qPCR results (ΔΔCt values) for CCL5 in the normal control group, early pancreatic cancer patient group, and late pancreatic cancer patient group.

FIG. 12 is a graph showing the Real-time qPCR results (ΔΔCt values) for CCR5 in the normal control group, early pancreatic cancer patient group, and late pancreatic cancer patient group.

FIG. 13 is a graph showing the Real-time qPCR results (ΔΔCt values) for CXCR4 in the normal control group, early pancreatic cancer patient group, and late pancreatic cancer patient group.

FIG. 14 is a graph showing the Real-time qPCR results (ΔΔCt values) for ARG1 in the normal control group, early pancreatic cancer patient group, and late pancreatic cancer patient group.

FIG. 15 is a graph showing the Real-time qPCR results (ΔΔCt values) for CXCR2 in the normal control group, early pancreatic cancer patient group, and late pancreatic cancer patient group.

FIG. 16 is a graph showing the Real-time qPCR results (ΔΔCt values) for PTGS2 in the normal control group, early pancreatic cancer patient group, and late pancreatic cancer patient group.

FIG. 17 is a graph showing the Real-time qPCR results (ΔΔCt values) for PTGES2 in the normal control group, early pancreatic cancer patient group, and late pancreatic cancer patient group.

FIG. 18 is a graph showing the Real-time qPCR results (ΔΔCt values) for SLC27A2 in the normal control group, early pancreatic cancer patient group, and late pancreatic cancer patient group.

FIG. 19 is a graph showing the Real-time qPCR results (ΔΔCt values) for PTGES in the normal control group, early pancreatic cancer patient group, and late pancreatic cancer patient group.

FIG. 20 is a graph showing the Real-time qPCR results (ΔΔCt values) for IFNB1 in pancreatic cancer patient groups and benign pancreatic disease patient groups.

FIG. 21 is a graph showing the Real-time qPCR results (ΔΔCt values) for IFNB1 in pancreatic cancer patient groups and cholangiocarcinoma patient groups.

FIG. 22 is a graph showing the Real-time qPCR results (ΔΔCt values) for IFNA1 in pancreatic cancer patient groups and benign pancreatic disease patient groups.

FIG. 23 shows ROC curve analysis using a combination of 9 markers (ARG1, CCL2, CLEC7A, SLC27A2, IFNG, PTGES, PTGES2, TNF, and FOXP3) in normal controls and early pancreatic cancer patients.

FIG. 24 shows ROC curve analysis using a combination of 9 markers (CCL5, PTGS2, CXCR2, CXCR4, SLC27A2, CXCL11, PTGES2, TNF, and VEGFA) in normal controls and late pancreatic cancer patients.

FIG. 25 shows ROC curve analysis using a combination of 9 markers (ARG1, CCL2, SLC27A2, IFNG, PTGES2, TNF, VEGFA, CXCR4, and CCL5) in normal controls and all pancreatic cancer patients.

FIG. 26a shows ROC curve analysis using all of 20 markers in normal controls and early-stage pancreatic cancer patients.

FIG. 26b shows ROC curve analysis using all of 20 markers in normal controls and late-stage pancreatic cancer patients.

FIG. 26c shows ROC curve analysis using all of 20 markers in normal controls and all pancreatic cancer patients.

FIG. 27a shows ROC curve analysis using 12 markers (CCL5, CXCR4, SLC27A2, PTGES2, TNF, VEGFA, ARG1, CCL2, CLEC7A, IFNG, PTGES, and FOXP3) in normal controls and early-stage pancreatic cancer patients.

FIG. 27b shows ROC curve analysis using 12 markers (CCL5, CXCR4, SLC27A2, PTGES2, TNF, VEGFA, ARG1, CCL2, CLEC7A, IFNG, PTGES, and FOXP3) in normal controls and late-stage pancreatic cancer patients.

FIG. 27c shows ROC curve analysis using 12 markers (CCL5, CXCR4, SLC27A2, PTGES2, TNF, VEGFA, ARG1, CCL2, CLEC7A, IFNG, PTGES, and FOXP3) in normal controls and all pancreatic cancer patients.

FIG. 28a shows ROC curve analysis using 15 markers (CCL5, CXCR4, SLC27A2, PTGES2, TNF, VEGFA, ARG1, CCL2, CLEC7A, IFNG, PTGES, FOXP3, PTGS2, CXCR2, and CXCL11) in normal controls and early-stage pancreatic cancer patients.

FIG. 28b shows ROC curve analysis using 15 markers (CCL5, CXCR4, SLC27A2, PTGES2, TNF, VEGFA, ARG1, CCL2, CLEC7A, IFNG, PTGES, FOXP3, PTGS2, CXCR2, and CXCL11) in normal controls and late-stage pancreatic cancer patients.

FIG. 28c shows ROC curve analysis using 15 markers (CCL5, CXCR4, SLC27A2, PTGES2, TNF, VEGFA, ARG1, CCL2, CLEC7A, IFNG, PTGES, FOXP3, PTGS2, CXCR2, and CXCL11) in normal controls and all pancreatic cancer patients.

FIG. 29a shows ROC curve analysis using 8 markers (CCL2, CCL5, CXCR2, SLC27A2, IFNL1, IFNG, PTGES2, and TNF) in normal controls and early-stage pancreatic cancer patients.

FIG. 29b shows ROC curve analysis using 8 markers (CCL2, CCL5, CXCR2, SLC27A2, IFNL1, IFNG, PTGES2, and TNF) in normal controls and late-stage pancreatic cancer patients.

FIG. 29c shows ROC curve analysis using 8 markers (CCL2, CCL5, CXCR2, SLC27A2, IFNL1, IFNG, PTGES2, and TNF) in normal controls and all pancreatic cancer patients.

FIG. 30 is table in which the results from FIGS. 23 to 29C are summarized.

**Mode for Invention**

forth to illustrate, but are not to be construed to limit, the scope of the present invention.

EXAMPLE 1: Sample Preparation

To investigate the expression patterns of pancreatic cancer biomarkers in pancreatic cancer, the mRNA expression levels of various markers were measured using buffy coat samples derived from the blood of patients with pancreatic cancer, patients with benign pancreatic diseases (including 3 cases of pancreatitis (chronic and acute pancreatitis), 12 cases of benign pancreatic tumors, 1 case of intraductal papillary mucinous neoplasm (IPMN), 2 cases of autoimmune pancreatitis (AIP), and other pancreatic diseases; a total of 20 cases), and patients with cholangiocarcinoma (40 cases). Additionally, for comparison, the same experiment was conducted using buffy coat samples derived from the blood of healthy individuals who did not have pancreatic cancer (normal control group).

First, blood samples (8 mL each) were collected from 105 patients with pancreatic cancer (36 cases of early-stage pancreatic cancer and 69 cases of late-stage pancreatic cancer) and 183 healthy individuals. The collected blood samples were placed into EDTA tubes and centrifuged at 1,800g for 10 minutes at 24°C within 2 hours of collection. The upper layer of the centrifuged blood, which was sequentially separated into plasma, buffy coat, and red blood cells, was processed by removing the plasma layer. Then, 250 μL of the buffy coat was isolated into a storage tube and stored at -80°C in a deep freezer.

The classification of pancreatic cancer patients into early-stage and late-stage groups was conducted according to the criteria presented in Table 2 below:

TABLE 2

| Stage criterium | | Classification upon statistical analysis |
|---|---|---|
| RPC | Resectable Pancreatic Cancer | Early-stage pancreatic cancer (Early PC) |
| BRPC | Borderline Resectable Pancreatic Cancer | Late-stage pancreatic cancer (Late PC) |
| LAPC | Locally late-stage pancreatic cancer | Late-stage pancreatic cancer (Late PC) |
| MPC | Metastatic Pancreatic Cancer | Late-stage pancreatic cancer (Late PC) |

EXAMPLE 2: Real-time qPCR

The gene expression in the samples prepared in Example 1 was measured using Real-time qPCR, and the Real-time qPCR was conducted using a probe-based multiplex PCR approach.

First, total RNA was extracted from 250 μL of frozen buffy coat samples (prepared in Example 1) using the NucleoSpin® RNA Blood Kit (MACHEREY-NAGEL) following the manufacturer's recommended protocol. One μg of RNA

from each sample was used for cDNA synthesis, which was performed using the GoScript™ Reverse Transcription System Kit (Promega).

For multiplex PCR, each marker probe was labeled with FAM dye, while GAPDH that serves as an internal reference gene was labeled with HEX dye. Primers and probes were purchased from IDT (Integrated DNA Technologies, Inc.).

To assess the expression of each gene in the samples, reactions were prepared in a final volume of 20 μL according to the protocol of the GoTaq® Probe qPCR Master Mix (Promega). The gene expression assay was conducted using the QuantStudio 3 and 5 Real-Time PCR Systems (Applied Biosystems) under Standard Cycling conditions provided by the instrument's software. In the Real-time qPCR results, the Ct values of each marker's mRNA expression level and the Ct value of GAPDH mRNA expression level were extracted. The difference between the mRNA expression level of each marker and GAPDH mRNA expression level was calculated to obtain the ΔCt value. To compare mRNA expression levels of each marker between healthy individuals and pancreatic cancer patients or between pancreatic cancer and benign pancreatic disease/cholangiocarcinoma groups, ΔΔCt values were calculated using the following formula: ΔΔCt = ΔCt (pancreatic cancer or benign pancreatic disease/cholangiocarcinoma sample) - ΔCt (average of healthy individuals). To visualize the increase or decrease in mRNA expression levels of each marker in pancreatic cancer patients compared to healthy individuals or benign pancreatic disease patients, values were converted using the $2^{-\Delta\Delta Ct}$ formula and analyzed using ANOVA. All other statistical analyses were conducted using ΔCt values. A higher ΔΔCt value indicates increased gene expression while a lower ΔΔCt value indicates decreased gene expression. Conversely, a decrease in the ΔCt value indicates actual gene expression upregulation, whereas an increase in the ΔCt value indicates actual gene expression downregulation.

The markers used in the experiment are listed in Table 3, and the nucleotide sequences (5'→3') of the primers and probes for each marker are provided in Table 4 below:

TABLE 3

| No. | Gene name | Ref. sequence (NCBI) | IDT Assay Name |
|---|---|---|---|
| 1 | IL29(IFNL1) | NM_172140 | Hs.PT.56a.21113836.g |
| 2 | IFNG | NM_000619 | Hs.PT.58.3781960 |
| 3 | CXCL11(I-TAC) | NM_005409 | Hs.PT.58.26723814 |
| 4 | TNF | NM_000594 | Hs. PT. 58.45380900 |
| 5 | IFNB1 | NM_002176 | Hs.PT.58.39481063 |
| 6 | IFNA1 | NM_024013 | Hs.PT.58.46311748.g |
| 7 | DECTIN-1(CLEC7A) | NM_022570(5) | Hs.PT.58.40018774 |
| 8 | IL-8(CXCL8) | NM_000584 | Hs.PT.58.38869678.G |
| 9 | FOXP3 | NM_014009 | Hs.PT.58.3671186 |
| 10 | VEGFA | NM_003376 | Hs.PT.58.1149801 |
| 11 | CCL2 | NM_002982 | Hs. PT. 58.45467977 |
| 12 | CCL5 | NM_002985 | Hs.PT.58.1724551 |
| 13 | CCR5 | NM_000579 | Hs.PT.58.2249633 |
| 14 | CXCR4 | NM_003467 | Hs.PT.58.22298491 |
| 15 | ARG1 | NM_001244438 | Hs.PT.56a.20779559 |
| 16 | CXCR2 | NM_001557 | Hs. PT. 58.40527202 |
| 17 | SLC27A2(FATP2) | NM_003645 | Hs.PT.58.38450787 |
| 18 | PTGS2(COX2) | NM_000963 | Hs.PT.58.77266 |
| 19 | PTGES2 | NM_198938 | Hs.PT.58.40424722 |
| 20 | PTGES(PTGES1) | NM_004878 | Hs.PT.58.39853040 |

TABLE 4

| Marker | | Sequence | SEQ ID NO |
|---|---|---|---|
| IL-29 (IFNL1) | Primer-F | 5'-GGTTCAAATCTCTGTCACCACA-3' | 1 |
| | Primer-R | 5'-GAAGACAGGAGAGCTGCAAC-3' | 2 |
| | Probe | 5'-FAM/TCAAGAAGG/ZEN/CCAGGGACGCC/IBFQ-3' | 3 |
| IFNG | Primer-F | 5'-gcaa caa aaa gaaa cga gat gac-3' | 4 |
| | Primer-R | 5'-cga cag ttc agc cat cac tt-3' | 5 |
| | Probe | 5'-/56-FAM/tcg gta act/ZEN/gac ttg aat gtc aa cgc/3IABkFQ/-3' | 6 |
| CXCL11 (I-TAC) | Primer-F | 5'-tat tgt gtg cta cag ttg ttc aag-3' | 7 |
| | Primer-R | 5'-ggt aca tta tgg agg ctt tct ca-3' | 8 |
| | Probe | 5'-/56-FAM/tgc cac ttt/ZEN/cac tgc ttt tac ccc a/3IABkFQ/-3' | 9 |
| TNF | Primer-F | 5'-tgcactttggagtgatcgg-3' | 10 |
| | Primer-R | 5'-tcagcttgagggtttgctac-3' | 11 |
| | Probe | 5'-/56-FAM/agatgatct/ZEN/gactgcctgggcc/3IABkFQ/-3' | 12 |
| IFNB1 | Primer-F | 5'-gaaactgaagatctcctagcct-3' | 13 |
| | Primer-R | 5'-gccatcagtcacttaaacagc-3' | 14 |
| | Probe | 5'-/56-FAM/tgaagcaat/ZEN/tgtccagtcccagagg/3IABkFQ/-3' | 15 |
| IFNA1 | Primer-F | 5'-ggaggttgtcagagcaga-3' | 16 |
| | Primer-R | 5'-aatgacagaattcatgaaagcgt-3' | 17 |
| | Probe | 5'-/56-FAM/tgagatccc/ZEN/tctctttatcaacaaacttgcaa/3IABkFQ/-3' | 18 |
| DECTIN1 (CLEC7A ) | Primer-F | 5-GAC TCT CAA AGC AAT ACC AGG A-3' | 19 |
| | Primer-R | 5'-CCA CAG CTA TCA CCA GTA TTA CC-3' | 20 |
| | Probe | 5'-/56-FAM/TGC AGC ACA/ZEN/CGA TCC TTT CTC TGA A/3IABkFQ/-3' | 21 |
| IL-8 (CXCL8) | Primer-F | 5'-TGT CTG GAC CCC AAG GAA-3' | 22 |
| | Primer-R | 5'-CAT CTT CAC TGA TTC TTG GAT ACC-3' | 23 |
| | Probe | 5'-56-FAM/ACT TCT CCA/ZEN/CAA CCC TCT GCA CC/3IABkFQ/-3' | 24 |
| FOXP3 | Primer-F | 5'-CTA CTT CAA GTT CCA CAA CAT GC-3' | 25 |
| | Primer-R | 5'-CCA GTG GTA GAT CTC ATT GAG TG-3' | 26 |
| | Probe | 5'-/56-FAM/CCT TTC ACC/ZEN/TAC GCC ACG CTC AT/3IABkFQ/-3' | 27 |
| VEGFA | Primer-F | 5'-GTA CAA GAT CCG CAG ACG TG-3' | 28 |
| | Primer-R | 5'-TTC TGT ATC AGT CTT TCC TGG TG-3' | 29 |
| | Probe | 5'-56-FAM/TCG TTT AAC/ZEN/TCA AGC TGC CTC GCC/3IABkFQ/-3' | 30 |

(continued)

| Marker | | Sequence | SEQ ID NO |
|---|---|---|---|
| CCL2 | Primer-F | 5'-AGC AGC CAC CTT CAT TCC-3' | 31 |
| | Primer-R | 5'-GCC TCT GCA CTG AGA TCT TC-3' | 32 |
| | Probe | 5'-/56-FAM/TCA ATG CCC/ZEN/CAG TCA CCT GCT /3IABkFQ/-3' | 33 |
| CCL5 | Primer-F | 5'-GCT GTC ATC CTC ATT GCT ACT-3' | 34 |
| | Primer-R | 5'-TGC CAC TGG TGT AGA AAT ACT C-3' | 35 |
| | Probe | 5'-/56-FAM/ATC TGC CTC/ZEN/CCC ATA TTC CTC GGA/3IABkFQ/-3' | 36 |
| CCR5 | Primer-F | 5'-GTC TAT ATG ATT GAT TTG CAC AGC TC-3' | 37 |
| | Primer-R | 5'-TCA TAG ATT GGA CTT GAC ACT TGA-3' | 38 |
| | Probe | 5'-/56-FAM/CGT TCC CCT/ZEN/ACA AGA AAC TCT CCC C/3IABkFQ/-3' | 39 |
| CXCR4 | Primer-F | 5'-AGC AGG TAG CAA AGT GAC G-3' | 40 |
| | Primer-R | 5'-CCT CGG TGT AGT TAT CTG AAG TG-3' | 41 |
| | Probe | 5'-/56-FAM/AGA TGC GGT/ZEN/GGC TAC TGG AGC/3IABkFQ/-3' | 42 |
| ARG1 | Primer-F | 5'-ACT CCA CTG ACA ACC ACA AG-3' | 43 |
| | Primer-R | 5'-TGG CAG ATA TAC AGG GAG TCA-3' | 44 |
| | Probe | 5'-/56-FAM/CCT TCA GGA/ZEN/GGA AAG ATA CAG GTT GTC CA/3IABkFQ/-3' | 45 |
| CXCR2 | Primer-F | 5'-TCT GAC TAC CAC CCAACC T-3' | 46 |
| | Primer-R | 5'-CTG GGC TTT TCA CCT GTA GG-3' | 47 |
| | Probe | 5'-/56-FAM/CCA CTC CAA/ZEN/TAA CAG CAG GTC ACA GC/3IABkFQ/-3' | 48 |
| FATP2 (SLC27A 2) | Primer-F | 5'-CTG TCA TTC AGT ATA TCG GTG AAC T-3' | 49 |
| | Primer-R | 5'-ATC TCC TCG TAA GCC ATT TCC-3' | 50 |
| | Probe | 5'-/56-FAM/TGC AAC TCA/ZEN/CCA CAG AAA CCA AAT GAC/3IABkFQ/-3' | 51 |
| COX2 (PTGS2) | Primer-F | 5'-GCA CTA CAT ACT TAC CCA CTT CA-3' | 52 |
| | Primer-R | 5'-GCC ATA GTC AGC ATT GTA AGT TG-3' | 53 |
| | Probe | 5'-/56-FAM/ACA TCC AGA/ZEN/TCA CAT TTG ATT GAC AGT CCA/3IABkFQ/-3' | 54 |

(continued)

| Marker | Sequence | | | SEQ ID NO |
|---|---|---|---|---|
| PTGES2 | Primer-F | 5'-GCA ACA ACT AAA TGA CTC CTC TG-3' | | 55 |
| | Primer-R | 5'-CTG GGT AGT AGG TGA TGA TCT CT-3' | | 56 |
| | Probe | 5'-/56-FAM/CCC GAC ACC/ZEN/AGG TAG GTC TTG AG/3IABkFQ/-3' | | 57 |
| PTGES (PTGES1 ) | Primer-F | 5'-GTG GAA CGC TGC CTC AG-3' | | 58 |
| | Primer-R | 5'-CCA GAA AGG AGT AGA CGA AGC-3' | | 59 |
| | Probe | 5'-/56-FAM/AGG AAG GGG/ZEN/TAG ATG GTC TCC ATG T/3IABkFQ/-3' | | 60 |
| GAPDH (Referenc e gene) | Primer-F | 5'-ACATCGCTCAGACACCATG-3' | | 61 |
| | Primer-R | 5'-TGTAGTTGAGGTCAATGAAGGG-3' | | 62 |
| | Probe | 5'-HEX/AAG GTC GGA/ZEN/GTC AAC GGA TTT GGT C/IBFQ-3' | | 63 |
| (In Table 4, the probe structure includes a 5' FAM dye, an internal ZEN Quencher, and a *3' Iowa Black® Fluorescent Quencher (IBFQ)). | | | | |

For qPCR analysis, data from samples with GAPDH Ct values above 26.5 were excluded. All statistical analyses, except for ANOVA using $\Delta\Delta$Ct values, were conducted using $\Delta$Ct values. For evaluating statistical significance between pancreatic cancer patients and healthy individuals for each marker, a Kruskal-Wallis test was conducted using GraphPad PRISM9 statistical software. For AUC, sensitivity, and specificity analysis based on marker combinations, logistic regression and ROC curve analysis were performed using MedCalc statistical software.

EXAMPLE 3: Diagnosis of Pancreatic Cancer Using Individual Markers

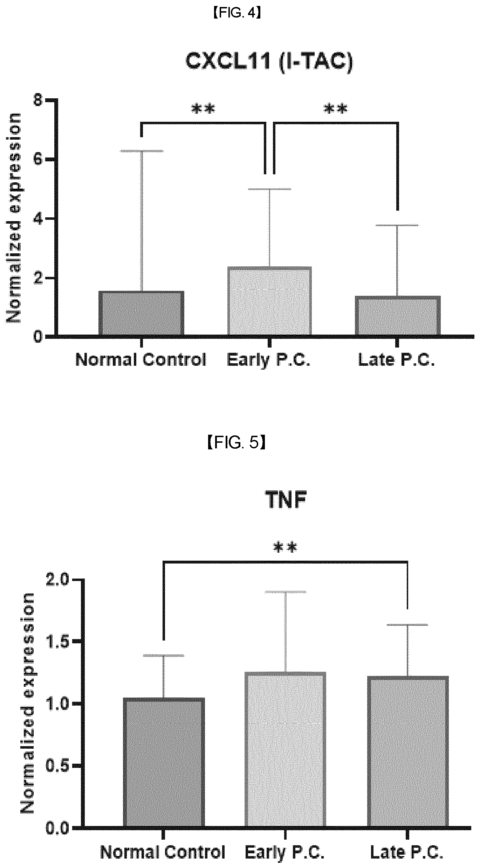

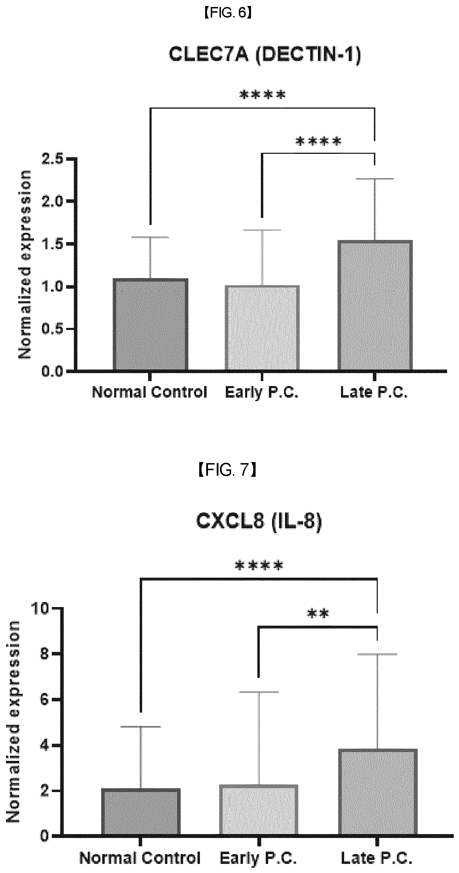

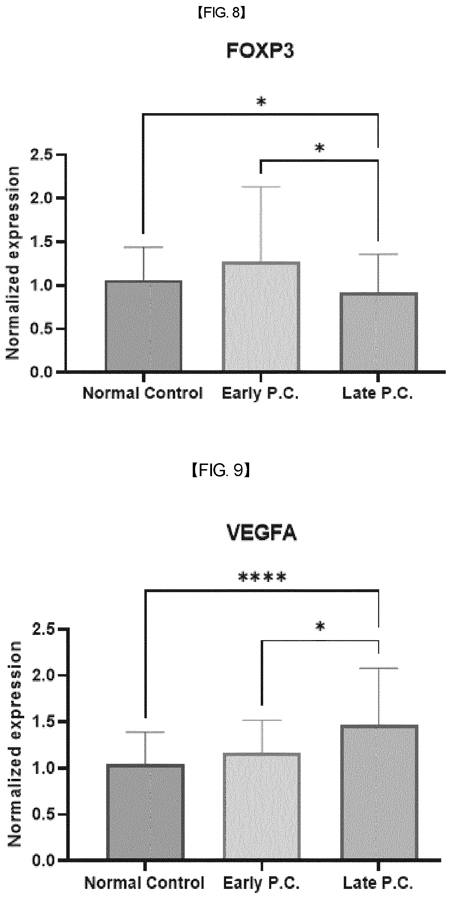

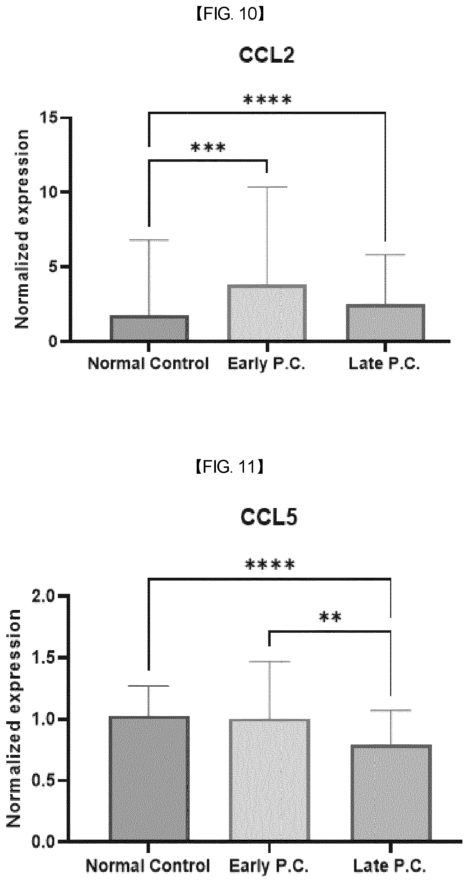

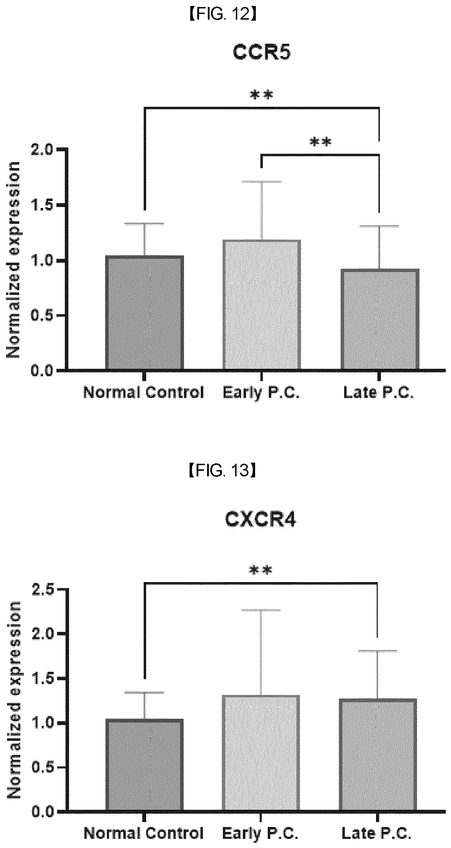

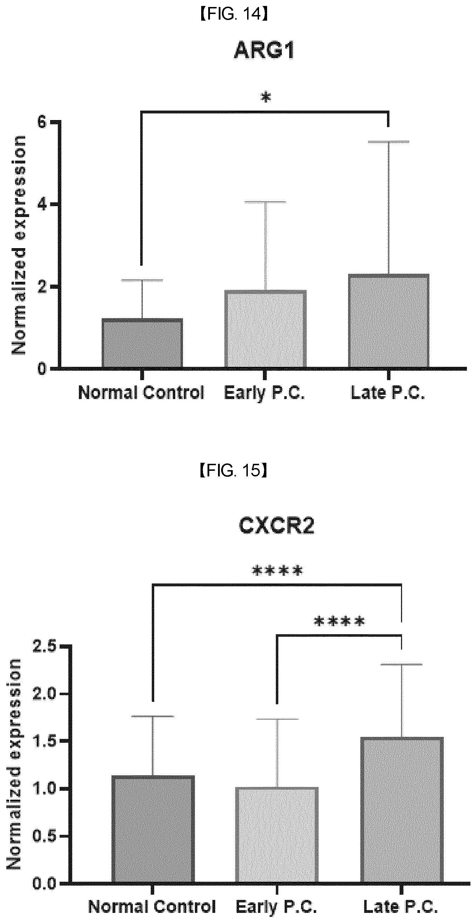

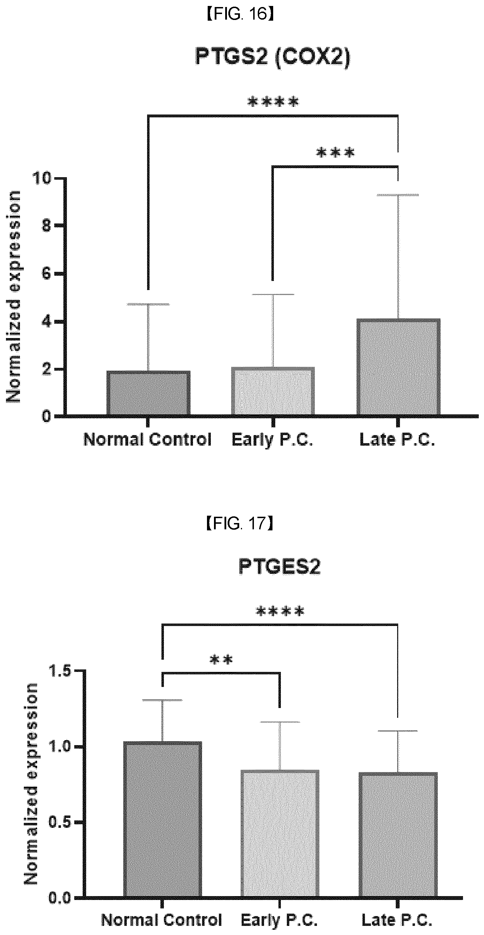

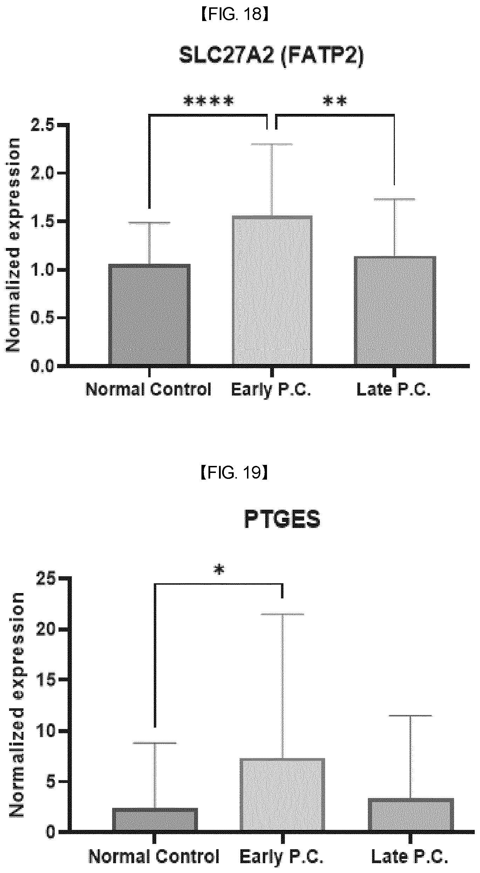

For 105 pancreatic cancer patients (36 early-stage pancreatic cancer patients and 69 late-stage pancreatic cancer patients) and 183 healthy individuals, buffy coat samples were obtained using the method described in Example 1. Using the method described in Example 2, Real-time qPCR was performed for each marker listed in Table 3, and the results ($\Delta\Delta$Ct values) are presented in FIGS. 2 to 19 and summarized in Table 5 below. (ANOVA analysis; NC: normal control (healthy group), PC: pancreatic cancer patient group, Early PC (E. PC): early-stage pancreatic cancer patient group, Late PC (L. PC): late-stage pancreatic cancer patient group):

TABLE 5

| Marker Name | Ref. Sequence (gene) | Ref. Sequence (protein) | Marker expression pattern in PC (relative to NC) | FIG. |
|---|---|---|---|---|
| IL-29(IFNL1) | NM_172140.1 | NP_742152.1 | Early: increased; Late: increased | 2 |
| IFNG | NM_000619.3 | NP_000610.2 | Early: decreased; Late: decreased | 3 |
| I-TAC(CXCL11 ) | NM_005409.5 | NP_005400.1 | Early: increased; Late: decreased | 4 |
| TNF | NM_000594.4 | NP_000585.2 | Early: increased; Late: increased | 5 |
| DECTIN1(CL EC7A) | NM_022570.5 | NP_072092.2 | Early: decreased; Late: increased | 6 |
| IL8(CXCL8) | NM_000584.4 | NP_000575.1 | Early: increased; Late: increased | 7 |
| FOXP3 | NM_014009.4 | NP_054728.2 | Early: increased; Late: decreased | 8 |
| VEGFA | NM_003376.6 | NP_003367.4 | Early: increased; Late: increased | 9 |
| CCL2 | NM_002982.4 | NP_002973.1 | Early: increased; Late: increased | 10 |

(continued)

| Marker Name | Ref. Sequence (gene) | Ref. Sequence (protein) | Marker expression pattern in PC (relative to NC) | FIG. |
|---|---|---|---|---|
| CCL5 | NM_002985.3 | NP_002976.2 | Early: decreased; Late: decreased | 11 |
| CCR5 | NM_000579.4 | NP_000570.1 | Early: increased; Late: decreased | 12 |
| CXCR4 | NM_003467.3 | NP_003458.1 | Early: increased; Late: increased | 13 |
| ARG1 | NM_001244438. 2 | NP_001231367. 1 | Early: increased; Late: increased | 14 |
| CXCR2 | NM_001557.4 | NP_001548.1 | Early: decreased; Late: increased | 15 |
| COX2(PTGS2 ) | NM_000963.4 | NP_000954.1 | Early: increased Late: increased | 16 |
| PTGES2 | NM_198938.3 | NP_945176.1 | Early: decreased; Late: decreased | 17 |
| FATP2 (SLC27A2) | NM_003645.4 | NP_003636.2 | Early: increased Late: increased | 18 |
| PTGES | NM_004878.5 | NP_004869.1 | Early: increased Late: increased | 19 |

As confirmed in Table 5, all tested markers exhibited differential expression patterns (increase and/or decrease) in pancreatic cancer patients (both early-stage and late-stage) compared to the normal group. Notably, at least one of the early-stage or late-stage pancreatic cancer groups showed a statistically significant difference in expression patterns compared to the normal group.

EXAMPLE 4: Differentiation of Pancreatic Cancer from Similar Diseases Using Markers IFNA1 and IFNB1

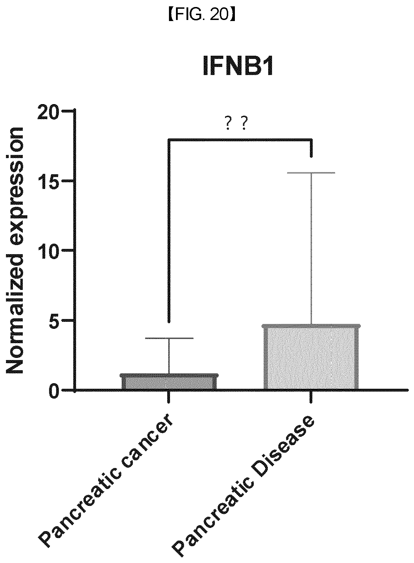

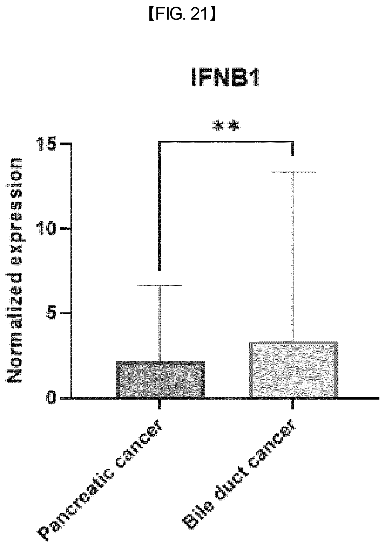

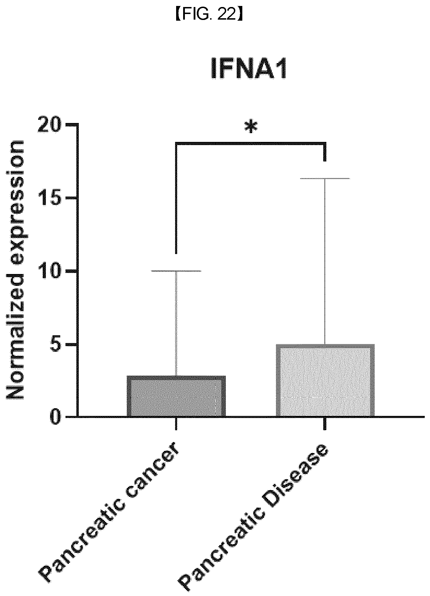

Using the method described in Example 1, buffy coat samples were obtained from 105 pancreatic cancer patients, 17 patients with benign pancreatic diseases (one patient with pancreatitis, 11 patients with benign pancreatic tumors, one patient with intraductal papillary mucinous neoplasm (IPMN), two patients with autoimmune pancreatitis (AIP), two 2 patients with other pancreatic diseases), and 40 patients with cholangiocarcinoma. Using the method described in Example 2, Real-time qPCR was performed for IFNA1 and IFNB1 markers, and the results ($\Delta\Delta$Ct values) are presented in FIGS. 20 and 21 (IFNB1) and FIG. 22 (IFNA1) (* $p < 0.05$, p-value: Non-parametric t-test analysis).

As shown in FIGS. 20 to 22, the IFNA1 and IFNB1 markers exhibited a statistically significant decrease in expression in pancreatic cancer patients (both early-stage and late-stage) compared to patients with benign pancreatic diseases and/or cholangiocarcinoma, which are similar diseases.

Based on the results from Examples 3 and 4, it was confirmed that each of the 20 markers listed in Table 3 can differentiate pancreatic cancer patients (early-stage and/or late-stage) from healthy individuals or patients with similar diseases (benign pancreatic diseases and/or cholangiocarcinoma).

EXAMPLE 5: Analysis for Relative Importance of Markers

Based on the previously measured $\Delta$Ct values, the relative importance of the 20 pancreatic cancer markers listed in Table 3 was verified through logistic regression analysis and feature selection analysis to assess statistical significance.

5.1 Logistic Regression Analysis

When two or more markers were combined for analysis, the impact of each marker on diagnostic performance within comparison groups was assessed. A logistic regression analysis (MedCalc software Ltd) was conducted to determine the Wald value of each marker when the markers were used in combination. Combinations of 20 markers were analyzed in various comparison groups (healthy group vs. early-stage pancreatic cancer patients, healthy group vs. late-stage pancreatic cancer patients, healthy group vs. all pancreatic cancer patients), and markers with a Wald value of ≥1 in each comparison group were selected.

The results obtained from this analysis are presented in Tables 6 to 8.

TABLE 6

| Normal vs. Early PC (RPC) | |
|---|---|
| Variable (marker) | Wald |
| SLC27A2 | 10.1892 |
| IFNG | 9.2174 |
| CCL2 | 5.7586 |
| TNF | 5.6291 |
| PTGES | 5.0075 |
| CLEC7A | 4.4323 |
| ARG1 | 4.347 |
| PTGES2 | 3.4967 |
| FOXP3 | 3.2434 |
| CXCR4 | 2.056 |
| VEGFA | 1.1967 |
| CXCR2 | 1.1056 |
| PTGS2 | 1.0355 |

TABLE 7

| Normal vs. Late PC (BRPC, LAPC, MPC) | |
|---|---|
| Variable (marker) | Wald |
| VEGFA | 16.3983 |
| CXCR4 | 13.3142 |
| SLC27A2 | 12.7877 |
| TNF | 10.231 |
| PTGES2 | 9.8378 |
| CCL5 | 7.2006 |
| CXCL11 | 4.5562 |
| CXCR2 | 3.4444 |
| FOXP3 | 1.6131 |
| PTGS2 | 1.4582 |
| CXCL8 | 1.4025 |

TABLE 8

| Normal vs. All PC | |
|---|---|
| Variable (marker) | Wald |
| SLC27A2 | 21.2921 |
| PTGES2 | 20.4726 |
| VEGFA | 16.394 |
| TNF | 14.0026 |
| CXCR4 | 13.7166 |
| IFNG | 5.0016 |
| CCL5 | 3.4707 |

(continued)

| Normal vs. All PC | |
|---|---|
| Variable (marker) | Wald |
| CXCR2 | 2.1417 |
| CCL2 | 1.8652 |
| CXCL11 | 1.7596 |
| CLEC7A | 1.1977 |
| ARG1 | 0.7049 |

As shown in Tables 6-8, the larger the Wald value, the greater the marker's influence on diagnostic performance.

5.2 Feature Selection Analysis

Feature selection was performed to determine the importance of each marker among the 20 markers in different comparison groups (healthy group vs. early-stage pancreatic cancer patients, healthy group vs. late-stage pancreatic cancer patients, and healthy group vs. all pancreatic cancer patients). Boruta package Miron B. Kursa, Witold R. Rudnicki (2010). Feature Selection with the Boruta Package. Journal of Statistical Software, 36(11), p. 1-13.) in R software was used for the analysis. With each marker considered as an individual feature (variable), the maximum importance value of the shadow feature was used as the threshold. Features (markers) with lower importance than the shadow feature's maximum importance were removed.

The results obtained from this analysis are presented in Tables 9 to 11.

TABLE 9

| Normal vs. Early PC (RPC) | |
|---|---|
| Variable (marker) | MeanImp |
| IFNG | 15.1389 |
| SLC27A2 | 9.1957 |
| CCL5 | 9.1607 |
| FOXP3 | 7.0243 |
| PTGES2 | 6.5731 |
| TNF | 6.4424 |
| IFNA1 | 4.6783 |
| IFNL1 | 3.9418 |
| CCL2 | 3.8931 |
| CXCR2 | 3.2156 |
| PTGES | 2.8860 |
| CXCR4 | 2.8348 |
| IFNB1 | 2.5217 |

TABLE 10

| Normal vs. Late PC (BRPC, LAPC, MPC) | |
|---|---|
| Variable (marker) | MeanImp |
| PTGES2 | 10.4895 |
| IFNG | 10.1975 |
| CCL5 | 9.5050 |

(continued)

| Normal vs. Late PC (BRPC, LAPC, MPC) | |
| --- | --- |
| Variable (marker) | MeanImp |
| VEGFA | 8.3364 |
| PTGS2 | 4.4072 |
| CLEC7A | 4.0338 |
| CXCR4 | 3.9958 |
| TNF | 3.8240 |
| FOXP3 | 3.4201 |
| CCL2 | 3.0325 |
| CXCR2 | 3.0066 |
| CXCL8 | 2.9039 |
| PTGES | 2.4986 |
| IFNL1 | 2.2232 |
| SLC27A2 | 2.2184 |

TABLE 11

| Normal vs. All PC | |
| --- | --- |
| Variable (marker) | MeanImp |
| IFNG | 15.1805 |
| PTGES2 | 11.1079 |
| CCL5 | 10.7329 |
| SLC27A2 | 8.5693 |
| VEGFA | 7.8181 |
| CCL2 | 6.8393 |
| TNF | 5.5759 |
| FOXP3 | 5.2303 |
| CXCR4 | 4.9659 |
| IFNA1 | 4.3472 |
| IFNL1 | 4.3132 |
| PTGES | 3.6010 |
| CCR5 | 3.0130 |
| CLEC7A | 2.6242 |
| IFNB1 | 2.5782 |
| CXCR2 | 2.5765 |
| PTGS2 | 2.5699 |

As shown in Tables 9 to 11, the meanImp value quantifies the importance of each marker in different comparison groups. Markers with an importance value higher than the maximum shadow feature importance were selected. The higher the value, the greater the marker's influence on pancreatic cancer diagnosis.

EXAMPLE 6: Diagnosis of Pancreatic Cancer Using Marker Combinations

From the markers listed in Table 3, marker combinations with high statistical significance, as determined in Example 5.1, were selected for ROC curve analysis.

Using the method in Example 2, real-time qPCR ($\Delta$Ct values) was obtained for markers with high significance (listed in Tables 6-8) in the buffy coat samples obtained from pancreatic cancer patients (both early-stage pancreatic patients and late-stage pancreatic patients) and healthy individuals as described in Example 1. Statistical analysis was performed on the combined results.

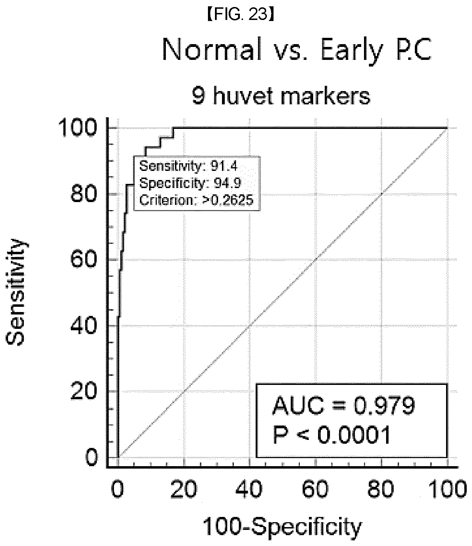

With logistic regression applied as the classifier, the analysis results were presented in Receiver Operating Characteristic (ROC) curves (FIGS. 23 to 30). From the ROC curve, the optimal sensitivity, specificity, and cut-off value for pancreatic cancer diagnosis were identified.

$$\text{Sensitivity (\%)} = (TP / (TP + FN)) * 100$$

$$\text{Specificity (\%)} = (TN / (TN + FP)) * 100$$

$$\text{Youden's Index (J)} = \max (\text{Sensitivity} + \text{Specificity} - 1)$$

AUC (Area Under the ROC Curve)
[TP (True Positive): Number of actual pancreatic cancer patients correctly diagnosed as positive
FN (False Negative): Number of actual pancreatic cancer patients incorrectly diagnosed as negative
TN (True Negative): Number of actual healthy individuals correctly diagnosed as negative
FP (False Positive): Number of actual healthy individuals incorrectly diagnosed as positive]

The ROC curve analysis results for a combination of nine biomarkers that demonstrated excellent discrimination ability between normal and early pancreatic cancer, as identified in Table 6 (9 Huvet markers (Early P.C.): ARG1, CCL2, CLEC7A, SLC27A2, IFNG, PTGES, PTGES2, TNF, and FOXP3), are shown in FIG. 23 and Table 12 (Normal group: 177 subjects; Early pancreatic cancer group: 35 subjects).

TABLE 12

| Sample comparison | Normal (healthy) vs. Early P.C (early-stage pancreatic cancer) |
|---|---|
| Sample size | 212 cases (Normal: 177 cases, Early P.C: 35 cases) |
| Classifier | Logistic Regression |
| Youden index | 0.8634 |
| AUC | 0.979 |
| Sensitivity (%) | 91.43 |
| Specificity (%) | 94.92 |
| No. of marker | 9 |
| Type of marker | ARG1, CCL2, CLEC7A, SLC27A2, IFNG, PTGES, PTGES2, TNF, FOXP3 |

As presented in FIG. 23 and Table 12, when using the nine-biomarker combination (ARG1, CCL2, CLEC7A, SLC27A2, IFNG, PTGES, PTGES2, TNF, and FOXP3), the AUC value for early pancreatic cancer (compared to the normal group) was close to 1 (0.979). Additionally, both sensitivity and specificity exceeded 90%, confirming that the biomarker combination can accurately and effectively distinguish between normal subjects and early pancreatic cancer patients.

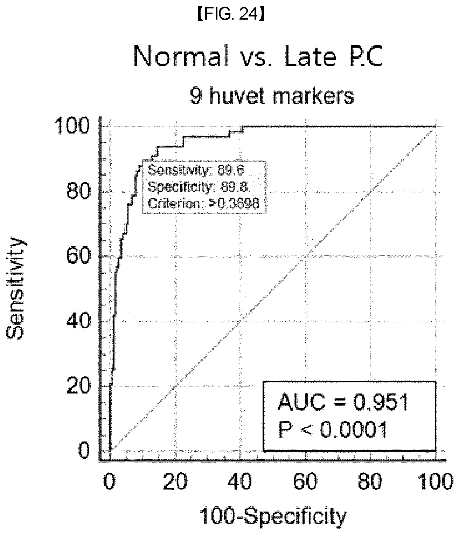

Furthermore, the ROC curve analysis results for a combination of nine biomarkers that demonstrated excellent discrimination ability between normal and late pancreatic cancer, as identified in Table 7 (9 Huvet markers (Late P.C.): CCL5, PTGS2, CXCR2, CXCR4, SLC27A2, CXCL11, PTGES2, TNF, and VEGFA), are shown in FIG. 24 and Table 13 (Normal group: 177 subjects; Late pancreatic cancer group: 67 subjects).

TABLE 13

| Sample comparison | Normal vs. Late P.C (late-stage pancreatic cancer) |
|---|---|
| Sample size | 244 cases (Normal: 177 cases, Late P.C: 67 cases) |
| Classifier | Logistic Regression |
| Youden index | 0.7938 |
| AUC | 0.951 |
| Sensitivity (%) | 89.55 |
| Specificity (%) | 89.83 |
| No. of marker | 9 |
| Type of marker | CCL5, PTGS2, CXCR2, CXCR4, SLC27A2, CXCL11, PTGES2, TNF, VEGFA |

As presented in FIG. 24 and Table 13, when using this nine-biomarker combination (CCL5, PTGS2, CXCR2, CXCR4, SLC27A2, CXCL11, PTGES2, TNF, and VEGFA), the AUC value for late pancreatic cancer (compared to the normal group) was close to 1 (0.951). Additionally, both sensitivity and specificity were approximately 90%, confirming that this biomarker combination can accurately and effectively distinguish between normal subjects and late pancreatic cancer patients.

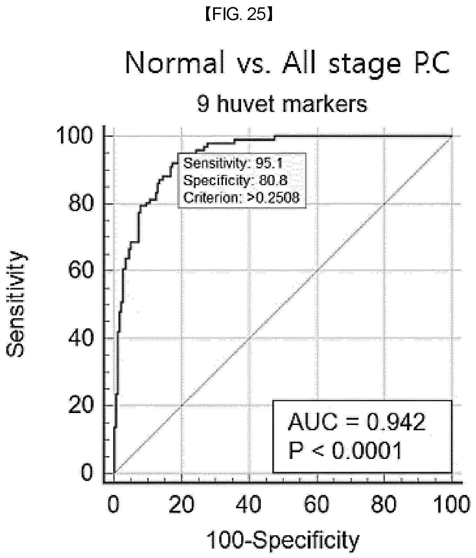

Moreover, the ROC curve analysis results for a combination of nine biomarkers that demonstrated excellent discrimination ability between normal and all pancreatic cancer patients (both early and late stages), as identified in Table 8 (9 Huvet markers (All P.C.): ARG1, CCL2, SLC27A2, IFNG, PTGES2, TNF, VEGFA, CXCR4, and CCL5), are shown in FIG. 25 and Table 14 (Normal group: 177 subjects; Pancreatic cancer group: 102 subjects, including 35 early pancreatic cancer patients and 67 late pancreatic cancer patients).

TABLE 14

| Sample comparison | Normal vs. All P.C (early- and late-stage pancreatic cancer) |
|---|---|
| Sample size | 279 cases (Normal: 177 cases, All P.C: 102 cases) |
| Classifier | Logistic Regression |
| Youden index | 0.7589 |
| AUC | 0.942 |
| Sensitivity (%) | 95.10 |
| Specificity (%) | 80.79 |
| No. of marker | 9 |
| Type of marker | ARG1, CCL2, SLC27A2, IFNG, PTGES2, TNF, VEGFA, CXCR4, CCL5 |

The results shown in FIG. 25 and Table 14 indicate that when using the nine-marker combination (ARG1, CCL2, SLC27A2, IFNG, PTGES2, TNF, VEGFA, CXCR4, and CCL5), the AUC value for all-stage pancreatic cancer (compared to the normal group) was close to 1 (0.942). The sensitivity was approximately 95%, and the specificity was around 81%, confirming that this nine-marker combination can accurately and effectively distinguish pancreatic cancer patients from normal individuals.

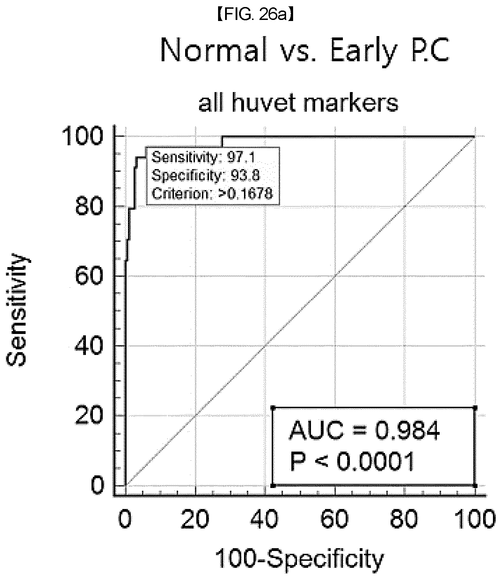

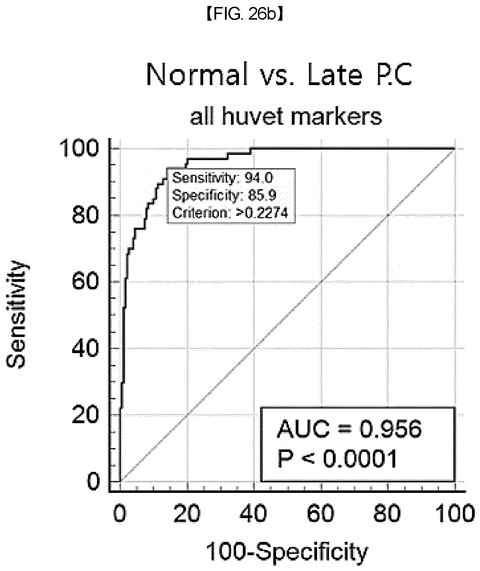

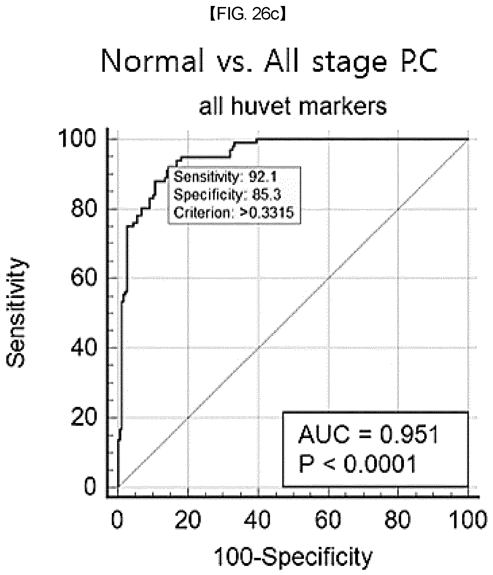

Additionally, using the combination of all 20 markers listed in Table 3 (All Huvet markers: ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA), a Logistic Regression analysis was conducted on samples from normal individuals (177) and pancreatic cancer patients (101; early-stage: 34, late-stage: 67). The ROC curve analysis results are shown in FIGS. 26a (normal vs. early pancreatic cancer), 26b (normal vs. late pancreatic cancer), and 26c (normal vs. all pancreatic cancers).

As shown in FIGS. 26a to 26c, when using the combination of 20 biomarkers (ARG1, CCL2, CCL5, CCR5, PTGS2, CXCR2, CXCR4, CLEC7A, SLC27A2, FOXP3, CXCL11, IFNG, IFNA1, IFNB1, IFNL1, CXCL8, PTGES, PTGES2, TNF, and VEGFA), the AUC value was close to 1 (0.95 or higher) for all patient groups, including early pancreatic cancer, late pancreatic cancer, and all-stage pancreatic cancer compared to the normal group. Additionally, sensitivity exceeded 92% and specificity exceeded 85%, confirming that this 20-biomarker combination can accurately and effectively differentiate between normal individuals and pancreatic cancer patients (early and/or late-stage pancreatic cancer).

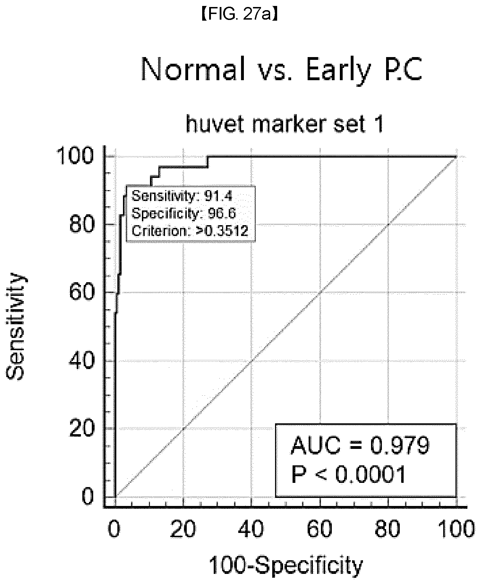

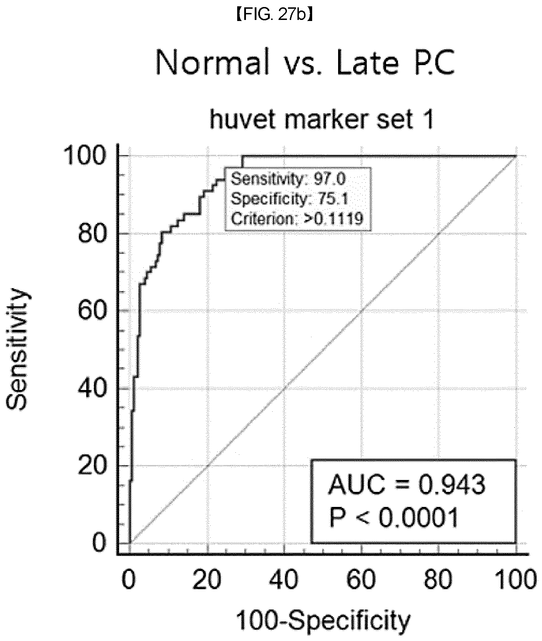

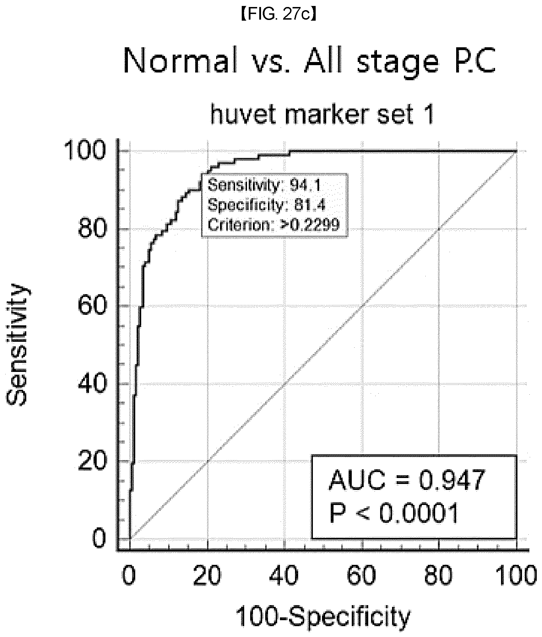

Furthermore, among the biomarkers listed in Table 3, a 12-biomarker subset (Marker Set 1) was selected by integrating nine biomarkers from Table 12 (Normal vs. Early Pancreatic Cancer) and nine biomarkers from Table 14 (Normal vs. All Pancreatic Cancer), with six overlapping markers. The selected biomarkers (CCL5, CXCR4, SLC27A2, PTGES2, TNF, VEGFA, ARG1, CCL2, CLEC7A, IFNG, PTGES, and FOXP3) were used for Logistic Regression analysis of samples from 177 normal subjects and 102 pancreatic cancer patients (35 early pancreatic cancer patients and 67 late pancreatic cancer patients), and the resulting ROC curve analysis is presented in FIGS. 27a (Normal vs. Early Pancreatic Cancer), 27b (Normal vs. Late Pancreatic Cancer), and 27c (Normal vs. All Pancreatic Cancer).

As shown in FIGS. 27a to 27c, when using the 12-biomarker combination (CCL5, CXCR4, SLC27A2, PTGES2, TNF, VEGFA, ARG1, CCL2, CLEC7A, IFNG, PTGES, and FOXP3), the AUC value exceeded 0.94 for all patient groups, including early pancreatic cancer, late pancreatic cancer, and all-stage pancreatic cancer compared to the normal group. Additionally, sensitivity exceeded 91% and specificity exceeded 75%, confirming that this 12-biomarker combination can accurately and effectively distinguish normal individuals from pancreatic cancer patients (early and/or late-stage pancreatic cancer).

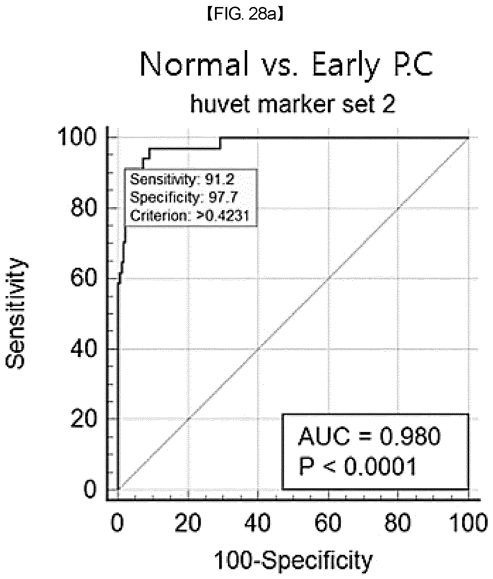

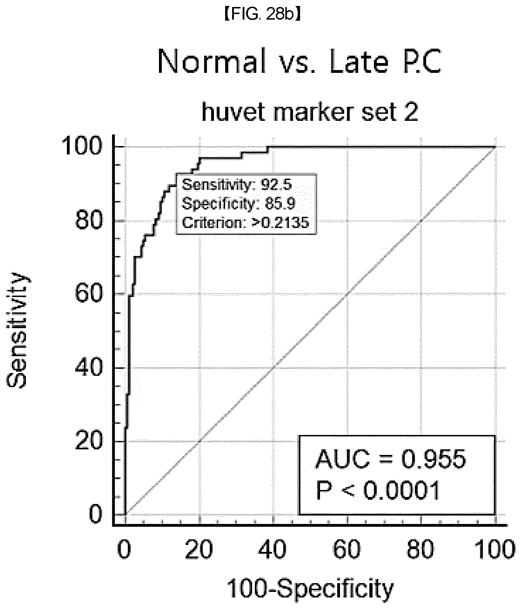

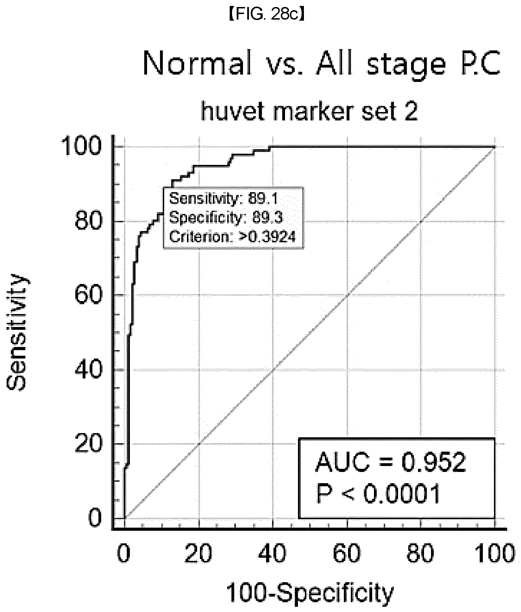

Furthermore, among the biomarkers listed in Table 3, a 15-biomarker subset (Marker Set 2) was selected by integrating nine biomarkers from Table 12 (Normal vs. Early Pancreatic Cancer) and nine biomarkers from Table 13 (Normal vs. Late Pancreatic Cancer), with three overlapping markers. The selected biomarkers (CCL5, CXCR4, SLC27A2, PTGES2, TNF, VEGFA, ARG1, CCL2, CLEC7A, IFNG, PTGES, FOXP3, PTGS2, CXCR2, and CXCL11) were used for Logistic Regression analysis of samples from 177 normal subjects and 102 pancreatic cancer patients (35 early pancreatic cancer patients and 67 late pancreatic cancer patients), and the resulting ROC curve analysis is presented in FIGS. 28a (Normal vs. Early Pancreatic Cancer), 28b (Normal vs. Late Pancreatic Cancer), and 28c (Normal vs. All Pancreatic Cancer).

As shown in FIGS. 28a to 28c, when using the 15-biomarker combination (CCL5, CXCR4, SLC27A2, PTGES2, TNF, VEGFA, ARG1, CCL2, CLEC7A, IFNG, PTGES, FOXP3, PTGS2, CXCR2, and CXCL11), the AUC value exceeded 0.95 for all patient groups, including early pancreatic cancer, late pancreatic cancer, and all-stage pancreatic cancer compared to the normal group. Additionally, sensitivity exceeded 89% and specificity exceeded 85%, confirming that this 15-biomarker combination can accurately and effectively differentiate normal individuals from pancreatic cancer patients (early and/or late-stage pancreatic cancer).

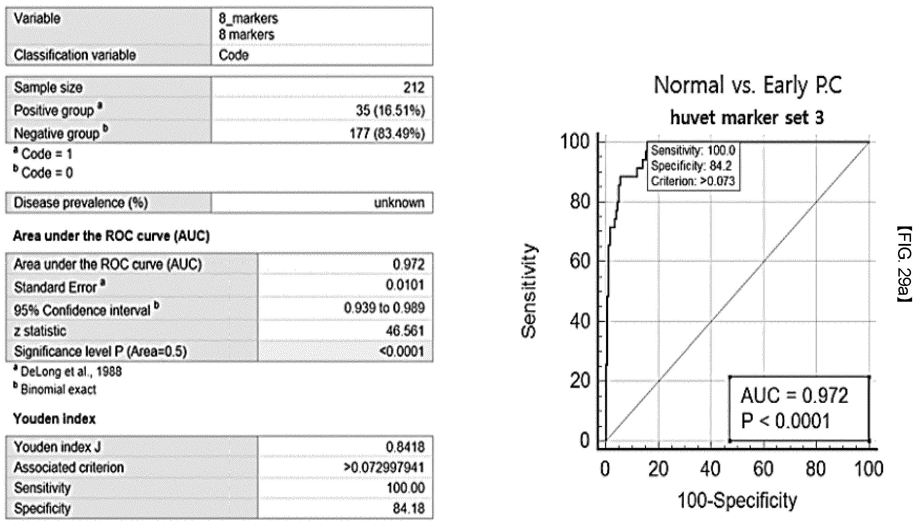

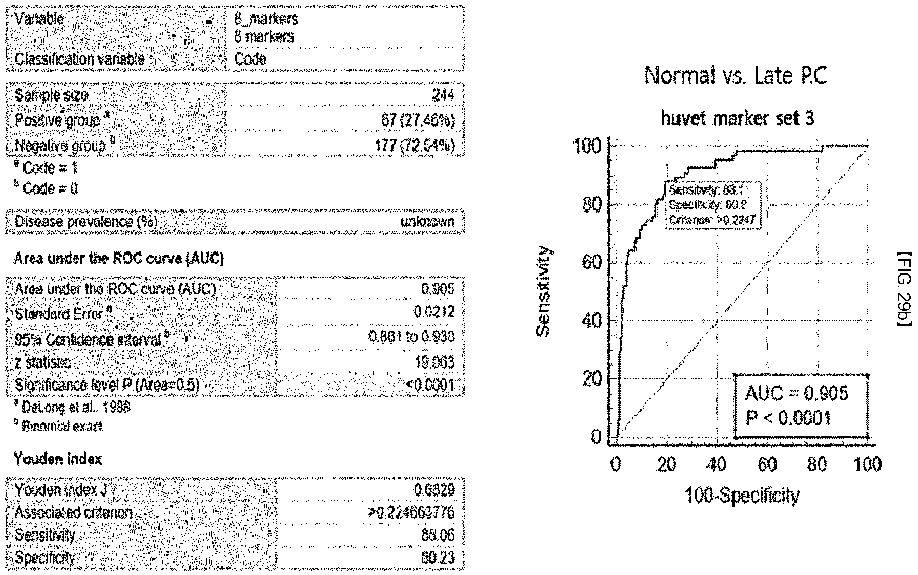

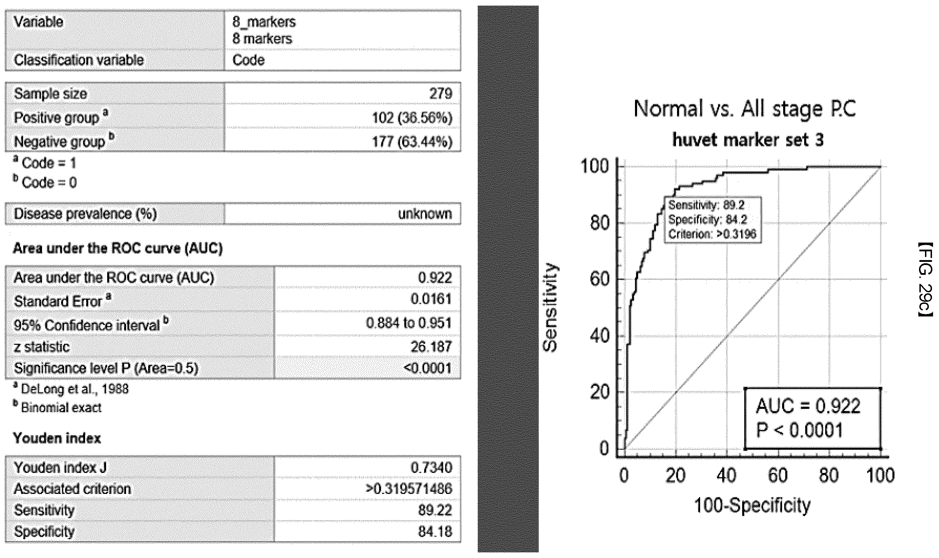

Furthermore, among the biomarkers listed in Table 3, a subset of eight biomarkers (Marker Set 3: CCL2, CCL5, CXCR2, SLC27A2, IFNL1, IFNG, PTGES2, and TNF) was selected. Using these biomarkers, Logistic Regression analysis was performed on samples from 177 normal subjects and 102 pancreatic cancer patients (35 early pancreatic cancer patients and 67 late pancreatic cancer patients), and the results of the ROC curve analysis are presented in FIGS. 29a (Normal vs. Early Pancreatic Cancer), 29b (Normal vs. Late Pancreatic Cancer), and 29c (Normal vs. All Pancreatic Cancer).

As shown in FIGS. 29a to 29c, when using the eight-biomarker combination (CCL2, CCL5, CXCR2, SLC27A2, IFNL1, IFNG, PTGES2, and TNF), the AUC value exceeded 0.9 across all patient groups, including early pancreatic cancer, late pancreatic cancer, and all-stage pancreatic cancer, compared to the normal group. Additionally, sensitivity exceeded 88%, and specificity exceeded 80%, confirming that this eight-biomarker combination can accurately and effectively distinguish normal individuals from pancreatic cancer patients (early and/or late-stage pancreatic cancer).

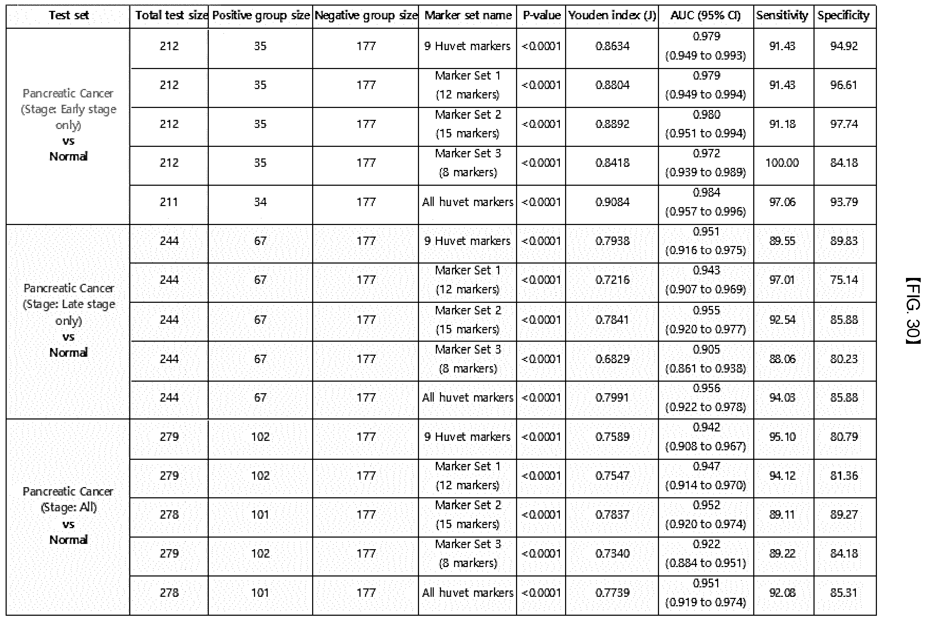

The results from FIGS. 23 to 29c are summarized in FIG. 30.

EXAMPLE 7: Development of Predictive Model for Pancreatic Cancer Diagnosis

To estimate the predictive model, stepwise logistic regression was performed for variable (marker) selection, and statistical analysis was conducted using SAS ver 9.4 (SAS Institute Inc., NC, Cary, USA). To balance underfitting and overfitting, 10-fold cross-validation was used for model validation and evaluation. The split ratios for the test set and training set were set to 90:10, 80:20, 75:25, and 70:30. For each iteration, diagnostic accuracy metrics were calculated, and the optimal performance point was identified using the maximum Youden's index in the ROC curve analysis to evaluate the model.

The odds ratio represents the likelihood of pancreatic cancer compared to the likelihood of not having pancreatic cancer. Assigning 1 for pancreatic cancer cases and 0 for non-cancer cases, the odds ratio was calculated using the following equation:

$$logit(p) = \ln\left(\frac{p}{1-p}\right)$$

$$logit(p) = b_0 + b_1 X_1 + b_2 X_2 + b_3 X_3 + \ldots + b_k X_k$$

Through optimal predictive model analysis, the $b_0$, b1, $b_2$, ..., $b_k$ regression coefficients were estimated for marker combinations. Taking the logarithm of both sides of the odds equation, the final predictive model equation was derived as the above equation: where X1, X2, X3, ..., Xk represent the actual measured values of each marker obtained from the sample using Real-time qPCR ($\Delta$Ct values), as described in Example 2.

In the comparison between 183 normal individuals and 36 early pancreatic cancer patients, the optimal diagnostic models for early pancreatic cancer were derived using an 8-biomarker combination (Model_R1) and a 13-biomarker combination (Model_R2). Additionally, in the comparison between 183 normal individuals and 106 total pancreatic cancer patients (including both early and late-stage pancreatic cancer patients), the optimal diagnostic models for total pancreatic cancer were derived using an 11-biomarker combination (Model_A1) and a 10-biomarker combination (Model_A2) (Table 15). By analyzing the biomarker expression levels in samples, the diagnostic prediction for pancreatic cancer can be performed by inputting the $\Delta$Ct values (measured using Real-time qPCR, as described in Example 2) into the model equations.

TABLE 15

| Model | Pancreati c cancer group | No. of Marker | Marker combination | Model equation logit(p) | Cut-off | AUC |
|---|---|---|---|---|---|---|
| Model _R1 | Early pan-creati c can-cer | 8 | TNF, IFNG, CCL2, CXCR2, ARG1, SLC27A2, PTGES2, PTGES | *logit(p)* =35.8476+(-2.5794)×TNF+(3.3956)×IFNG+(-0.5380)×-CCL2+(1.4027)×CXC-R2+(-1.143)×AR-G1+(-4.3771)×SLC27-A2+(3.1076)×PTGES 2+(-0.6396)×PTGES | > - 1.85599 4 | 0.9787 |
| Model _R2 | Early pan-creati c can-cer | 13 | TNF, IFNG, IFNL1, CXCL11, CLEC7A, VEGFA, CCL$^2$, CXCR4, CXCR2, ARG1, SLC27A2, PTGES2, PTGES | *logit(p)* =44.4033+(-2.8042)×TNF+(3.7082)×IFNG+(0.235 8)×IFNL1+(0.1568)×CXC-L11+(1.0951 )×CLE-C7A+(-0.8407)×VEG-FA+(-0.6622)×CC-L2+(-0.9422)×CXC-R4+(1.0804)×CXCR2+(--1.2971)×ARG1+(-4.7971)×SLC27A2+(3.1934)×PTGES 2+(-0.7064)×PTGES | > - 1.90875 | 0.9804 |
| Model _A1 | All pancreati c cancer | 11 | TNF, IFNG, CXCL11, VEGFA, CCL2, CCL5, CXCR4, CXCR2, PTGS2, SLC27A2, PTGES2 | *logit(p)* =36.9898+(-2.220)×TNF+(0.9955)×IFNG+(0.3413) ×CXCL11+(-2.799)×VEG-FA+(-0.1913)×CC-L2+(1.3628)×CC-L5+(-2.0521)×CXC-R4+(0.7037)×CXCR2+(--0.064)×PTGS2+(-3.1754)×SLC27A2+(4.4765)×PTGES 2 | > - 0.25109 | 0.9493 |

(continued)

| Model | Pancreatic cancer group | No. of Marker | Marker combination | Model equation logit(p) | Cut-off | AUC |
|---|---|---|---|---|---|---|
| Model_A2 | All pancreatic cancer | 10 | IFNG, VEG-FA, SLC27A2, PTGES2, TNF, CXCR4, CXCR2, CCL5, CCL2, CXCL11 | $logit(p)$=36.6273 +(1.0041)×IFNG +(-2.7473)×VEGFA +(-3.199)×SLC27A2 +(4.4958)×PTGES2 +(-2.2286)×TNF +(-2.0986)×CXCR4 +(0.6388)×CXCR2 +( 1.3874)×CCL5 +(-0.224)×CCL2 +(0.3704)×CXCL11 | > - 0.29033 | 0.9495 |

Analysis results obtained by applying the model equation *logit(p)* =35.8476+(-2.5794)×TNF+(3.3956)×IFNG+(-0.5380)×CCL2+(1.4027)×CXCR2+(-1.143)×ARG1+(-4.3771)×SLC27A2+(3.1076)×PTGES2+(-0.6396)×PTGES of Model_R1 to actual patient samples are as follows:

For sample HV18, the calculated value is '35.8476 + (-2.5794)×6.503 + (3.3956)×6.88 + (-0.5380)×10.72 + (1.4027)×0.535 + (-1.143)×7.035 + (-4.3771)×10.654 + (3.1076)×5.68 + (-0.6396)×10.994 = -6.637'. Since this value is lower than the cut-off value of -1.855994, sample HV18 is predicted to have a low risk of pancreatic cancer and is classified as normal. The actual sample HV18 belonged to the normal group.
For sample HV119, the calculated value is '35.8476 + (-2.5794)×6.304 + (3.3956)×9.499 + (-0.5380)×10.502 + (1.4027)×1.496 + (-1.143)×4.878 + (-4.3771)×9.708 + (3.1076)×4.963 + (-0.6396)×11.822 = 8.083'. Since this value is higher than the cut-off value of -1.855994, sample HV119 is predicted to have a high risk of pancreatic cancer. The actual sample HV119 belonged to the early pancreatic cancer group.

Analysis results obtained by applying Model_R2's equation: logit(p)= 44.4033 + (-2.8042)×TNF + (3.7082)×IFNG + (0.2358)×IFNL1 + (0.1568)×CXCL11 + (1.0951)×CLEC7A + (-0.8407)×VEGFA + (-0.6622)×CCL2 + (-0.9422)×CXCR4 + (1.0804)×CXCR2 + (-1.2971)×ARG1 + (-4.7971)×SLC27A2 + (3.1934)×PTGES2 + (-0.7064)×PTGES to actual patient samples are as follows:

For sample HV18, the calculated value is '44.4033 + (-2.8042)×6.5032 + (3.7082)×6.8802 + (0.2358)×9.4032 + (0.1568)×10.5360 + (1.0951)×1.4825 + (-0.8407)×7.7399 + (-0.6622)×10.7275 + (-0.9422)×2.9629 + (1.0804)×0.5352 + (-1.2971)×7.0352 + (-4.7971)×10.6540 + (3.1934)×5.6802 + (-0.7064)×10.9943 = - 8.512'. Since this value is lower than the cut-off value of -1.908747, sample HV18 is predicted to have a low risk of pancreatic cancer and is classified as normal. The actual sample HV18 belonged to the normal group.
For sample HV119, the calculated value is '44.4033 + (-2.8042)×6.3042 + (3.7082)×9.4998 + (0.2358)×7.8130 + (0.1568)×10.3085 + (1.0951)×1.1619 + (-0.8407)×7.0730 + (-0.6622)×10.5027 + (-0.9422)×0.0542 + (1.0804)×1.4966 + (-1.2971)×4.8782 + (-4.7971)×9.7083 + (3.1934)×4.9634 + (-0.7064)×11.8220 = 9.948'. Since this value is higher than the cut-off value of -1.908747, sample HV119 is predicted to have a high risk of pancreatic cancer. The actual sample HV119 belonged to the early pancreatic cancer group.

Analysis results obtained by applying Model_A1's equation logit(p)= 36.9898 + (-2.220)×TNF + (0.9955)×IFNG + (0.3413)×CXCL11 + (-2.799)×VEGFA + (-0.1913)×CCL2 + (1.3628)×CCL5 + (-2.0521)×CXCR4 + (0.7037)×CXCR2 + (-0.064)×PTGS2 + (-3.1754)×SLC27A2 + (4.4765)×PTGES2 to actual patient samples are as follows:

For sample HV18, the calculated value is '36.9898 + (-2.220)×6.5032- + (0.9955)×6.8802 + (0.3413)×10.5360 + (-2.799)×7.7399 + (-0.1913)×10.7275 + (1.3628)×1.0155 + (-2.0521)×2.9629 + (0.7037)×0.5352 + (-0.064)×3.9349 + (-3.1754)×10.6540 + (4.4765)×5.6802 = -3.693'. Since this value is lower than the cut-off value of -0.251091, sample HV18 is predicted to have a low risk of pancreatic cancer and is classified as normal. The actual sample HV18 belonged to the normal group.
For sample HV119, the calculated value is '36.9898 + (-2.220)×6.3042 + (0.9955)×9.4998 + (0.3413)×10.3085 + (-2.799)×7.0730 + (-0.1913)×10.5027 + (1.3628)×0.5032 + (-2.0521)×0.542 + (0.7037)×1.4966 + (-0.064)×5.5966 + (-3.1754)×9.7083 + (4.4765)×4.9634 = 6.824'. Since this value is higher than the cut-off value of -0.251091, sample HV119 is predicted to have a high risk of pancreatic cancer. The actual sample HV119 belonged to the early pancreatic cancer group.

For sample HV131, the calculated value is '36.9898 + (-2.220)×6.5761 + (0.9955)×8.7846 + (0.3413)×10.7145 + (-2.799)×7.3347 + (-0.1913)×8.8499 + (1.3628)×1.5673 + (-2.0521)×3.4826+ (0.7037)×2.0970+ (-0.064)×4.7682 + (-3.1754)×11.1328 + (4.4765)×6.3557 = 1.830'. Since this value is lower than the cut-off value of -0.251091, sample HV131 is predicted to have a high risk of pancreatic cancer. The actual sample HV131 belonged to pancreatic cancer group (LAPC; locally late-stage pancreatic cancer).

Analysis results obtained by applying Model_A2's equation logit(p)= 36.6273 + (1.0041)×IFNG + (-2.7473)×VEGFA + (-3.199)×SLC27A2 + (4.4958)×PTGES2 + (-2.2286)×TNF + (-2.0986)×CXCR4 + (0.6388)×CXCR2 + (1.3874)×CCL5 + (-0.224)×CCL2 + (0.3704)×CXCL11 to actual patient samples are as follows:

For sample HV18, the calculated value is '36.6273 + (1.0041)×6.8802 + (-2.7473)×7.7399 + (-3.199)×10.6540 + (4.4958)×5.6802 + (-2.2286)×6.5032 + (-2.0986)×2.9629 + (0.6388)×0.5352 + (1.3874)×1.0155 + (-0.224)×10.7275 + (0.3704)×10.5360 = -3.734. Since this value is lower than the cut-off value of - 0.290325, sample HV18 is predicted to have a low risk of pancreatic cancer and is classified as normal. The actual sample HV18 belonged to the normal group.

For sample HV119, the calculated value is '36.6273 + (1.0041)×9.4998 + (-2.7473)×7.0730 + (-3.199×9.7083 + (4.4958)×4.9634 + (-2.2286)×6.3042 + (-2.0986)×0.0542 + (0.6388)×1.4966 + (1.3874)×0.5032 + (-0.224)×10.5027 + (0.3704)×10.3085 = 6.948'. Since this value is higher than the cut-off value of - 0.290325, sample HV119 is predicted to have a high risk of pancreatic cancer. The actual sample HV119 belonged to the early pancreatic cancer group.

For sample HV131, the calculated value is '36.6273 + (1.0041)×8.7846 + (-2.7473)×7.3347 + (-3.199)×11.1328 + (4.4958)×6.3557 + (-2.2286)×6.5761 + (-2.0986)×3.4826 + (0.6388)×2.0970 + (1.3874)×1.5673 + (-0.224)×8.8499 + (0.3704)×10.7145 = 1.794'. Since this value is higher than the cut-off value of - 0.290325, sample HV131 is predicted to have a high risk of pancreatic cancer. The actual sample HV131 belonged to the pancreatic cancer group (LAPC; locally late-stage pancreatic cancer).

The results of the performance validation for the four models (Model_R1, Model_R2, Model_A1, and Model_A2) are summarized in Table 16.

TABLE 16

| **Model** | Method | Test set | Sensitivity | Specificty | AUC | ACCURACY | YOUDEN |
|---|---|---|---|---|---|---|---|
| ModeL_R1 | 0.9-10-fold Cross-Validation | train | 0.98966 | 0.89586 | 0.94276 | 0.91133 | |
| | 0.9-10-fold Cross-Validation | test | 1 | 0.94537 | 0.97268 | 0.95643 | 0.88551 0.94537 |
| | 0.8-10-fold Cross-Validation | train | 0.9931 | 0.88958 | 0.94134 | 0.90603 | 0.88267 |

(continued)

| Model | Method | Test set | Sensitivity | Specificty | AUC | ACCURACY | YOUDEN |
|---|---|---|---|---|---|---|---|
| **Healthy** vs. **Early pan-creatic cancer** | 0.8-10-fold Cross-Validation | test | 0.98889 | 0.94112 | 0.96506 | 0.94921 | 0.93011 |
| | 0.75-10-fold Cross-Validation | train | 0.97487 | 0.90548 | 0.94017 | 0.91667 | 0.88035 |
| | 0.75-10-fold Cross-Validation | test | 1 | 0.91805 | 0.95902 | 0.9314 | 0.91805 |
| | 0.7-10-fold Cross-Validation | train | 0.99545 | 0.90136 | 0.94841 | 0.91692 | 0.89682 |
| | 0.7-10-fold Cross-Validation | test | 0.98322 | 0.87276 | 0.92799 | 0.89004 | 0.85597 |
| Madel_R2 | 0.9-10-fold Cross-Validation | train | 0.95086 | 0.92653 | 0.93869 | 0.93025 | 0.87738 |
| | 0.9-10-fold Cross-Validation | test | 0.98 | 0.926 | 0.963 | 0.9399 | 0.906 |
| | 0.8-10-fold Cross-Validation | train | 0.96536 | 0.91659 | 0.94097 | 0.92445 | 0.88195 |
| | 0.8-10-fold Cross-Validation | test | 0.97071 | 0.90593 | 0.93832 | 0.91379 | 0.87664 |
| **Healthy** vs. **Early pan-creatic** cancer | 0.75-10-fold Cross-Validation | train | 0.9611 | 0.92195 | 0.94152 | 0.92829 | 0.88304 |
| | 0.75-10-fold Cross-Validation | test | 0.98889 | 0.86241 | 0.92565 | 0.88113 | 0.85129 |
| | 0.7-10-fold Cross-Validation | train | 0.95998 | 0.93674 | 0.94836 | 0.94087 | 0.89672 |
| | 0.7-10-fold Cross-Validation | test | 0.86588 | 0.90655 | 0.88622 | 0.90206 | 0.77244 |
| Mode_A1 | 0.9-10-fold Cross-Validation | train | 0.89559 | 0.89833 | 0.89696 | 0.89729 | 0.79392 |
| | 0.9-10-fold Cross-Validation | test | 0.91921 | 0.88818 | 0.90369 | 0.8949 | 0.80739 |
| | 0.8-10-fold Cross-Validation | train | 0.89534 | 0.89794 | 0.89664 | 0.89707 | 0.79328 |

(continued)

| Model | Method | Test set | Sensitivity | Specificty | AUC | ACCURACY | YOUDEN |
|---|---|---|---|---|---|---|---|
| **Healthy** vs. **All pancreatic cancers** | 0.8-10-fold Cross-Validation | test | 0.92336 | 0.86819 | | 0.8877 | 0.79155 |
| | 0.75-10-fold Cross-Validation | train | 0.89937 | 0.88907 | 0.89422 | 0.89272 | 0.78844 |
| | 0.75-10-fold Cross-Validation | test | 0.92221 | 0.89996 | 0.91109 | 0.90623 | |
| | 0.7-10-fold Cross-Validation | train | 0.89529 | 0.90104 | 0.89816 | 0.89916 | 0.79633 |
| | 0.7-10-fold Cross-Validation | test | 0.91562 | 0.87892 | 0.89727 | 0.8915 | 0.79453 |
| Model_A2 | 0.9-10-fold Cross-Validation | train | 0.85445 | 0.8996 | | 0.8977 | 0.79405 |
| | 0.9-10-fold Cross-Validation | test | 0.91921 | 0.88818 | 0.90369 | 0.8949 | 0.80739 |
| | 0.8-10-fold Cross-Validation | train | 0.88771 | 0.9063 | 0.897 | 0.89977 | 0.794 |
| | 0.8-10-fold Cross-Validation | test | 0.91364 | 0.88011 | 0.89688 | 0.8914 | 0.79375 |
| **Healthy** vs. **All pancreatic cancers** | 0.75-10-fold Cross-Validation | train | 0.89814 | 0.88914 | 0.89364 | 0.89226 | 0.78727 |
| | 0.75-10-fold Cross-Validation | test | 0.94046 | 0.87897 | 0.90971 | 0.89766 | 0.81943 |
| | 0.7-10-fold Cross-Validation | train | 0.8966 | 0.90088 | 0.89874 | 0.89966 | 0.79749 |
| | 0.7-10-fold Cross-Validation | test | 0.91495 | 0.87853 | 0.89674 | 0.89147 | 0.79347 |

As confirmed in Table 16, all pancreatic cancer diagnostic models demonstrated high diagnostic accuracy, with sensitivity ranging from 86% to 100% and specificity ranging from 86% to 94%.

From the comparison between normal individuals (183 samples) and early pancreatic cancer patients (36 samples), an optimal model equation (Model_R3) was derived using an 8-marker combination (identical to Marker Set 3) for early pancreatic cancer diagnosis. From the comparison between normal individuals (183 samples) and all pancreatic cancer patients (106 samples), an optimal model equation (Model_A3) was derived using an 8-marker combination (identical to Marker Set 3) for diagnosis of all pancreatic cancers (Table 18). The expression values of each marker, obtained from sample analysis via Real-time qPCR ($\Delta$Ct values) as described in Example 2, can be input into the model equation for pancreatic cancer diagnosis prediction. The $\Delta$Ct values were preprocessed using the Robust Scaler method (implemented in Python) before being applied to the model equation. This preprocessing was performed using Equation 5

below:

$$x'=[x - median(x)]/IQR \text{ [Equation 5]}$$

(x: the ΔCt value of each marker in the diagnostic subject, median(x): the median of the ΔCt values for each marker in an arbitrary population including the diagnostic subject, IQR (Interquartile Range): the difference between the third quartile (Q3) and the first quartile (Q1) ( Q3-Q1))

TABLE 17

| Model_R3 | Median | IQR | Model_A3 | Median | IQR |
|---|---|---|---|---|---|
| IFNG | 7.9761 | 0.8860 | IFNG | 8.0259 | 0.9636 |
| SLC27A2 | 10.7505 | 0.7249 | SLC27A2 | 10.7522 | 0.6863 |
| CCL5 | 1.2762 | 0.5589 | CCL5 | 1.3601 | 0,5938 |
| PTGES2 | 5.5063 | 0.5048 | PTGES2 | 5.5562 | 0.4893 |
| TNF | 6.9300 | 0.7141 | TNF | 6.8653 | 0.7976 |
| IFNL1 | 9.8998 | 2.4137 | IFNL1 | 9.6981 | 2.5056 |
| CCL2 | 10.7752 | 1.5991 | CCL2 | 10.4742 | 1.7941 |
| CXCR2 | 0.9542 | 1.0059 | CXCR2 | 0.8587 | 1.0916 |

TABLE 18

| Model | Pancrea cancer group | No. marker | Marker combination | Model equation logit(p) | Cut-off | AUC |
|---|---|---|---|---|---|---|
| Model | Early-sta pancreat cancer | 8 | IFNG, SLC27 CCL5, PTGE TNF, IFN CCL2, CXCR2 | logit(p)=-1.95757578511082+(1.942999) + (-2.370157)×SLC27A2 (0.236554)×CCL5 (1.781672)×PTGES2 + 1.328085)×TNF + 0.170127)×IFNL1 + 0.495828)×CCL2 (0.611586)×CXCR2 | > 0.282 | 0.973 |
| Model | All pancreat cancer | 8 | IFNG, SLC27 CCL5, PTGE TNF, IFN CCL2, CXCR2 | logit(p)=0.6543628925241534 (1.397767)×IFNG + 1.874652)×SLC27A2 (0.855608)×CCL5 (1.782533)×PTGES2 + 2.378006)×TNF + 0.000140)×IFNL1 + 0.535484)×CCL2 (0.300359)×CXCR2 | > 0.732 | 0.922 |

Analysis results obtained by applying Model_R3's equation $logit(p)$= - 1.95757578511082 + (1.942999)×IFNG + (-2.370157)×SLC27A2 + (0.236554)×CCL5 + (1.781672)×PTGES2 + (-1.328085)×TNF + (-0.170127)×IFNL1 + (-0.495828)×CCL2 + (0.611586)×CXCR2 to actual patient samples are as follows:

For sample HV18, the calculated value is '-1.95757578511082 + (1.942999)×(-1.2370) + (-2.370157)×(-0.1331) + (0.236554)×(-0.4664) + (1.781672)×0.3444 + (-1.328085)×(-0.5977) + (-0.170127)×(-0.2057) + (-0.495828)×(0.0298) + (0.611586)×(-0.4166) = -2.953'. Since this value is lower than the cut-off value of 0.282, sample HV18 is predicted to have a low risk of pancreatic cancer and is classified as normal. The actual sample HV18 belonged to the normal group.

For sample HV119, the calculated value is '-1.95757578511082 + (1.942999)×1.7197 + (-2.370157)×(-1.4376) + (0.236554)×(-1.3830) + (1.781672)×(-1.0756) + (-1.328085)×(-0.8764) + (-0.170127)×(-0.8646) + (-0.495828)×(-0.1705) + (0.611586)×(0.5392) = 4.273'. Since this value is higher than the cut-off value of 0.282, sample HV119 is predicted to have a high risk of pancreatic cancer. The actual sample HV119 belonged to the early pancreatic cancer group.

Analysis results obtained by applying Model_A3's equation *logit(p)*= =0.6543628925241534 + (1.397767)×IFNG + (-1.874652)×SLC27A2 + (0.855608)×CCL5 + (1.782533)×PTGES2 + (-2.378006)×TNF + (-0.000140)×IFNL1 + (-0.535484)×CCL2 + (0.300359)×CXCR2 to actual patient samples are as follows:

For sample HV18, the calculated value is '0.6543628925241534 + (1.397767)×(-1.1890) + (-1.874652)×(-0.1430) + (0.855608)×(-0.5804) + (1.782533)×0.2535 + (-2.378006)×(-0.4540) + (-0.000140)×(-0.1177) + (-0.535484)×0.1412 + (0.300359)×(-0.2964) = 0.131'. Since this value is lower than the cut-off value of 0.732, sample HV18 is predicted to have a low risk of pancreatic cancer and is classified as normal. The actual sample HV18 belonged to the normal group.

For sample HV119, the calculated value is '0.6543628925241534 + (1.397767)×1.5295 + (-1.874652)×(-1.5209) + (0.855608)×(-1.4431) + (1.782533)×(-1.2116) + (-2.378006)×(-0.7036) + (-0.000140)×(-0.7524) + (-0.535484)×0.0158 + (0.300359)×0.5844 = 4.089'. Since this value is higher than the cut-off value of 0.732, sample HV119 is predicted to have a high risk of pancreatic cancer. The actual sample HV119 belonged to the early pancreatic cancer group.

For sample HV131, the calculated value is '0.6543628925241534 + (1.397767)×0.78734 + (-1.874652)×0.55463 + (0.855608)×0.34889 + (1.782533)×1.6339 + (-2.378006)×(-0.36270) + (-0.000140)×0.73377 + (-0.535484)×(-0.90540) + (0.300359)×1.13440 = 5.614'. Since this value is higher than the cut-off value of 0.732, sample HV131 is predicted to have a high risk of pancreatic cancer. Actually, the sample HV131 belonged to the pancreatic cancer group (LAPC; locally late-stage pancreatic cancer).

## Claims

1. A composition for diagnosing pancreatic cancer, comprising an agent capable of detecting a biomarker for diagnosing pancreatic cancer,
wherein the biomarker for diagnosing pancreatic cancer is at least one selected from the group consisting of SLC27A2, CXCL11, CLEC7A, CXCL8, FOXP3, VEGFA, CCL2, CCL5, CCR5, CXCR4, ARG1, CXCR2, PTGS2, PTGES2, PTGES, IFNG, IFNL1, TNF, IFNB1, and IFNA1, a coding gene therefor, or a combination thereof.

2. The composition of Claim 1,
wherein the biomarker for diagnosing pancreatic cancer is selected from:

(1) at least one selected from the group consisting of SLC27A2, IFNG, CCL2, TNF, PTGES, CLEC7A, ARG1, PTGES2, FOXP3, CXCR4, VEGFA, CXCR2, and PTGS2;

at least one selected from the group consisting of SLC27A2, IFNG, CCL5, FOXP3, PTGES2, TNF, IFNA1, IFNL1, CCL2, CXCR2, PTGES, CXCR4, and IFNB1;
at least one selected from the group consisting of SLC27A2, IFNL1, IFNG, CXCL11, CCL2, PTGES2, and PTGES;
at least one selected from the group consisting of SLC27A2, CCL5, CXCR4, PTGES2, TNF, VEGFA, ARG1, CCL2, CLEC7A, IFNG, PTGES, FOXP3, PTGS2, CXCR2, and CXCL11;
at least one selected from the group consisting of SLC27A2, CCL2, CCL5, CXCR2, IFNL1, IFNG, PTGES2, and TNF;
at least one selected from the group consisting of SLC27A2, TNF, IFNG, IFNL1, CXCL11, CLEC7A, VEGFA, CCL2, CXCR4, CXCR2, ARG1, PTGES2, and PTGES;
at least one selected from the group consisting of SLC27A2, TNF, IFNG, CCL2, CXCR2, ARG1, PTGES2, and PTGES;
at least one selected from the group consisting of SLC27A2, VEGFA, CXCR4, TNF, PTGES2, CCL5, CXCL11, CXCR2, FOXP3, PTGS2, and CXCL8;
at least one selected from the group consisting of SLC27A2, PTGES2, IFNG, CCL5, VEGFA, PTGS2, CLEC7A, CXCR4, TNF, FOXP3, CCL2, CXCR2, CXCL8, PTGES, and IFNL1;
at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2;
at least one selected from the group consisting of SLC27A2, CCL5, PTGS2, CXCR2, CXCR4, CXCL11, PTGES2, TNF, and VEGFA;
at least one selected from the group consisting of SLC27A2, PTGES2, VEGFA, TNF, CXCR4, IFNG, CCL5, CXCR2, CCL2, CXCL11, CLEC7A, and ARG1;
at least one selected from the group consisting of SLC27A2, IFNG, PTGES2, CCL5, VEGFA, CCL2, TNF, FOXP3, CXCR4, IFNA1, IFNL1, PTGES, CCR5, CLEC7A, IFNB1, CXCR2, and PTGS2;
at least one selected from the group consisting of SLC27A2, PTGES2, VEGFA, TNF, CXCR4, IFNG, CCL5, CXCR2, CCL2, and CLEC7A;
at least one selected from the group consisting of SLC27A2, ARG1, CCL2, IFNG, PTGES2, TNF, VEGFA, CXCR4, and CCL5;
at least one selected from the group consisting of SLC27A2, TNF, IFNG, CXCL11, VEGFA, CCL2, CCL5, CXCR4, CXCR2, PTGS2, and PTGES2; or
at least one selected from the group consisting of SLC27A2, IFNG, VEGFA, PTGES2, TNF, CXCR4, CXCR2, CCL5, CCL2, and CXCL11;

(2) a coding gene for the above (1); or
(3) a combination of the above (1) and (2).

3. The composition of Claim 1, wherein the biomarker for diagnosing pancreatic cancer comprises:

(1) at least one selected from the group consisting of SLC27A2, IFNG, CCL2, TNF, PTGES, CLEC7A, ARG1, PTGES2, FOXP3, CXCR4, VEGFA, CXCR2, and PTGS2;

at least one selected from the group consisting of SLC27A2, IFNG, CCL5, FOXP3, PTGES2, TNF, IFNA1, IFNL1, CCL2, CXCR2, PTGES, CXCR4, and IFNB1;
at least one selected from the group consisting of SLC27A2, IFNL1, IFNG, CXCL11, CCL2, PTGES2, and PTGES;
at least one selected from the group consisting of SLC27A2, CCL5, CXCR4, PTGES2, TNF, VEGFA, ARG1, CCL2, CLEC7A, IFNG, PTGES, FOXP3, PTGS2, CXCR2, and CXCL11;
at least one selected from the group consisting of SLC27A2, CCL2, CCL5, CXCR2, IFNL1, IFNG, PTGES2, and TNF;
at least one selected from the group consisting of SLC27A2, TNF, IFNG, IFNL1, CXCL11, CLEC7A, VEGFA, CCL2, CXCR4, CXCR2, ARG1, PTGES2, and PTGES; or
at least one selected from the group consisting of SLC27A2, TNF, IFNG, CCL2, CXCR2, ARG1, PTGES2, and PTGES;

(2) a coding gene for the above (1); or
(3) a combination of the above (1) and (2), and

the composition is for use in diagnosing early-stage pancreatic cancer.

4. The composition of Claim 1, wherein the biomarker for diagnosing pancreatic cancer comprises:

(1) SLC27A2, ARG1, CCL2, CCL5, CCR5, PTGS2, CXCR2, CXCR4, CLEC7A, FOXP3, CXCL11, IFNG, IFNA1, IFNB1, IFNL1, CXCL8, PTGES, PTGES2, TNF, and VEGFA;

SLC27A2, IFNG, CCL2, TNF, PTGES, CLEC7A, ARG1, PTGES2, FOXP3, CXCR4, VEGFA, CXCR2, and PTGS2;
SLC27A2, IFNG, CCL5, FOXP3, PTGES2, TNF, IFNA1, IFNL1, CCL2, CXCR2, PTGES, CXCR4, and IFNB1;
SLC27A2, IFNG, CCL2, TNF, PTGES, FOXP3, PTGES2, CXCR4, and CXCR2;
SLC27A2, IFNL1, IFNG, CXCL11, CCL2, PTGES2, and PTGES;
SLC27A2, ARG1, CCL2, CLEC7A, IFNG, PTGES, PTGES2, TNF, and FOXP3;
SLC27A2, CCL5, CXCR4, PTGES2, TNF, VEGFA, ARG1, CCL2, CLEC7A, IFNG, PTGES, and FOXP3;
SLC27A2, CCL5, CXCR4, PTGES2, TNF, VEGFA, ARG1, CCL2, CLEC7A, IFNG, PTGES, FOXP3, PTGS2, CXCR2, and CXCL11;
SLC27A2, CCL2, CCL5, CXCR2, IFNL1, IFNG, PTGES2, and TNF;
SLC27A2, TNF, IFNG, IFNL1, CXCL11, CLEC7A, VEGFA, CCL2, CXCR4, CXCR2, ARG1, PTGES2, and PTGES; or
SLC27A2, TNF, IFNG, CCL2, CXCR2, ARG1, PTGES2, and PTGES;

(2) a coding gene for the above (1); or
(3) a combination of the above (1) and (2), and

the composition is for use in diagnosing early-stage pancreatic cancer.

5. The composition of Claim 1, wherein the biomarker for diagnosing pancreatic cancer comprises:

(1) at least one selected from the group consisting of SLC27A2, VEGFA, CXCR4, TNF, PTGES2, CCL5, CXCL11, CXCR2, FOXP3, PTGS2, and CXCL8;

at least one selected from the group consisting of SLC27A2, PTGES2, IFNG, CCL5, VEGFA, PTGS2, CLEC7A, CXCR4, TNF, FOXP3, CCL2, CXCR2, CXCL8, PTGES, and IFNL1;
at least one selected from the group consisting of IFNG, TNF, CLEC7A, CXCL8, FOXP3, VEGFA, CCL2, CCL5, CCR5, CXCR4, ARG1, CXCR2, PTGS2, and PTGES2;
at least one selected from the group consisting of SLC27A2, CCL5, PTGS2, CXCR2, CXCR4, CXCL11, PTGES2, TNF, and VEGFA;
at least one selected from the group consisting of SLC27A2, CCL5, CXCR4, PTGES2, TNF, VEGFA, ARG1, CCL2, CLEC7A, IFNG, PTGES, FOXP3, PTGS2, CXCR2, and CXCL11; or
at least one selected from the group consisting of SLC27A2, CCL2, CCL5, CXCR2, IFNL1, IFNG, PTGES2, and TNF;

(2) a coding gene for the above (1); or
(3) a combination of the above (1) and (2), and

the composition is for use in diagnosing late-stage pancreatic cancer.

6. The composition of Claim 1, wherein the biomarker for diagnosing pancreatic cancer comprises:

(1) SLC27A2, ARG1, CCL2, CCL5, CCR5, PTGS2, CXCR2, CXCR4, CLEC7A, FOXP3, CXCL11, IFNG, IFNA1, IFNB1, IFNL1, CXCL8, PTGES, PTGES2, TNF, and VEGFA;

SLC27A2, VEGFA, CXCR4, TNF, PTGES2, CCL5, CXCL11, CXCR2, FOXP3, PTGS2, 및 CXCL8;
SLC27A2, PTGES2, IFNG, CCL5, VEGFA, PTGS2, CLEC7A, CXCR4, TNF, FOXP3, CCL2, CXCR2, CXCL8, PTGES, and IFNLA1;
SLC27A2, VEGFA, CXCR4, TNF, PTGES2, CCL5, CXCR2, FOXP3, PTGS2, and CXCL8;
IFNG, TNF, CLEC7A, CXCL8, FOXP3, VEGFA, CCL2, CCL5, CCR5, CXCR4, ARG1, CXCR2, PTGS2, and PTGES2;
SLC27A2, CCL5, PTGS2, CXCR2, CXCR4, CXCL11, PTGES2, TNF, and VEGFA;
SLC27A2, CCL5, CXCR4, PTGES2, TNF, VEGFA, ARG1, CCL2, CLEC7A, IFNG, PTGES, and FOXP3;
SLC27A2, CCL5, CXCR4, PTGES2, TNF, VEGFA, ARG1, CCL2, CLEC7A, IFNG, PTGES, FOXP3, PTGS2, CXCR2, and CXCL11; or
SLC27A2, CCL2, CCL5, CXCR2, IFNL1, IFNG, PTGES2, and TNF;

(2) a coding gene for the above (1); or
(3) a combination of the above (1) and (2), and

the composition is for use in diagnosing late-stage pancreatic cancer.

7. The composition of Claim 1, wherein the biomarker for diagnosing pancreatic cancer comprises:

(1) at least one selected from the group consisting of SLC27A2, PTGES2, VEGFA, TNF, CXCR4, IFNG, CCL5, CXCR2, CCL2, CXCL11, CLEC7A, and ARG1;

at least one selected from the group consisting of SLC27A2, IFNG, PTGES2, CCL5, VEGFA, CCL2, TNF, FOXP3, CXCR4, IFNA1, IFNL1, PTGES, CCR5, CLEC7A, IFNB1, CXCR2, and PTGS2;
at least one selected from the group consisting of SLC27A2, ARG1, CCL2, IFNG, PTGES2, TNF, VEGFA, CXCR4, and CCL5;
at least one selected from the group consisting of SLC27A2, CCL5, CXCR4, PTGES2, TNF, VEGFA, ARG1, CCL2, CLEC7A, IFNG, PTGES, FOXP3, PTGS2, CXCR2, and CXCL11;
at least one selected from the group consisting of SLC27A2, CCL2, CCL5, CXCR2, IFNL1, IFNG, PTGES2, and TNF;

at least one selected from the group consisting of SLC27A2, TNF, IFNG, CXCL11, VEGFA, CCL2, CCL5, CXCR4, CXCR2, PTGS2, and PTGES2; or

at least one selected from the group consisting of SLC27A2, IFNG, VEGFA, PTGES2, TNF, CXCR4, CXCR2, CCL5, CCL2, and CXCL11;

(2) a coding gene for the above (1); or

(2) a combination of the above (1) and (2), and

the composition is for use in diagnosing either or both of early-stage pancreatic cancer and late-stage pancreatic cancer.

8. The composition of Claim 1, wherein the biomarker for diagnosing pancreatic cancer comprises:

(1) SLC27A2, ARG1, CCL2, CCL5, CCR5, PTGS2, CXCR2, CXCR4, CLEC7A, FOXP3, CXCL11, IFNG, IFNA1, IFNB1, IFNL1, CXCL8, PTGES, PTGES2, TNF, and VEGFA;

SLC27A2, PTGES2, VEGFA, TNF, CXCR4, IFNG, CCL5, CXCR2, CCL2, CXCL11, CLEC7A, and ARG1;

SLC27A2, IFNG, PTGES2, CCL5, VEGFA, CCL2, TNF, FOXP3, CXCR4, IFNA1, IFNL1, PTGES, CCR5, CLEC7A, IFNB1, CXCR2, and PTGS2;

SLC27A2, PTGES2, VEGFA, TNF, CXCR4, IFNG, CCL5, CXCR2, CCL2, and CLEC7A;

IFNG, CCL2, and PTGES2;

SLC27A2, ARG1, CCL2, IFNG, PTGES2, TNF, VEGFA, CXCR4, and CCL5;

SLC27A2, CCL5, CXCR4, PTGES2, TNF, VEGFA, ARG1, CCL2, CLEC7A, IFNG, PTGES, and FOXP3;

SLC27A2, CCL5, CXCR4, PTGES2, TNF, VEGFA, ARG1, CCL2, CLEC7A, IFNG, PTGES, FOXP3, PTGS2, CXCR2, and CXCL11;

SLC27A2, CCL2, CCL5, CXCR2, IFNL1, IFNG, PTGES2, and TNF;

SLC27A2, TNF, IFNG, CXCL11, VEGFA, CCL2, CCL5, CXCR4, CXCR2, PTGS2, and PTGES2; or

SLC27A2, IFNG, VEGFA, PTGES2, TNF, CXCR4, CXCR2, CCL5, CCL2, and CXCL11;

(2) a coding gene for the above (1); or

(3) a combination of the above (1) and (2), and

the composition is for use in diagnosing either or both of early-stage pancreatic cancer and late-stage pancreatic cancer.

9. The composition of Claim 1, wherein

the biomarker for diagnosing pancreatic cancer comprises at least one selected from the group consisting of IFNB1 and IFNA1, a coding gene therefor, or a combination thereof, and

the composition distinguishes either or both of early-stage pancreatic cancer and late-stage pancreatic cancer from a pancreatic disease other than pancreatic cancer.

10. The composition of Claim 1,

which is for use in diagnosing either or both of early-stage pancreatic cancer and late-stage pancreatic cancer, and

wherein the biomarker for diagnosing pancreatic cancer comprises one selected from the following biomarker sets, a coding gene therefor, or a combination thereof:

SLC27A2, ARG1, CCL2, CCL5, CCR5, PTGS2, CXCR2, CXCR4, CLEC7A, FOXP3, CXCL11, IFNG, IFNA1, IFNB1, IFNL1, CXCL8, PTGES, PTGES2, TNF, and VEGFA;

SLC27A2, IFNG, CCL2, TNF, PTGES, CLEC7A, ARG1, PTGES2, FOXP3, CXCR4, VEGFA, CXCR2, and PTGS2;

SLC27A2, IFNG, CCL5, FOXP3, PTGES2, TNF, IFNA1, IFNL1, CCL2, CXCR2, PTGES, CXCR4, and IFNB1;

SLC27A2, IFNG, CCL2, TNF, PTGES, FOXP3, PTGES2, CXCR4, and CXCR2;

SLC27A2, IFNL1, IFNG, CXCL11, CCL2, PTGES2, and PTGES;

SLC27A2, ARG1, CCL2, CLEC7A, IFNG, PTGES, PTGES2, TNF, and FOXP3;

SLC27A2, CCL5, CXCR4, PTGES2, TNF, VEGFA, ARG1, CCL2, CLEC7A, IFNG, PTGES, and FOXP3;

SLC27A2, CCL5, CXCR4, PTGES2, TNF, VEGFA, ARG1, CCL2, CLEC7A, IFNG, PTGES, FOXP3, PTGS2, CXCR2, and CXCL11;

SLC27A2, CCL2, CCL5, CXCR2, IFNL1, IFNG, PTGES2, and TNF;

SLC27A2, TNF, IFNG, IFNL1, CXCL11, CLEC7A, VEGFA, CCL2, CXCR4, CXCR2, ARG1, PTGES2, and PTGES;

SLC27A2, TNF, IFNG, CCL2, CXCR2, ARG1, PTGES2, and PTGES;

SLC27A2, VEGFA, CXCR4, TNF, PTGES2, CCL5, CXCL11, CXCR2, FOXP3, PTGS2, and CXCL8;

SLC27A2, PTGES2, IFNG, CCL5, VEGFA, PTGS2, CLEC7A, CXCR4, TNF, FOXP3, CCL2, CXCR2, CXCL8, PTGES, and IFNL1;

SLC27A2, VEGFA, CXCR4, TNF, PTGES2, CCL5, CXCR2, FOXP3, PTGS2, and CXCL8;

IFNG, TNF, CLEC7A, CXCL8, FOXP3, VEGFA, CCL2, CCL5, CCR5, CXCR4, ARG1, CXCR2, PTGS2, and PTGES2;

SLC27A2, CCL5, PTGS2, CXCR2, CXCR4, CXCL11, PTGES2, TNF, and VEGFA;

SLC27A2, PTGES2, VEGFA, TNF, CXCR4, IFNG, CCL5, CXCR2, CCL2, CXCL11, CLEC7A, and ARG1;

SLC27A2, IFNG, PTGES2, CCL5, VEGFA, CCL2, TNF, FOXP3, CXCR4, IFNA1, IFNL1, PTGES, CCR5, CLEC7A, IFNB1, CXCR2, and PTGS2;

SLC27A2, PTGES2, VEGFA, TNF, CXCR4, IFNG, CCL5, CXCR2, CCL2, and CLEC7A;

IFNG, CCL2, and PTGES2;

SLC27A2, ARG1, CCL2, IFNG, PTGES2, TNF, VEGFA, CXCR4, and CCL5;

SLC27A2, TNF, IFNG, CXCL11, VEGFA, CCL2, CCL5, CXCR4, CXCR2, PTGS2, and PTGES2; and

SLC27A2, IFNG, VEGFA, PTGES2, TNF, CXCR4, CXCR2, CCL5, CCL2, and CXCL11.

11. The composition of any one of Claims 1 to 10, wherein the composition is applied to a blood-derived buffy coat.

12. The composition of Claim 11, wherein the buffy coat is a whole white blood cell layer formed between a top plasma layer and a bottom red blood cell layer after centrifuging blood and is obtained from the intermediate buffy coat layer among the sequentially separated plasma layer (top), buffy coat layer (middle), and red blood cell layer (bottom) after centrifuging the blood under one or more conditions selected from the following conditions (1) to (3):

    (1) temperature: 2 to 30°C;
    (2) speed: 300g to 2000g; and
    (3) duration: 5 to 20 minutes.

13. The composition of Claim 11, wherein the buffy coat is obtained from the intermediate buffy coat layer among the sequentially separated plasma layer (top), buffy coat layer (middle), and red blood cell layer (bottom) after centrifuging the blood under one or more conditions selected from the following conditions (1) to (3):

    (1) temperature: 4 to 25°C;
    (2) speed: 500g to 1800g; and
    (3) duration: 7 to 20 minutes.

14. The composition of any one of Claims 1 to 10, wherein the agent capable of detecting a biomarker is at least one selected from the group consisting of a small molecule compound, a protein, a peptide, and a nucleic acid, which bind to the biomarker for diagnosing pancreatic cancer.

15. A kit for diagnosing pancreatic cancer, comprising the composition of any one of Claims 1 to 10.

16. The kit of Claim 15, wherein the kit is applied to a blood-derived buffy coat.

17. A method of providing information for diagnosis of pancreatic cancer, the method comprising a step of detecting a biomarker for diagnosing pancreatic cancer in a blood sample isolated from a subject, wherein the biomarker for diagnosing pancreatic cancer is at least one selected from the group consisting of SLC27A2, IFNL1, IFNG, CXCL11, TNF, IFNB1, IFNA1, CLEC7A, CXCL8, FOXP3, VEGFA, CCL2, CCL5, CCR5, CXCR4, ARG1, CXCR2, PTGS2, PTGES2, and PTGES, a coding gene therefor, or a combination thereof.

18. The method of Claim 17, wherein the blood sample is a blood-derived buffy coat.

**19.** The method of Claim 18, wherein the buffy coat is obtained from the intermediate buffy coat layer among the sequentially separated plasma layer (top), buffy coat layer (middle), and red blood cell layer (bottom) after centrifuging the blood under one or more conditions selected from the following conditions (1) to (3):

(1) temperature: 2 to 30°C;
(2) speed: 300g to 2000g; and
(3) duration: 5 to 20 minutes.

【FIG. 1】

【FIG. 2】

IFNL1 (IL-29)

【FIG. 3】

IFNG

【FIG. 4】

## CXCL11 (I-TAC)

【FIG. 5】

## TNF

【FIG. 6】

## CLEC7A (DECTIN-1)

【FIG. 7】

## CXCL8 (IL-8)

【FIG. 8】

**FOXP3**

【FIG. 9】

**VEGFA**

【FIG. 10】

## CCL2

【FIG. 11】

## CCL5

【FIG. 12】

## CCR5

【FIG. 13】

## CXCR4

【FIG. 14】

## ARG1

【FIG. 15】

## CXCR2

【FIG. 16】

## PTGS2 (COX2)

【FIG. 17】

## PTGES2

【FIG. 18】

**SLC27A2 (FATP2)**

【FIG. 19】

**PTGES**

【FIG. 20】

# IFNB1

【FIG. 21】

【FIG. 22】

IFNA1

【FIG. 23】

# Normal vs. Early P.C

## 9 huvet markers

Sensitivity: 91.4
Specificity: 94.9
Criterion: >0.2625

AUC = 0.979
P < 0.0001

【FIG. 24】

# Normal vs. Late P.C

## 9 huvet markers

Sensitivity: 89.6
Specificity: 89.8
Criterion: >0.3698

AUC = 0.951
P < 0.0001

【FIG. 25】

Normal vs. All stage P.C

9 huvet markers

Sensitivity: 95.1
Specificity: 80.8
Criterion: >0.2508

AUC = 0.942
P < 0.0001

【FIG. 26a】

# Normal vs. Early P.C

## all huvet markers

【FIG. 26b】

Normal vs. Late P.C

all huvet markers

【FIG. 26c】

Normal vs. All stage P.C

all huvet markers

【FIG. 27a】

Normal vs. Early P.C

huvet marker set 1

【FIG. 27b】

# Normal vs. Late P.C

## huvet marker set 1

Sensitivity: 97.0
Specificity: 75.1
Criterion: >0.1119

AUC = 0.943
P < 0.0001

Sensitivity

100-Specificity

【FIG. 27c】

Normal vs. All stage P.C

huvet marker set 1

Sensitivity: 94.1
Specificity: 81.4
Criterion: >0.2299

AUC = 0.947
P < 0.0001

【FIG. 28a】

# Normal vs. Early P.C
## huvet marker set 2

Sensitivity: 91.2
Specificity: 97.7
Criterion: >0.4231

AUC = 0.980
P < 0.0001

Sensitivity

100-Specificity

【FIG. 28b】

# Normal vs. Late P.C

## huvet marker set 2

Sensitivity: 92.5
Specificity: 85.9
Criterion: >0.2135

AUC = 0.955
P < 0.0001

【FIG. 28c】

# Normal vs. All stage P.C
## huvet marker set 2

Sensitivity: 89.1
Specificity: 89.3
Criterion: >0.3924

AUC = 0.952
P < 0.0001

Sensitivity

100-Specificity

| Variable | 8_markers |
|---|---|
| | 8 markers |
| Classification variable | Code |

| Sample size | 212 |
|---|---|
| Positive group [a] | 35 (16.51%) |
| Negative group [b] | 177 (83.49%) |

[a] Code = 1

[b] Code = 0

| Disease prevalence (%) | unknown |
|---|---|

### Area under the ROC curve (AUC)

| Area under the ROC curve (AUC) | 0.972 |
|---|---|
| Standard Error [a] | 0.0101 |
| 95% Confidence interval [b] | 0.939 to 0.989 |
| z statistic | 46.561 |
| Significance level P (Area=0.5) | <0.0001 |

[a] DeLong et al., 1988

[b] Binomial exact

### Youden index

| Youden index J | 0.8418 |
|---|---|
| Associated criterion | >0.072997941 |
| Sensitivity | 100.00 |
| Specificity | 84.18 |

Normal vs. Early P.C
huvet marker set 3

Sensitivity: 100.0
Specificity: 84.2
Criterion: >0.073

AUC = 0.972
P < 0.0001

[FIG. 29a]

| Variable | 8_markers<br>8 markers |
|---|---|
| Classification variable | Code |

| Sample size | 244 |
|---|---|
| Positive group [a] | 67 (27.46%) |
| Negative group [b] | 177 (72.54%) |

[a] Code = 1
[b] Code = 0

| Disease prevalence (%) | unknown |
|---|---|

### Area under the ROC curve (AUC)

| Area under the ROC curve (AUC) | 0.905 |
|---|---|
| Standard Error [a] | 0.0212 |
| 95% Confidence interval [b] | 0.861 to 0.938 |
| z statistic | 19.063 |
| Significance level P (Area=0.5) | <0.0001 |

[a] DeLong et al., 1988
[b] Binomial exact

### Youden index

| Youden index J | 0.6829 |
|---|---|
| Associated criterion | >0.224663776 |
| Sensitivity | 88.06 |
| Specificity | 80.23 |

Normal vs. Late P.C

huvet marker set 3

Sensitivity: 88.1
Specificity: 80.2
Criterion: >0.2247

AUC = 0.905
P < 0.0001

[FIG. 29b]

| Variable | 8_markers<br>8 markers |
| --- | --- |
| Classification variable | Code |

| Sample size | 279 |
| --- | --- |
| Positive group [a] | 102 (36.56%) |
| Negative group [b] | 177 (63.44%) |

[a] Code = 1
[b] Code = 0

| Disease prevalence (%) | unknown |
| --- | --- |

**Area under the ROC curve (AUC)**

| Area under the ROC curve (AUC) | 0.922 |
| --- | --- |
| Standard Error [a] | 0.0161 |
| 95% Confidence interval [b] | 0.884 to 0.951 |
| z statistic | 26.187 |
| Significance level P (Area=0.5) | <0.0001 |

[a] DeLong et al., 1988
[b] Binomial exact

**Youden index**

| Youden index J | 0.7340 |
| --- | --- |
| Associated criterion | >0.319571486 |
| Sensitivity | 89.22 |
| Specificity | 84.18 |

[FIG. 29c]

Normal vs. All stage P.C
huvet marker set 3

Sensitivity: 89.2
Specificity: 84.2
Criterion: >0.3196

AUC = 0.922
P < 0.0001

| Test set | Total test size | Positive group size | Negative group size | Marker set name | P-value | Youden index (J) | AUC (95% CI) | Sensitivity | Specificity |
|---|---|---|---|---|---|---|---|---|---|
| Pancreatic Cancer (Stage: Early stage only) vs Normal | 212 | 35 | 177 | 9 Huvet markers | <0.0001 | 0.8634 | 0.979 (0.949 to 0.993) | 91.43 | 94.92 |
| | 212 | 35 | 177 | Marker Set 1 (12 markers) | <0.0001 | 0.8804 | 0.979 (0.949 to 0.994) | 91.43 | 96.61 |
| | 212 | 35 | 177 | Marker Set 2 (15 markers) | <0.0001 | 0.8892 | 0.980 (0.951 to 0.994) | 91.18 | 97.74 |
| | 212 | 35 | 177 | Marker Set 3 (8 markers) | <0.0001 | 0.8418 | 0.972 (0.939 to 0.989) | 100.00 | 84.18 |
| | 211 | 34 | 177 | All huvet markers | <0.0001 | 0.9084 | 0.984 (0.957 to 0.996) | 97.06 | 93.79 |
| Pancreatic Cancer (Stage: Late stage only) vs Normal | 244 | 67 | 177 | 9 Huvet markers | <0.0001 | 0.7938 | 0.951 (0.916 to 0.975) | 89.55 | 89.83 |
| | 244 | 67 | 177 | Marker Set 1 (12 markers) | <0.0001 | 0.7216 | 0.943 (0.907 to 0.969) | 97.01 | 75.14 |
| | 244 | 67 | 177 | Marker Set 2 (15 markers) | <0.0001 | 0.7841 | 0.955 (0.920 to 0.977) | 92.54 | 85.88 |
| | 244 | 67 | 177 | Marker Set 3 (8 markers) | <0.0001 | 0.6829 | 0.905 (0.861 to 0.938) | 88.06 | 80.23 |
| | 244 | 67 | 177 | All huvet markers | <0.0001 | 0.7991 | 0.956 (0.922 to 0.978) | 94.03 | 85.88 |
| Pancreatic Cancer (Stage: All) vs Normal | 279 | 102 | 177 | 9 Huvet markers | <0.0001 | 0.7589 | 0.942 (0.908 to 0.967) | 95.10 | 80.79 |
| | 279 | 102 | 177 | Marker Set 1 (12 markers) | <0.0001 | 0.7547 | 0.947 (0.914 to 0.970) | 94.12 | 81.36 |
| | 278 | 101 | 177 | Marker Set 2 (15 markers) | <0.0001 | 0.7837 | 0.952 (0.920 to 0.974) | 89.11 | 89.27 |
| | 279 | 102 | 177 | Marker Set 3 (8 markers) | <0.0001 | 0.7340 | 0.922 (0.884 to 0.951) | 89.22 | 84.18 |
| | 278 | 101 | 177 | All huvet markers | <0.0001 | 0.7739 | 0.951 (0.919 to 0.974) | 92.08 | 85.31 |

## INTERNATIONAL SEARCH REPORT

| International application No. |
| --- |
| **PCT/KR2023/015129** |

**A. CLASSIFICATION OF SUBJECT MATTER**

**C12Q 1/6886**(2018.01)i; **G01N 33/574**(2006.01)i

According to International Patent Classification (IPC) or to both national classification and IPC

**B. FIELDS SEARCHED**

Minimum documentation searched (classification system followed by classification symbols)

C12Q 1/6886(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 췌장암(pancreatic cancer), 진단(diagnosis), 바이오마커(biomarker)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | GE, W. et al. The YY1/miR-548t-5p/CXCL11 signaling axis regulates cell proliferation and metastasis in human pancreatic cancer. Cell Death and Disease. 2020, vol. 11, document no. 294, pp. 1-18.<br>See abstract; and page 13, right column. | 1-3,5,7,14-15,17<br>11-13,16,18-19 |
| Y | KR 10-2226826 B1 (HUVET BIO, INC.) 11 March 2021 (2021-03-11)<br>See claims 1-8. | 11-13,16,18-19 |
| A | KR 10-2020-0102746 A (HUVET BIO, INC.) 01 September 2020 (2020-09-01)<br>See entire document. | 1-3,5,7,11-19 |
| A | KR 10-2022-0046904 A (THE CATHOLIC UNIVERSITY OF KOREA INDUSTRY-ACADEMIC COOPERATION FOUNDATION) 15 April 2022 (2022-04-15)<br>See entire document. | 1-3,5,7,11-19 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **13 February 2024** | **14 February 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/015129**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/KR2023/015129** |

**Box No. III    Observations where unity of invention is lacking (Continuation of item 3 of first sheet)**

This International Searching Authority found multiple inventions in this international application, as follows:

The invention of group 1: parts of claims 1-3, 5, 7 and 11-19 pertain to a composition for diagnosing pancreatic cancer, comprising a feature related to one gene selected from a plurality of genes (20 or less), a kit for diagnosing pancreatic cancer, comprising the composition for diagnosing pancreatic cancer, and a method for providing information for pancreatic cancer diagnosis,

The invention of group 2: parts of claims 1, 4, 6, 8 and 10-19 pertain to a composition for diagnosing pancreatic cancer, comprising a feature related to a gene combination selected from gene combinations including a plurality of genes, a kit for diagnosing pancreatic cancer, comprising the composition for diagnosing pancreatic cancer, and a method for providing information for pancreatic cancer diagnosis,

The invention of group 3: parts of claims 1, 9 and 11-19 pertain to a composition for diagnosing pancreatic cancer, comprising a feature related to a gene selected from two genes, a kit for diagnosing pancreatic cancer, comprising the composition for diagnosing pancreatic cancer, and a method for providing information for pancreatic cancer diagnosis.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-3, 5, 7 and 11-19**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/KR2023/015129**

| Patent document cited in search report | | | Publication date (day/month/year) | Patent family member(s) | | | Publication date (day/month/year) |
|---|---|---|---|---|---|---|---|
| KR | 10-2226826 | B1 | 11 March 2021 | CN | 116096917 | A | 09 May 2023 |
| | | | | EP | 4194567 | A1 | 14 June 2023 |
| | | | | JP | 2023-538836 | A | 12 September 2023 |
| | | | | US | 2023-349908 | A1 | 02 November 2023 |
| | | | | WO | 2022-031072 | A1 | 10 February 2022 |
| KR | 10-2020-0102746 | A | 01 September 2020 | None | | | |
| KR | 10-2022-0046904 | A | 15 April 2022 | WO | 2022-075773 | A1 | 14 April 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220123808

**Non-patent literature cited in the description**

- **MIRON B. KURSA** ; **WITOLD R. RUDNICKI**. Feature Selection with the Boruta Package.. *Journal of Statistical Software*, 2010, vol. 36 (11), 1-13